# EUROPEAN PATENT APPLICATION

(11) **EP 4 523 697 A1**
(43) Date of publication of application: **19.03.2025**
(21) Application number: 23802842.7
(22) Date of filing: 08.05.2023
(51) Int. Cl.: A61K 35/761, A61K 35/763, A61K 35/768, C12N 15/62, C12N 5/10, C12N 7/01, C07K 16/28, A61K 39/395, A61P 35/00

(54) **THERAPEUTIC AGENT COMPRISING MULTISPECIFIC ANTIBODY AND USE THEREOF IN TUMOR THERAPY**

(30) Priority: 09.05.2022 CN 202210496949
(71) Applicant: Hangzhou Viromab Biotech Co., Ltd., Hangzhou, Zhejiang 311100 (CN)
(72) Inventor: XU, Wenxin, Hangzhou, Zhejiang 311100 (CN); HU, Fang, Hangzhou, Zhejiang 311121 (CN); CHEN, Can, Hangzhou, Zhejiang 311121 (CN); CAI, Jinlu, Hangzhou, Zhejiang 311121 (CN); DING, Lieming, Hangzhou, Zhejiang 311100 (CN)
(74) Representative: J A Kemp LLP
(86) International application number: PCT/CN2023/092707
(87) International publication number: WO 2023/217072

(57) **Abstract**

Disclosed are a therapeutic agent comprising a labeling polypeptide introduced into a tumor cell and a multispecific antibody capable of binding to and recognizing the labeling polypeptide, the multispecific antibody, a kit comprising the therapeutic agent, and use of the therapeutic agent or the multispecific antibody in the manufacture of a medicament for treating a tumor and/or a cancer.

## Description

### TECHNICAL FIELD

The present disclosure relates to the field of biopharmaceutics, in particular to a therapeutic agent comprising a multispecific antibody and use thereof in the treatment of a tumor.

### BACKGROUND

According to the data published by the world health organization (WHO), tumors have become one of the most serious diseases endangering human health, with a continuous increase in the number of cases and high mortality rates. There are still many unmet clinical needs in current traditional tumor treatment methods. An oncolytic virus therapy provides a novel tumor immunotherapy in which oncolytic viruses selectively replicate in tumor cells, leading to direct lysis to destroy tumor cells, and thereby stimulate host anti-tumor immune response to kill tumor cells without affecting the growth of normal cells, which holds promise for a wide range of patients with malignant tumors (Shi T etal., Front Immunol, 2020;11: 683). Compared to other tumor immunotherapies, oncolytic virus therapies exhibit a number of advantages including high tumor-killing efficiency, good targeting, low drug resistance, and low cost (Heo et al., 2012).

Like other anti-tumor therapies, oncolytic virus therapies also suffer from limitations such as viral delivery to the target, penetration into the tumor mass, and strong anti-viral immune responses elicited, and limited by physical barriers and host immunity. Therefore, the oncolytic virus therapy is often required to be combined with chemotherapy or immunotherapeutic drugs to improve the therapeutic effect. Globally, oncolytic viral drugs that are commercially available or under investigation are largely involved in experimental trials for combination drug treatments, for example, in combination with an immune checkpoint or a CAR-T cell therapy.

Bispecific antibodies (BsAbs) are antibodies that can specifically bind to two antigens or epitopes simultaneously. Since the concept of BsAbs was first proposed by Nisonoff and his collaborators in 1960, the development of bispecific antibodies has advanced rapidly with the maturation of genetic engineering technology for antibodies. As BsAbs can target multiple antigens or antigenic epitopes, they have more advantages than monoclonal antibodies and have also gradually shown higher therapeutic effects than monoclonal antibody combination therapy. For example, specific immune effector cells can be redirected to adjacent tumor cells to enhance tumor killing; moreover, the specificity of binding can be increased through the interaction between two different cell surface antigens; meanwhile, compared with combinations with single antibodies, BsAbs enable reductions in development costs and clinical trials. Bispecific antibodies have now become a popular research topic in the area of antibody engineering and have wide application prospects in tumor treatment, autoimmune diseases, and the like. Furthermore, with the development of technology, antibodies targeting more antigens or more antigenic epitopes, such as trispecific antibodies or multifunctional fusion antibodies, have emerged in response to the demand.

Despite the endless emergence of various therapies, there is an unmet clinical need, and there is an urgent need to develop new immunotherapies.

### SUMMARY

The present disclosure provides a therapeutic agent comprising a multispecific antibody and use thereof in the treatment of a tumor. A tumor cell or a cancer cell is labeled with a labeling polypeptide by a first active ingredient in the therapeutic agent, and the labeling polypeptide labeled on the tumor cell or the cancer cell is recognized by a multispecific antibody. Another antigen-binding moiety of the multispecific antibody can recruit immune cells, for example, T cells, NK cells, and the like, to kill the tumor cell or the cancer cell. The therapeutic agent provided by the present disclosure can significantly improve the effect of tumor treatment. Furthermore, tumor cells or cancer cells can be labeled with the labeling polypeptide by administering an oncolytic virus, the tumor killing effect can be achieved before the multispecific antibody is administered, and the purpose of tumor treatment can be achieved by the significantly improved treatment effect after administration of the multispecific antibody. To this end, the present disclosure further provides a novel immunotherapy comprising administering to a subject a therapeutically effective amount of an oncolytic virus and a multispecific antibody, separately, for the purpose of tumor treatment by a combination of the oncolytic virus therapy and the multispecific antibody therapy. The immunotherapy provided by the present disclosure provides a brand-new and creative therapy for the treatment of a tumor.

Specifically, the present disclosure provides the following technical solutions:

A first aspect of the present disclosure provides a therapeutic agent comprising:
(a) a first composition comprising a first active ingredient, wherein the first active ingredient comprises or contains a nucleic acid having a labeling polypeptide encoding sequence for being introduced into a tumor cell and/or a cancer cell; the labeling polypeptide has an extracellular antigen determining region, a spacer portion, and a transmembrane portion that are operably linked; an amino acid sequence of the extracellular antigen determining region comprises the amino acid sequences of one or more antigenic epitope polypeptides; and in a natural state, an amino acid sequence of a cell membrane protein or a secreted protein of a mammal does not comprise the amino acid sequence of the antigenic epitope polypeptide; and
(b) a second composition comprising a second active ingredient, wherein the second active ingredient comprises a multispecific antibody, and the multispecific antibody comprises at least a first antigen-binding moiety and a second antigen-binding moiety, wherein the first antigen-binding moiety is capable of specifically recognizing and binding to the extracellular antigen determining region of the labeling polypeptide, and
   the second antigen-binding moiety is capable of specifically recognizing and binding to an immune cell antigen or a first cytokine or a receptor thereof; and
   the multispecific antibody may also optionally comprise a third antigen-binding moiety specifically recognizing and binding to a tumor antigen or an immune checkpoint or a second cytokine or a receptor thereof.

A second aspect of the present disclosure provides a multispecific antibody comprising at least a first antigen-binding moiety and a second antigen-binding moiety, wherein the first antigen-binding moiety comprises one or more domains capable of specifically recognizing and binding to an amino acid sequence of Strep tag I or Strep tag II, and the second antigen-binding moiety comprises one or more domains capable of specifically recognizing and binding to an immune cell antigen or a first cytokine or a receptor thereof; and the multispecific antibody may also optionally comprise a third antigen-binding moiety comprising one or more domains capable of specifically recognizing and binding to a tumor antigen or an immune checkpoint or a second cytokine or a receptor thereof. The first antigen-binding moiety is capable of specifically recognizing and binding to Strep tag I or Strep tag II, and these tags are confirmed to be absent or nearly absent in a mammalian body. By labeling mammalian, especially human, tumor cells with the corresponding tags, the first antigen-binding moiety is capable of specifically targeting these tags, thereby enabling the multispecific antibody to specifically target the tumor cells. Then, an immune cell or a cytokine or a receptor thereof is recruited by the second antigen-binding moiety to specifically kill the tumor cells, thereby achieving a tumor-specific targeting and killing effect. The multispecific antibody can further comprise a third antigen-binding moiety capable of specifically targeting a tumor antigen or an immune checkpoint or a cytokine or a receptor thereof according to requirements to further exert the tumor targeting and killing effect.

A third aspect of the present disclosure provides a polynucleotide encoding the multispecific antibody according to the second aspect of the present disclosure.

A fourth aspect of the present disclosure provides a construct comprising the polynucleotide according to the third aspect of the present disclosure.

A fifth aspect of the present disclosure provides a host cell comprising the construct according to the fourth aspect of the present disclosure.

A sixth aspect of the present disclosure provides a method for producing the multispecific antibody according to the second aspect, comprising: culturing the host cell according to the fifth aspect, and collecting the multispecific antibody from the culture.

A seventh aspect of the present disclosure provides use of the therapeutic agent or the multispecific antibody in the manufacture of a medicament for treating a tumor and/or a cancer, wherein the therapeutic agent mentioned is the therapeutic agent mentioned in the first aspect of the present disclosure, and the multispecific antibody is the multispecific antibody according to the second aspect of the present disclosure.

An eighth aspect of the present disclosure provides a kit of combinational drugs with a synergistic effect for treating a tumor and/or a cancer, comprising: a first container containing the first composition in the therapeutic agent according to the first aspect; a second container containing the second composition in the therapeutic agent according to the first aspect or containing the multispecific antibody according to the second aspect, wherein the first container and the second container are independent; and instructions for the timing and mode of administration.

A ninth aspect of the present disclosure provides a method for treating a tumor and/or a cancer, comprising: administering to a subject the first composition in the therapeutic agent according to any one of the first aspect; and administering to the subject the second composition in the therapeutic agent according to the first aspect, or the multispecific antibody according to the second aspect.

A tenth aspect of the present disclosure provides a novel immunotherapy comprising: administering to a subject a therapeutically effective amount of an oncolytic virus, wherein a genome of the oncolytic virus comprises a labeling polypeptide encoding sequence, and the labeling polypeptide has an extracellular antigen determining region, a spacer portion, and a transmembrane portion that are operably linked; an amino acid sequence of the extracellular antigen determining region comprises an amino acid sequence of one or more antigenic epitope polypeptides; and in a natural state, an amino acid sequence of a cell membrane protein or a secreted protein of the subject does not comprise the amino acid sequence of the antigenic epitope polypeptide; and
administering to the subject a therapeutically effective amount of a multispecific antibody, wherein the multispecific antibody comprises at least a first antigen-binding moiety and a second antigen-binding moiety, wherein the first antigen-binding moiety is capable of specifically recognizing and binding to the extracellular antigen determining region of the labeling polypeptide, and the second antigen-binding moiety is capable of specifically recognizing and binding to an immune cell antigen or a first cytokine or a receptor thereof; and
the multispecific antibody may also optionally comprise a third antigen-binding moiety capable of specifically recognizing and binding to a tumor antigen or an immune checkpoint or a second cytokine or a receptor thereof.

The present disclosure has achieved the following beneficial technical effects:
The present disclosure provides a therapeutic agent comprising a first composition and a second composition, wherein the first composition comprises a first active ingredient that labels a surface of a tumor and/or cancer cell with an exogenous labeling polypeptide, and the labeling polypeptide is recognized through a multispecific antibody in the second composition. Through the combined use of both compositions, the therapeutic agent can effectively kill tumor cells. Moreover, when the oncolytic virus is used as a vector to mediate the expression of the exogenous labeling polypeptide in tumor cells, the multispecific antibody targeting the labeling polypeptide can effectively eliminate residual tumor cells infected by the oncolytic virus, building upon the existing oncolytic killing effect of the oncolytic virus. Meanwhile, as the oncolytic virus destroys the microenvironment of the tumor, the killing capacity of the multispecific antibody to the tumor can be further improved, thereby significantly improving the effectiveness of tumor treatment, in particular the therapeutic effect on a solid tumor. Specifically, the present disclosure utilizes a labeling polypeptide amino acid sequence having an extracellular antigen determining region, a spacer portion, and a transmembrane portion, and a nucleic acid encoding the labeling polypeptide, such that the tumor cells and/or the cancer cells can express the labeling polypeptide after transfection of the nucleic acid into the tumor cells and/or the cancer cells or after infection of the tumor cells and/or the cancer cells by a recombinant virus obtained by insertion of the nucleic acid into a viral genome. The labeling polypeptide can be ultimately modified on the surfaces of the tumor cells and/or the cancer cells. As the amino acid sequence of the extracellular antigen determining region of the labeling polypeptide comprises the amino acid sequences of one or more antigenic epitope polypeptides, the problems of heterogeneity of solid tumor antigen expression and tumor evasion of immune surveillance can be effectively solved. Meanwhile, by the combined use with the multispecific antibody capable of recognizing an extracellular antigen determining region, the multispecific antibody is used to recognize and bind to immune cells, and thus to achieve the bridging of the immune cells and the tumor cells and kill the tumor cells specifically. Moreover, compared with the killing of the immune cells modified by CAR, the killing of the immune cells mediated by the multispecific antibody can greatly reduce the drug cost and toxic and side effects. Particularly, CAR-T cells have a longer survival time within the body, and the continuous presence of CAR-T cells targeting the labeling polypeptide can hinder subsequent repeated administration of the nucleic acid encoding the labeling polypeptide or the recombinant virus; and the multispecific antibody has a certain half-life and does not present continuously *in vivo*, allowing for more flexible maintenance of its drug concentration *in vivo* by adjusting the administration cycle and frequency.

Furthermore, when the oncolytic virus is used as a vector, its ability to specifically replicate within tumor cells allows the introduction of the labeling polypeptide encoding nucleic acid into tumor cells and/or cancer cells, so that the oncolytic virus can significantly improve the expression of the antigenic epitope peptide on the surfaces of the tumor cells while killing the tumor cells and/or the cancer cells, and thereby exhibit a synergistic therapeutic effect when combined with immune cells recruited by the multispecific antibody. More importantly, the on-target off-tumor effect on normal tissues is avoided. As the oncolytic virus destroys the microenvironment of the tumor, the killing capacity of the multispecific antibody to the tumor cells is further improved, thereby significantly improving the effectiveness of tumor (specifically, solid tumor) treatment. In addition, the multispecific antibody can also effectively eliminate tumor cells that are infected by the oncolytic virus but fail to complete a replication cycle and generate a sufficient quantity of progeny viruses to be lysed, thereby achieving a still further synergistic effect. In addition, the antigens released by the tumor cells lysed by the oncolytic virus can further activate the anti-tumor immunity of the organism, such that a better tumor killing effect can be achieved compared with that of the independent use of the oncolytic virus or the multispecific antibody, and a synergistic therapeutic effect is achieved.

In a further invention, the present disclosure provides an improvement in the amino acid sequence and/or the protein structure of the multispecific antibody targeting the labeling polypeptide, including the humanized modification of the first antigen-binding moiety, the second antigen-binding moiety, and the constant domains of the heavy chain/light chain, thereby reducing the immunogenicity of the therapeutic agent of the present disclosure. In addition, by modifying the first antigen-binding moiety and the second antigen-binding moiety of the multispecific antibody, the affinities of at least these two moieties for the target antigen are improved. This improvement allows the multispecific antibody not only to effectively exert anti-tumor effects but also to avoid safety problems such as immune storm caused by excessive affinity for immune cell antigens. For example, through the humanized modification of the first antigen-binding moiety targeting the labeling polypeptide, the immunogenicity possibly caused is effectively reduced while the affinity for the labeling polypeptide target antigen is ensured, such that the risk generated in a human body can be reduced, and the affinity for the labeling polypeptide target antigen is kept at a very high level, such that the tumor killing capacity of the multispecific antibody can be ensured; furthermore, through the humanized modification of the second antigen-binding moiety targeting the immune cell antigen, the affinity activity of the multispecific antibody for the target antigen on the immune cell is maintained at a relatively moderate level when the multispecific antibody recruits the immune cells, such that the tumor killing can be achieved, and the safety problem can be avoided. The design and screening of the present disclosure allow the multispecific antibody to exhibit particularly suitable affinity overall. The modified multispecific antibody has excellent anti-tumor effect and safety.

### BRIEF DESCRIPTION OF THE DRAWINGS

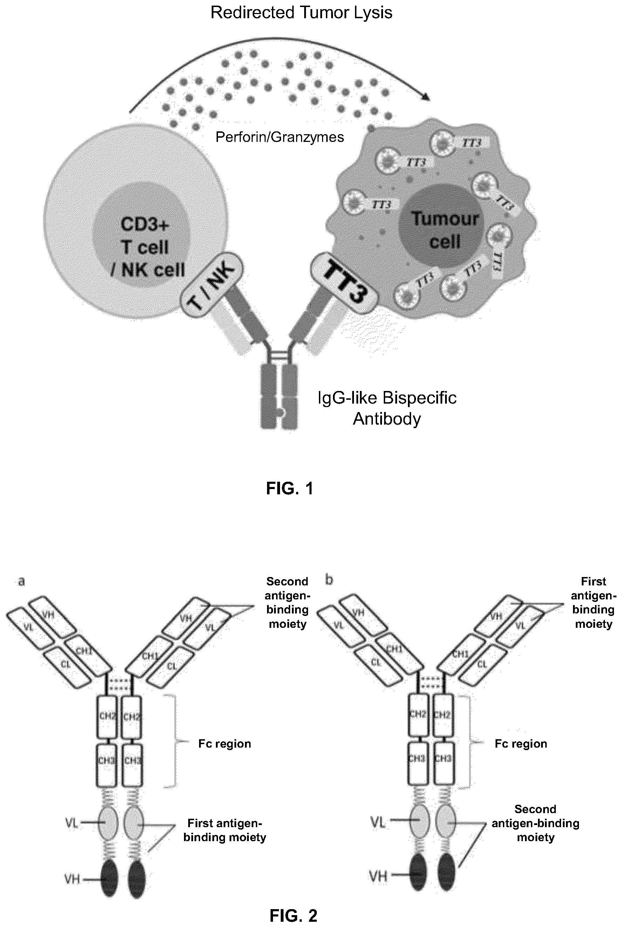

FIG. 1 shows a schematic diagram of a therapeutic agent provided according to an example of the present disclosure exerting a therapeutic effect.
FIG. 2 shows a schematic diagram of the structures of bispecific antibodies provided according to an example of the present disclosure, wherein "a" shows a schematic diagram of the structure of one bispecific antibody, and "b" shows a schematic diagram of the structure of another bispecific antibody.

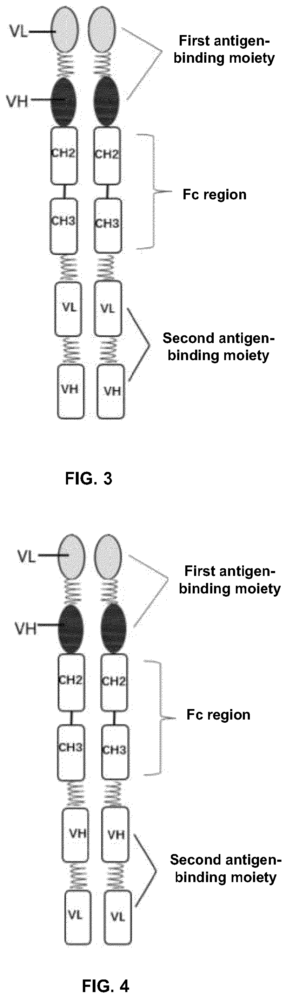

FIG. 3 shows a schematic diagram of the structure of a bispecific antibody provided according to an example of the present disclosure, wherein in the first antigen-binding moiety, the gray ovals represent VLs and the black ovals represent VHs.
FIG. 4 shows a schematic diagram of the structure of a bispecific antibody provided according to an example of the present disclosure, wherein in the first antigen-binding moiety, the gray ovals represent VLs and the black ovals represent VHs.

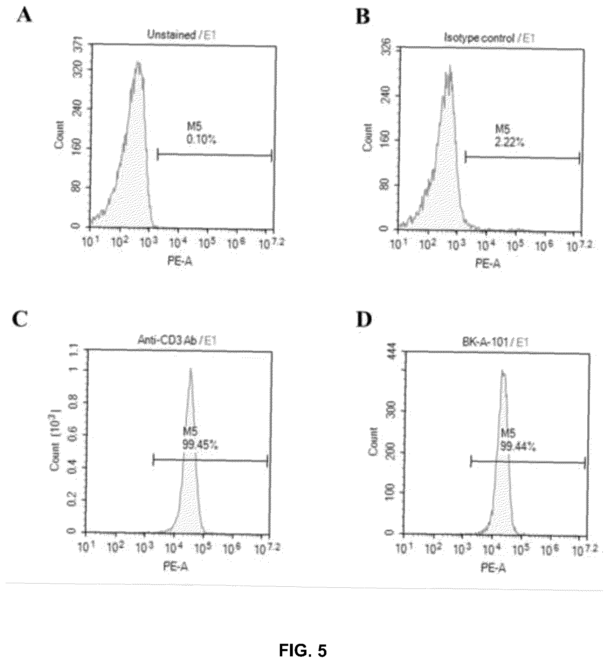

FIG. 5 shows a FACS assay result for the binding activity of a bispecific antibody to T cells provided according to Example 1 of the present disclosure. A to D of FIG. 5 are all flow cytometry histograms, wherein A of FIG. 5 shows a flow cytometry assay plot without antibody staining (blank control), B of FIG. 5 shows a flow cytometry assay plot with isotype control antibody staining (negative control), C of FIG. 5 shows a flow cytometry assay plot with anti-CD3 monoclonal antibody staining (positive control), and D of FIG. 5 shows a flow cytometry assay plot with bispecific antibody staining (bispecific antibody). In the plots, the horizontal axis represents the fluorescence intensity readings displayed on the flow cytometer, and the vertical axis indicates the relative cell count.

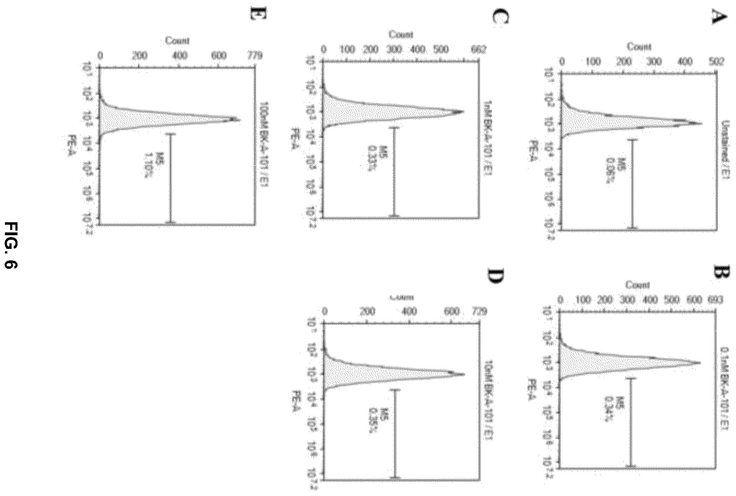

FIG. 6 shows a FACS assay result for the binding activity of a bispecific antibody to wild-type cell strain HCT116 provided according to Example 1 of the present disclosure. A of FIG. 6 shows a result for a blank control, B of FIG. 6 shows a result for the staining with 0.1 nM bispecific antibody, C of FIG. 6 shows a result for the staining with 1 nM bispecific antibody, D of FIG. 6 shows a result for the staining with 10 nM bispecific antibody, and E of FIG. 6 shows a result for the staining with 100 nM bispecific antibody. In the plots, the horizontal axis represents the fluorescence intensity readings displayed on the flow cytometer, and the vertical axis indicates the relative cell count.

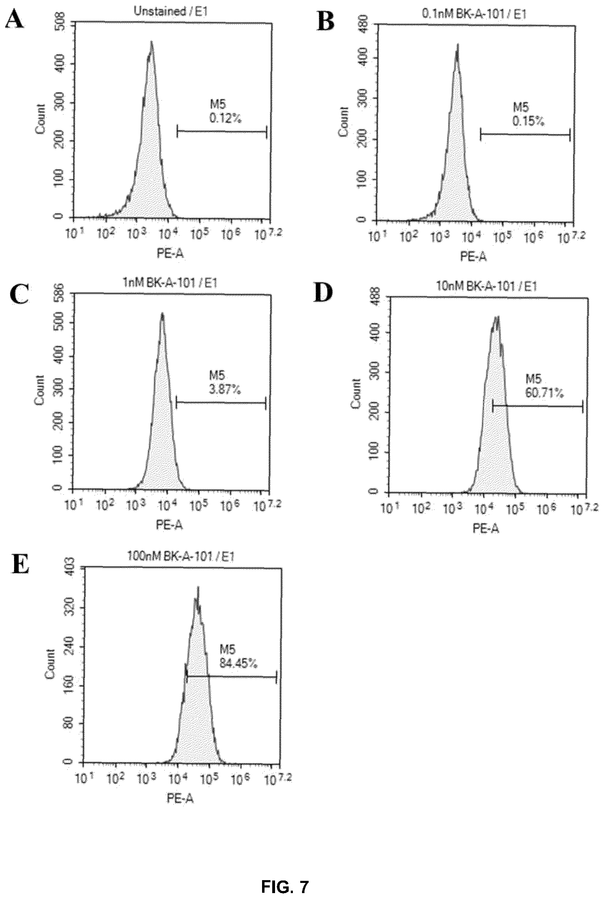

FIG. 7 shows a FACS assay result for the binding activity of a bispecific antibody to cell strain HCT116-TT3 provided according to Example 1 of the present disclosure. A of FIG. 7 shows a result for a blank control, B of FIG. 7 shows a result for the staining with 0.1 nM bispecific antibody, C of FIG. 7 shows a result for the staining with 1 nM bispecific antibody, D of FIG. 7 shows a result for the staining with 10 nM bispecific antibody, and E of FIG. 7 shows a result for the staining with 100 nM bispecific antibody. In the plots, the horizontal axis represents the fluorescence intensity readings displayed on the flow cytometer, and the vertical axis indicates the relative cell count.

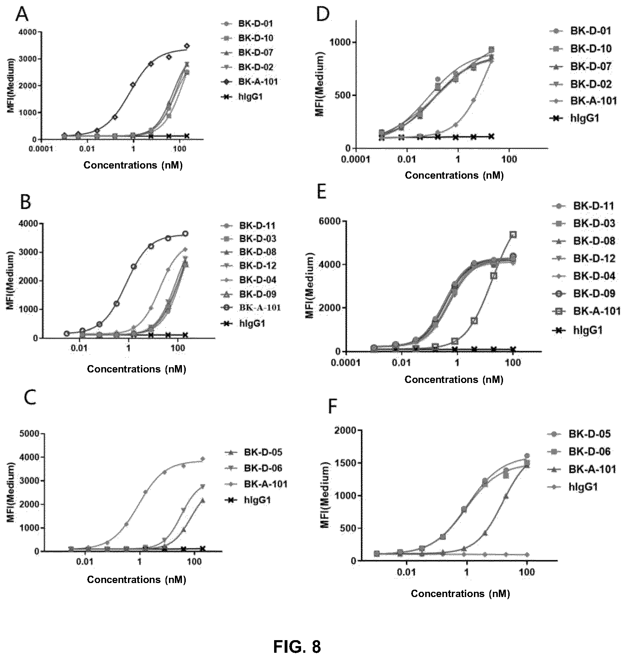

FIG. 8 shows results for the affinities of the bispecific antibodies provided according to Example 1 of the present disclosure for the TT3 end and CD3 end, wherein A, B, and C show results for the affinities of different bispecific antibodies for the CD3 end, and D, E, and F show results for the affinities of different bispecific antibodies for the TT3 end.

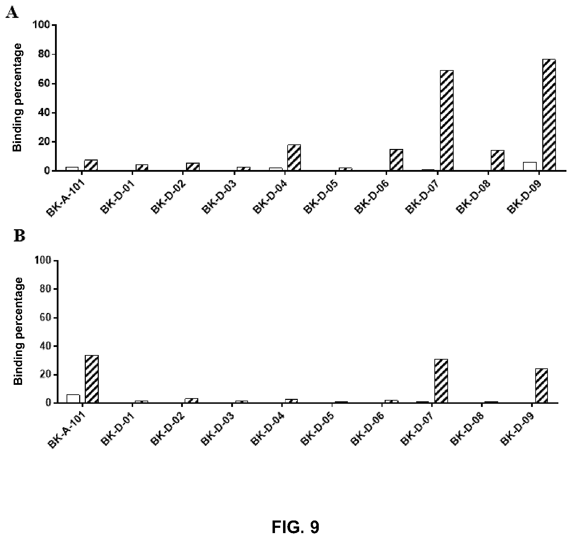

FIG. 9 shows FACS assay results for the binding specificity of bispecific antibodies to wild-type tumor cell strain HCT116 (shown in A of FIG. 9) and MC38 (shown in B of FIG. 9) provided according to Example 1 of the present disclosure. The concentrations of the bispecific antibodies are 10 nM (white) and 100 nM (black slash). In the plots, the horizontal axis represents different monoclonal antibodies or bispecific antibodies, and the vertical axis indicates the percentage of antibody binding.

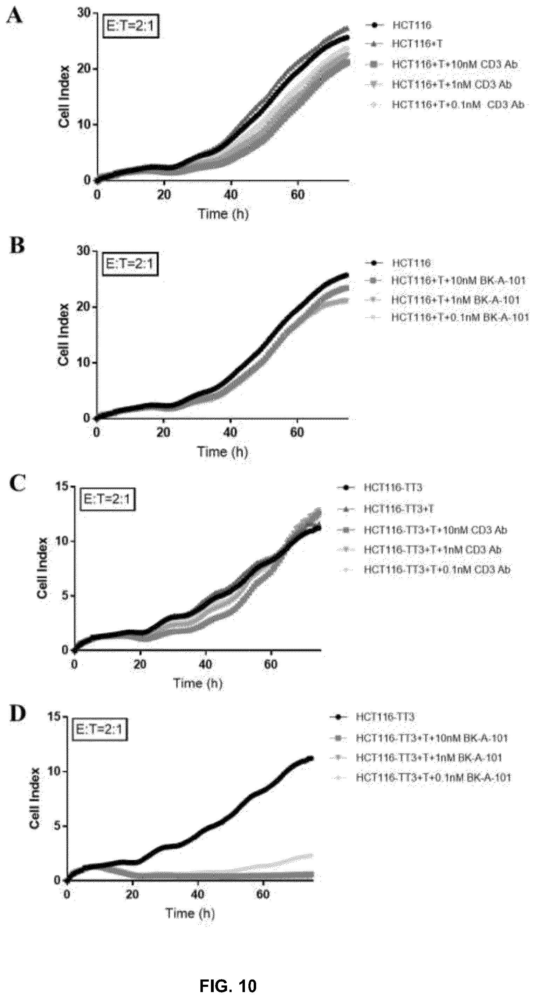

FIG. 10 shows results for an assessment of the killing effect of a bispecific antibody, combined *in vitro* with pre-activated expanded T cells, using an RTCA real-time killing system provided according to Example 2 of the present disclosure. In the plots, the horizontal axis represents time in hours, with the plating of the tumor cell strain as the starting point; the vertical axis represents the cell growth index; the effector cell:the target cell (E:T) = 2:1; and each point shown in the figure is the mean value of duplicate wells.
A of FIG. 10 shows results for the killing of wild-type cell strain HCT116 by an anti-CD3 monoclonal antibody, combined *in vitro* with T cells, wherein the black circle represents the HCT116 group (HCT116), the dark gray triangle represents the HCT116 + T cell group (HCT116 + T), the gray square represents the HCT116 + T cell + 10 nM anti-CD3 monoclonal antibody group (HCT116 + T + 10 nM CD3 Ab), the light gray inverted triangle represents the HCT116 + T cell + 1 nM anti-CD3 monoclonal antibody group (HCT116 + T + 1 nM CD3 Ab), and the grayish-white diamond represents the HCT116 + T cell + 0.1 nM anti-CD3 monoclonal antibody group (HCT116 + T + 0.1 nM CD3 Ab).
B of FIG. 10 shows results for the killing of wild-type cell strain HCT116 by the bispecific antibody, combined *in vitro* with T cells, wherein the black circle represents the HCT116 group (HCT116), the gray square represents the HCT116 + T cell + 10 nM bispecific antibody group (HCT116 + T + 10 nM BK-A-101), the light gray inverted triangle represents the HCT116 + T cell + 1 nM bispecific antibody group (HCT116 + T + 1 nM BK-A-101), and the grayish-white diamond represents the HCT116 + T cell + 0.1 nM bispecific antibody group (HCT116 + 0.1 nM + BK-A-101).
C of FIG. 10 shows results for the killing of cell strain HCT116-TT3 by the anti-CD3 monoclonal antibody, combined *in vitro* with T cells, wherein the black circle represents the HCT116-TT3 group (HCT116-TT3), the dark gray triangle represents the HCT116-TT3 + T cell group (HCT116-TT3 + T), the gray square represents the HCT116-TT3 + T cell + 10 nM anti-CD3 monoclonal antibody group (HCT116-TT3 + T + 10 nM CD3 Ab), the light gray inverted triangle represents the HCT116-TT3 + T cell + 1 nM anti-CD3 monoclonal antibody group (HCT116-TT3 + T + 1 nM CD3 Ab), and the grayish-white diamond represents the HCT116-TT3 + T cell + 0.1 nM anti-CD3 monoclonal antibody group (HCT116-TT3 + T + 0.1 nM CD3 Ab).
D of FIG. 10 shows results for the killing of cell strain HCT116-TT3 by the bispecific antibody, combined *in vitro* with T cells, wherein the black circle represents the HCT116-TT3 group (HCT116-TT3), the gray square represents the HCT116-TT3 + T cell + 10 nM bispecific antibody group (HCT116-TT3 + T + 10 nM BK-A-101), the light gray inverted triangle represents the HCT116-TT3 + T cell + 1 nM bispecific antibody group (HCT116-TT3 + T + 1 nM BK-A-101), and the grayish-white diamond represents the HCT116-TT3 + T cell + 0.1 nM bispecific antibody group (HCT116-TT3 + T + 0.1 nM BK-A-101).

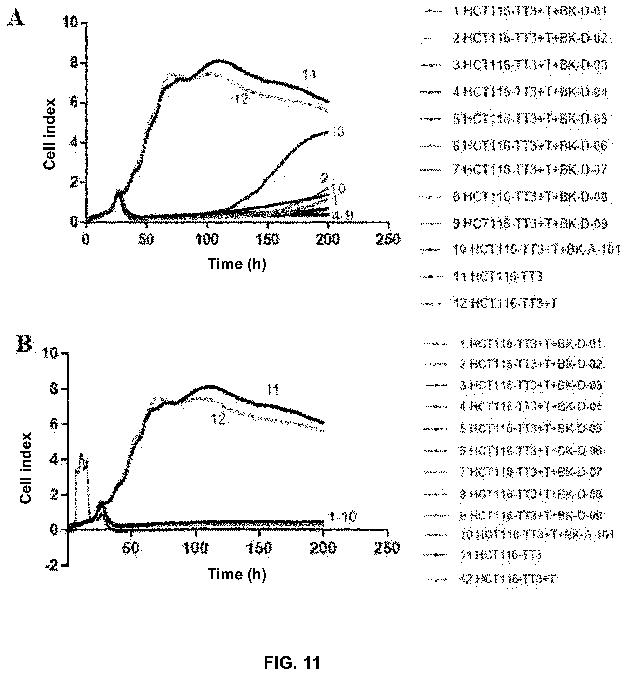

FIG. 11 shows RTCA assay results for the killing effects of bispecific antibodies, combined *in vitro* with T cells, provided according to Example 2 of the present disclosure. In the plots, the horizontal axis represents time in hours, with the plating of the tumor cell strain as the starting point; the vertical axis represents the cell growth index; and the effector cell:the target cell (E:T) = 5:1. The concentration of the bispecific antibodies in plot A is 0.1 nM, and the concentration of the bispecific antibodies in plot B is 1 nM. Labels 1-9 represent the groups of HCT116-TT3 + T cells + bispecific antibodies BK-D-01 to BK-D-09, respectively, label 10 represents the HCT116-TT3 + T cell + BK-A-101 group, label 11 represents the HCT116-TT3 cells only, without the treatment with bispecific antibodies and T cells, and label 12 represents the HCT116-TT3 cells treated with T cells only, without the addition of the bispecific antibodies.

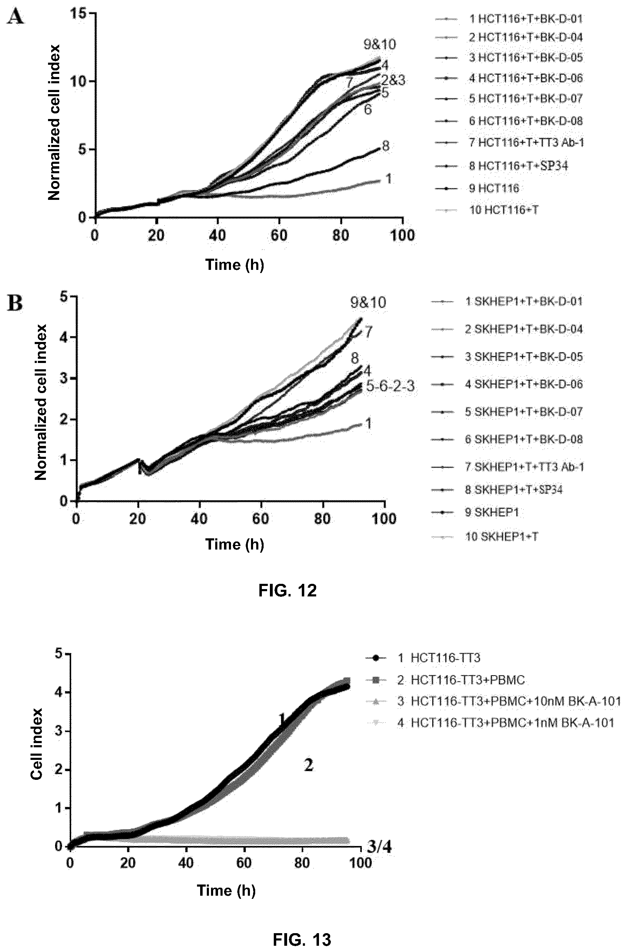

FIG. 12 shows RTCA assay results for the killing effects of bispecific antibodies, combined *in vitro* with T cells, provided according to Example 2 of the present disclosure. In the plots, the horizontal axis represents time in hours, with the plating of the tumor cell strain as the starting point; the vertical axis represents the cell growth index; and the effector cell:the target cell (E:T) = 5:1. In plot A, the concentration of the bispecific antibodies is 100 nM, and the tumor cell strain is cell strain HCT116; and in plot B, the concentration of the bispecific antibodies is 10 nM, and the tumor cell strain is cell strain SKHEP1, wherein labels 1-6 represent the groups of the wild-type tumor cell strain + T cells + bispecific antibodies BK-D-01, 04, 05, 06, 07, and 08, respectively, label 7 represents the wild-type tumor cell strain + T cell + TT3 monoclonal antibody-1 group, label 8 represents the wild-type tumor cell strain + T cell + CD3 monoclonal antibody SP34 group, label 9 represents the wild-type tumor cell strain cells only, without the treatment with antibodies and T cells, and label 10 represents the wild-type tumor cell strain cell treated with T cells only, without the addition of the antibodies.
FIG. 13 shows RTCA assay results for the killing effect of a bispecific antibody, combined *in vitro* with PMBCs, provided according to Example 3 of the present disclosure. In the plot, the horizontal axis represents time in hours, with the plating of the tumor cell strain as the starting point; the vertical axis represents the cell growth index; the effector cell:the target cell (E:T) = 2:1; and each point shown in the figure is the mean value of duplicate wells. The black circle (label 1) represents the HCT116-TT3 group (HCT116-TT3), the dark gray square (label 2) represents the HCT116-TT3 + PBMC group (HCT116-TT3 + PBMC), the gray triangle (label 3) represents the HCT116-TT3 + PBMC + 10 nM bispecific antibody group (HCT116-TT3 + PBMC + 10 nM BK-A-101), and the grayish-white inverted triangle (label 4) represents the HCT116-TT3 + PBMC + 1 nM bispecific antibody group (HCT116-TT3 + PBMC + 1 nM BK-A-101).

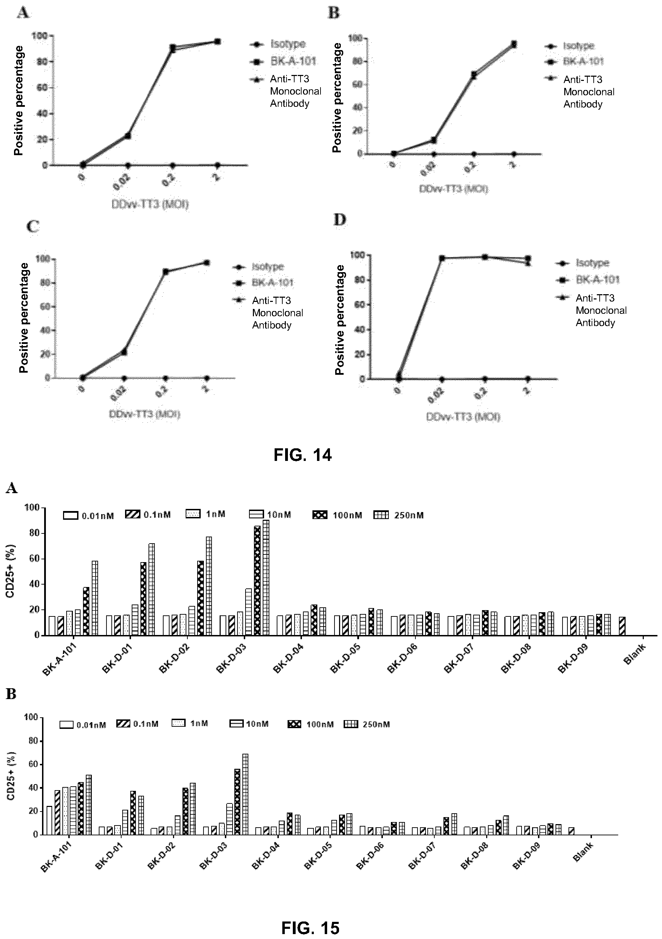

FIG. 14 shows FACS assay results for the binding activity of bispecific antibody BK-A-101 to tumor cell strains infected with DDvv-TT3 provided according to Example 4 of the present disclosure. Plot A shows results for the human pancreatic cancer cell strain (PANC-1), plot B shows results for the human melanoma cell strain (A375), plot C shows results for the human glioma cell strain (U251), and plot D shows results for the human ovarian cancer cell strain (SKOV3). In the plots, the horizontal axis represents different MOIs of DDvv-TT3, and the vertical axis represents the percentage of flow cytometry positive cells in the cell population. The circle represents the detection results of isotype control antibody staining (negative control), the square represents the detection results of bispecific antibody staining (BK-A-101), and the triangle represents the detection results of commercial antibody (GenScript) staining (positive control).
FIG. 15 shows FACS assay results for the stimulation of the activation of T cells and PBMCs cultured *in vitro* by bispecific antibodies provided according to Example 5 of the present disclosure. The concentrations of the bispecific antibodies are 0.01 nM (white), 0.1 nM (black slash), 1 nM (black dot), 10 nM (black horizontal bar), 100 nM (black cross-bar), and 250 nM (black grid). Plot A shows results for T cells cultured *in vitro,* and plot B shows results for PBMCs. The horizontal axis represents different bispecific antibodies, and the vertical axis indicates the expression percentage of the early activation marker CD25 on the surface of T cells.

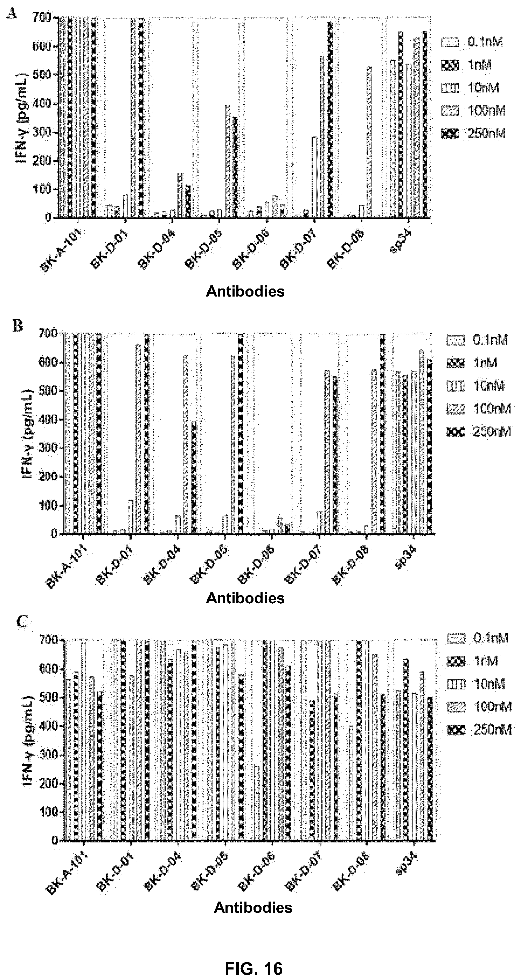

FIG. 16 shows the IFN-γ contents in the supernatants after 3 days of co-culture of the bispecific antibodies and PBMCs as measured by ELISA provided according to Example 5 of the present disclosure. The concentrations of the bispecific antibodies are 0.1 nM (black dot), 1 nM (black grid), 10 nM (black vertical bar), 100 nM (black slash), and 250 nM (black cross-bar). Plot A shows supernatant assay results for bispecific antibodies co-cultured with PBMCs for 3 days, plot B shows supernatant assay results for bispecific antibodies co-cultured with PBMCs and cell strain SKHEP1 for 3 days, and plot C shows supernatant assay results for bispecific antibodies co-cultured with PBMCs and cell strain SKHEP1-TT3 for 3 days. In the plots, the horizontal axis represents the CD3 monoclonal antibody SP34 and different bispecific antibodies BK-A-101 as well as BK-D-01, 04, 05, 06, 07, and 08, and the vertical axis indicates the IFN-γ contents in the co-culture supernatants (pg/mL).

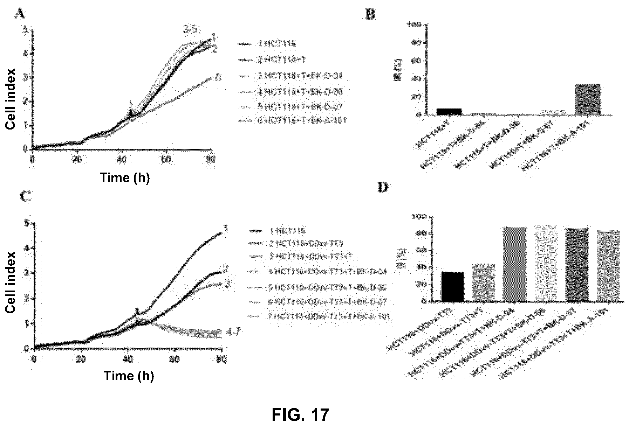

FIG. 17 shows RTCA assay results for the killing effects of bispecific antibodies, combined *in vitro* with T cells and a recombinant oncolytic poxvirus, provided according to Example 6 of the present disclosure. Plots A and C show *in vitro* real-time killing results. In the plots, the horizontal axis represents time in hours, with the plating of the tumor cell strain as the starting point, and the vertical axis represents the cell growth index. The effector cell:the target cell (E:T) = 10:1, the concentration of the bispecific antibodies is 1 nM, and the MOI of the recombinant oncolytic poxvirus DDvv-TT3 is 1. Plots B and D show tumor inhibition rate results at the 80-hour time point of *in vitro* real-time killing, wherein the horizontal axis represents different experimental groups, and the vertical axis represents the tumor growth inhibition rate. Plots A and B show results for wild-type cell strain HCT116, and plots C and D show results for wild-type cell strain HCT116 + DDvv-TT3. In plot A, label 1 represents the HCT116 group, label 2 represents the HCT116 + T cell group, labels 3-5 represent groups of HCT116 + T cells + bispecific antibodies BK-D-04, 06, and 07, and label 6 represents HCT116 + T cell + BK-A-101; and in plot C, label 1 represents the HCT116 group, label 2 represents the HCT116 + DDvv-TT3 group, dark label 3 represents the HCT116 + DDvv-TT3 + T group, labels 4-6 represent groups of HCT116 + DDvv-TT3 + T cells + bispecific antibodies BK-D-04, 06, and 07, and label 7 represents the HCT116 + DDvv-TT3 + T cell + BK-A-101 group.

It should be noted that the same numerical labels (e.g., 1, 2, 3, etc.) in FIGs. 11, 12, 3, 17, and the like do not correspond to the exact same contents, and the meaning of the numerical labels in each accompanying drawing is listed above.

### DETAILED DESCRIPTION

The technical solutions of the present disclosure are described in detail below with reference to specific examples. Meanwhile, to facilitate understanding for a person skilled in the art, some terms of the present disclosure are explained and described. It should be noted that these explanations and descriptions are merely used to facilitate understanding for a person skilled in the art and should not be construed as limiting the protection scope of the present disclosure.

As used in this specification and the claims, the terms "a", "an", and "the" include plural referents unless the context clearly dictates otherwise. The words "tumor", "cancer", "tumor cell", and "cancer cell" encompass meanings commonly recognized in the art.

The word "oncolytic virus" as used herein refers to a virus that is capable of selectively replicating in and lysing tumor cells.

The term "therapeutically effective amount" as used herein refers to an amount of a drug or formulation or active ingredient capable of exhibiting a detectable therapeutic or inhibitory effect. The detectable therapeutic or inhibitory effect mentioned herein may be detected by any detection method known in the art.

The terms "first", "second", and "third" are used for descriptive purposes only and should not be construed as indicating or implying relative importance or implicitly indicating the number of indicated technical features, nor should they be used to indicate an order of appearance. For example, a first composition and a second composition, and a first active ingredient and a second active ingredient, do not indicate an order of appearance, nor do they signify importance, but are used for distinction only.

The term "MOI", or "multiplicity of infection" as used herein, i.e., a ratio between the number of viruses and the number of cells, refers to the number of virus particles used to initiate viral infection per cell. MOI = pfu/cell. PFU refers to plaque forming unit and is used to describe the number of virus particles with infectious activity.

The present disclosure provides a therapeutic agent comprising:
(a) a first composition comprising a first active ingredient, wherein the first active ingredient comprises or contains a nucleic acid having a labeling polypeptide encoding sequence for being introduced into a tumor cell and/or a cancer cell; the labeling polypeptide has an extracellular antigen determining region, a spacer portion, and a transmembrane portion that are operably linked; an amino acid sequence of the extracellular antigen determining region comprises the amino acid sequences of one or more antigenic epitope polypeptides; and in a natural state, an amino acid sequence of a cell membrane protein or a secreted protein of a mammal does not comprise the amino acid sequence of the antigenic epitope polypeptide; and
(b) a second composition comprising a second active ingredient, wherein the second active ingredient comprises a multispecific antibody, and the multispecific antibody comprises at least a first antigen-binding moiety and a second antigen-binding moiety, wherein the first antigen-binding moiety is capable of specifically recognizing and binding to the extracellular antigen determining region of the labeling polypeptide, and
   the second antigen-binding moiety is capable of specifically recognizing and binding to an immune cell antigen or a first cytokine or a receptor thereof; and
   the multispecific antibody may also optionally comprise a third antigen-binding moiety specifically recognizing and binding to a tumor antigen or an immune checkpoint or a second cytokine or a receptor thereof.

For easier understanding, FIG. 1 shows a schematic diagram of the therapeutic effect exerted by the provided therapeutic agent. In FIG. 1, an IgG-like bispecific antibody is used as an example, and TT3 is used as an example of a labeling polypeptide for explanation. An antigenic epitope polypeptide not comprised in the amino acid sequence of an artificially synthesized mammalian cell membrane protein or secreted protein (the antigenic epitope polypeptide serves as at least a portion of the extracellular antigen determining region, forming a TT3 labeling polypeptide along with a spacer portion and a transmembrane portion) is carried to a tumor cell and is labeled on the surface of the tumor cell. In one aspect, the bispecific antibody is capable of specifically recognizing and binding to TT3. In another aspect, the bispecific antibody is capable of specifically recognizing and binding to immune cells, for example, T cells and NK cells as shown in the figure. By linking immune cells to tumor cells via the bispecific antibody, redirected tumor lysis is facilitated, and immune cells are activated to produce cytotoxic molecules including perforin and granzymes, which kill tumor cells, thereby exerting specific killing effects. It should be noted that the IgG-like bispecific antibody shown in FIG. 1 is only used as an example, and does not represent that the multispecific antibodies mentioned herein are specific to this IgG-type structure.

When the oncolytic virus is used as a vector, its specific replication within the tumor enables the expression of the labeling polypeptide in tumor cells and/or cancer cells, so that the oncolytic virus can significantly improve the expression of the antigenic epitope peptide on the surface of the tumor cells while killing the tumor cells and/or the cancer cells, and thereby exhibit a synergistic therapeutic effect when combined with immune cells bridged to the multispecific antibody.

### Labeling Polypeptide

The labeling polypeptide mentioned herein is described in the PCT patent application with the International Application No. PCT/CN2019/102480 and the International Publication No. WO2020038490A, which is now incorporated herein in its entirety or in part as desired. The labeling polypeptide mentioned is used for modifying a surface of a tumor cell and/or a cancer cell, has an extracellular antigen determining region, a spacer portion, and a transmembrane portion that are operably linked, and can be expressed to modify the surface of the tumor cell and/or the cancer cell; an amino acid sequence of the extracellular antigen determining region comprises the amino acid sequences of one or more antigenic epitope polypeptides; and in a natural state, an amino acid sequence of a cell membrane protein or a secreted protein of a mammal does not comprise the amino acid sequence of the antigenic epitope polypeptide.

The term "extracellular antigen determining region" as used herein refers to the portion of the labeling polypeptide that is located outside the cell membrane and comprises the antigenic epitope polypeptide when it is expressed on the cell surface.

According to an embodiment of the present disclosure, the amino acid sequence of the antigenic epitope polypeptide is derived from an amino acid sequence of a naturally occurring protein or is an artificially synthesized amino acid sequence that is not naturally occurring. The naturally occurring protein includes mammalian intracellular proteins and proteins of organisms other than mammals. The proteins of organisms other than mammals include viral proteins, bacterial proteins, fungal proteins, protozoan proteins, plant proteins, insect proteins, and proteins from animals other than mammals.

In some embodiments of the present disclosure, the amino acid sequence of the antigenic epitope polypeptide is derived from amino acid sequences of the following tags: a Myc tag, an HA tag, Strep tag I, Strep tag II, a Flag tag, a HAT tag, an S tag, an Sl tag, a protein C tag, a tag-100 tag, an E2 tag, a TAP tag, an HSV tag, a KT3 tag, a V5 tag, a VSV-G tag, a His tag, an RFP tag, or the like. The amino acid sequences and nucleotide sequences of these tags are known and available from public databases commonly used in the art.

In a preferred embodiment of the present disclosure, the amino acid sequence of the antigenic epitope polypeptide is derived from amino acid sequences of the following tags: a human Myc tag (corresponding labeling polypeptide is denoted as TT1), an influenza virus HA tag (corresponding labeling polypeptide is denoted as TT2), Strep tag II (corresponding labeling polypeptide is denoted as TT3), or Strep tag I. The Strep tag I or Strep tag II mentioned has an amino acid sequence of at least 3 amino acids in length, for example, 3 amino acids, 4 amino acids, 5 amino acids, 6 amino acids, 7 amino acids, 8 amino acids, 9 amino acids, 10 amino acids, or 11, 12, 13, 14, or 15 amino acids, and may comprise an amino acid derived from the sequence set forth in NWSHPQFEK (SEQ ID NO: 59) or AWRHPQFGG (SEQ ID NO: 60), and may also be a partial sequence or a combination of partial sequences of the sequence set forth in SEQ ID NO: 59 or SEQ ID NO: 60. More specifically, the amino acid sequence of Strep tag II may be, for example, set forth in WSHPQFEK (SEQ ID NO: 58) and NWSHPQFEK (SEQ ID NO: 59). The amino acid sequence of the Strep tag I mentioned may be, for example, set forth in AWRHPQFGG (SEQ ID NO: 60). Further preferably, the amino acid sequence of the antigenic epitope polypeptide is consistent with a sequence of amino acids at positions 410-419 of the human Myc protein; or the amino acid sequence of the antigenic epitope polypeptide is consistent with a sequence of amino acids at positions 119-127 of the influenza virus HA protein; or the amino acid sequence of the antigenic epitope polypeptide is consistent with Strep tag II (for the detailed description of Strep tag II, see the document "Molecular Interaction Between the Strep-tag Affinity Peptide and its Cognate Target, Streptavidin, Thomas G. M. Schmidt, Jurgen Koepke, Ronald Frank, and Arne Skerra"). In some other embodiments of the present disclosure, the amino acid sequences of the antigenic epitope polypeptides of TT1 and TT2 may extend upstream and downstream of the above sequences by no more than 10 amino acids. The amino acid sequence of the human Myc protein may be the amino acid sequence numbered P01106 isoforml in UniProtKB. The amino acid sequence of the influenza virus HA protein may be the amino acid sequence numbered Q03909 in UniProtKB.

In a specific embodiment, the amino acid sequence of the extracellular antigen determining region of the labeling polypeptide comprises the amino acid sequences of one or more said antigenic epitope polypeptides, wherein when the amino acid sequence of the extracellular antigen determining region of the labeling polypeptide comprises the amino acid sequences of a plurality of the antigenic epitope polypeptides, every two adjacent antigenic epitope polypeptides are operably linked, for example, may be linked using a linker, or may be linked directly without a linker. The amino acid sequence of the linker may be, for example: G (as used in the labeling polypeptides C1&2a and C1&2b), GGS (as used in C1&2a and C1&2b), GGGGSGGGGS (as used in TT1-TT3).

In order to enhance the immunogenicity of the labeling polypeptide, the extracellular antigen determining region of the labeling polypeptide preferably comprises n antigenic epitope polypeptides, wherein n is an integer greater than or equal to 1, for example, n = 1, 2, 3, 4, etc. Preferably, n is an integer of 1-10; further preferably, n is an integer of 2-5; and further preferably, n = 2 or 3. For example, the extracellular antigen determining region of the labeling polypeptide may comprise 3 repeated antigenic epitope polypeptides derived from the Myc tag (see, e.g., TT1), or comprise 3 repeated antigenic epitope polypeptides derived from the HA tag (see, e.g., TT2), or comprise 3 repeated antigenic epitope polypeptides derived from the Strep tag II (see, e.g., TT3), or comprise 3 repeated antigenic epitope polypeptides derived from the Myc tag and 3 repeated antigenic epitope polypeptides derived from the HA tag (see, e.g., C1&2a), or comprise 2 repeated antigenic epitope polypeptides derived from the Myc tag and 2 repeated antigenic epitope polypeptides derived from the HA Tag (see, e.g., C1&2b).

In one embodiment of the present disclosure, the amino acid sequences of the extracellular antigen determining regions are set forth in SEQ ID NO: 1 (corresponding to TT1), SEQ ID NO: 2 (corresponding to TT2), SEQ ID NO: 3 (corresponding to TT3), SEQ ID NO: 4 (corresponding to C1&2a), and SEQ ID NO: 5 (corresponding to C1&2b).

Preferably, the transmembrane portion is derived from the transmembrane region of CD8, CD3ζ, CD4, or CD28 whose full-length amino acid sequence and nucleotide sequence are known and available from public databases commonly used in the art. Further preferably, the transmembrane portion is derived from the transmembrane region of human CD8α. More preferably, the amino acid sequence of the transmembrane portion comprises the amino acid sequence set forth in SEQ ID NO: 7. CD8 is a transmembrane glycosylated membrane protein composed of α and β subunits, which through combined action with the T cell surface receptor enables T cell to bind to a specific antigen. CD8 specifically binds to MHC I and mediates the killing effect of cytotoxic T cells. The transmembrane region is typically a hydrophobic α helix that spans the cell membrane.

In the labeling polypeptide of the present disclosure, the transmembrane portion and the extracellular antigen determining region can be linked by a spacer portion. The structure of this region should be flexible, allowing the extracellular antigen determining region to adapt to different directions, thereby promoting the recognition and binding of the corresponding multispecific antibody. The simplest form of the spacer portion is a hinge region of immunoglobulin IgG1, and can also be a portion of an immunoglobulin CH₂CH₃ region. It has been found through researches and experimental investigations that the spacer portion is preferably derived from a hinge region of CD8α, and the transmembrane region is preferably derived from a transmembrane region of CD8α. Preferably, the amino acid sequence of the spacer portion is set forth in SEQ ID NO: 6. Further preferably, the spacer portion and the transmembrane portion constitute a spacer transmembrane portion, and an amino acid sequence of the spacer transmembrane portion is consistent with a sequence of amino acids at positions Y-210 of CD8α, and 118 ≤ Y ≤ 128, Y being an integer. The UniProtKB numbering of the amino acid sequence of CD8α may be P01732. That is, the amino acid sequence of the spacer transmembrane portion is preferably selected from amino acids at positions 118-210 of CD8α and comprises amino acids at positions 128-210. For example, the amino acid sequence of the spacer transmembrane portion is shown as any one of the amino acid sequences of the following amino acid sequence group: amino acids at positions 118-210, amino acids at positions 119-210, amino acids at positions 120-210, amino acids at positions 121-210, amino acids at positions 122-210, amino acids at positions 123-210, amino acids at positions 124-210, amino acids at positions 125-210, amino acids at positions 126-210, amino acids at positions 127-210, or amino acids at positions 128-210 of CD8α.

The terms "spacer portion" and "transmembrane portion" used herein are known in the art. For details, please refer to "'Immunology introductory theory', Yu Shanqian, Higher Education Press, 2008"; and "'Immunobiology', Seventh edition, Kenneth Murphy, Paul Travers, Mark Walport, et al.".

In the nucleic acid having the labeling polypeptide encoding sequence of the present disclosure, a signal peptide encoding sequence is preferably comprised before the 5' end of the labeling polypeptide encoding sequence, and the signal peptide has a function of guiding the secretion of a target protein to the cell surface. The present disclosure has found that a combination of the extracellular antigen determining region with the signal peptide from a GM-CSFα chain allows the labeling polypeptide to be expressed on the surface of tumor cells. The GM-CSFα chain signal peptide is a leader sequence for targeting the labeling polypeptide of the present disclosure to a secretory pathway, and an encoding sequence of which is first translated into a protein in a cell together with the encoding sequence of the labeling polypeptide and guides the synthesized protein into an intracellular secretion pathway. The signal peptide is removed prior to expression of the labeling polypeptide on the cell surface. The full-length amino acid sequence and nucleotide sequence of the GM-CSFα chain are known and available from public databases commonly used in the art. Preferably, the amino acid sequence of the signal peptide is selected from amino acids at positions 1-22 of a human GM-CSFα chain. More preferably, the amino acid sequence of the signal peptide is set forth in SEQ ID NO: 8. The amino acid sequence of the GM-CSFα chain is derived from UniProtKB-P15509.

In a specific embodiment of the present disclosure, the labeling polypeptide comprises the following amino acid sequences operably linked in serial order: an extracellular antigen determining region (which includes the amino acid sequences of one or more said antigenic epitope polypeptides), a spacer portion, and a transmembrane portion. In a more specific embodiment of the present disclosure, the amino acid sequence of the antigenic epitope polypeptide of the extracellular antigen determining region is derived from the following amino acid sequences of the antigenic epitope polypeptides among the polypeptides: a Myc tag derived from human intracellular protein Myc, an HA tag derived from an influenza virus HA protein, and Strep tag II that is not naturally occurring. The spacer portion is derived from a human CD8α hinge region, and the transmembrane portion is derived from a human CD8α transmembrane region.

As described above, a linkage is operably made between the signal peptide and the extracellular antigen determining region, between the extracellular antigen determining region and the spacer portion, and between the spacer portion and the transmembrane portion, for example, may be a linkage using a linker, or may be a direct linkage without a linker. In one embodiment of the present disclosure, the signal peptide and the extracellular antigen determining region are linked by a linker, for example, GAHADITS (as used in TT1-TT3), GAHAAQLTLTKGNK (as used in Cl&2a), and GAHA (as used in Cl&2b). The extracellular antigen determining region and the spacer portion are linked by a linker, for example,-Ala-Ser- and G, and the spacer portion and the transmembrane portion are linked directly without a linker.

The present disclosure has further found that the labeling polypeptide can express two kinds of antigenic epitope polypeptides simultaneously, and specifically, the present disclosure has designed labeling polypeptide Cl&2a with an antigen determining region thereof comprising three repeats of each of Myc tag and HA tag. The present disclosure has further designed labeling polypeptide Cl&2b with an antigen determining region thereof comprising two repeats of each of Myc tag and HA tag; and to further stabilize expression of the labeling polypeptide on a cell membrane surface, a spacer region of TIGIT is added to a spacer portion of Cl&2b, wherein an amino acid sequence of the added spacer region is consistent with a sequence of amino acids at positions 24-140 of TIGIT. The amino acid sequence of TIGIT is derived from UniProtKB-Q495Al. More preferably, an amino acid sequence of the added TIGIT spacer is set forth in SEQ ID NO: 9, and the amino acid sequence of the spacer transmembrane portion of Cl&2b is set forth in SEQ ID NO: 10.

| SEQ ID NO: | Sequence |
|---|---|
| 1 | EQKLISEEDLGGGGSGGGGSEQKLISEEDLGGGGSGGGGSEQKLISEEDL |
| 2 | YPYDVPDYAGGGGSGGGGSYPYDVPDYAGGGGSGGGGSYPYDVPDYA |
| 3 | NWSHPQFEKGGGGSGGGGSNWSHPQFEKGGGGSGGGGSNWSHPQFEK |
| 4 | EQKLISEEDLGEQKLISEEDLGEQKLISEEDLGGSYPYDVPDYAGYPYDV PDYAGYPYDVPDYA |
| 5 | EQKLISEEDLGEQKLISEEDLGGSYPYDVPDYAGYPYDVPDYA |
| 6 | FVPVFLPAKPTTTPAPRPPTPAPTIASQPLSLRPEACRPAAGGAVHTRGLD FACD |
| 7 | IYIWAPLAGTCGVLLLSLVITLYCNHRN |
| 8 | MLLLVTSLLLCELPHPAFLLIP |
| 9 | TGTIETTGNISAEKGGSIILQCHLSSTTAQVTQVNWEQQDQLLAICNADL GWHISPSFKDRVAPGPGLGLTLQSLTVNDTGEYFCIYHTYPDGTYTGRIF LEVLESSVAEHGARFQI |
| 10 | TGTIETTGNISAEKGGSIILQCHLSSTTAQVTQVNWEQQDQLLAICNADL GWHISPSFKDRVAPGPGLGLTLQSLTVNDTGEYFCIYHTYPDGTYTGRIF LEVLESSVAEHGARFQIASFVPVFLPAKPTTTPAPRPPTPAPTIASQPLSLR PEACRPAAGGAVHTRGLDFACDIYIWAPLAGTCGVLLLSLVITLYCNHR N |

Preferably, the amino acid sequence of the labeling polypeptide is set forth in SEQ ID NO: 11 (corresponding to TT1), SEQ ID NO: 12 (corresponding to TT2), SEQ ID NO: 13 (corresponding to TT3), SEQ ID NO: 14 (corresponding to C1&2a), or SEQ ID NO: 15 (corresponding to C1&2b).
SEQ ID NO:11:

EQKLISEEDLGGGGSGGGGSEQKLISEEDLGGGGSGGGGSEQKLISEEDLG

GGGSGGGGSASFVPVFLPAKPTTTPAPRPPTPAPTIASQPLSLRPEACRPAAGG
AVHTRGLDFACDIYIWAPLAGTCGVLLLSLVITLYCNHRN

The boldface shows the antigenic epitope polypeptide, the gray portion shows the spacer portion from the CD8 hinge region, and the underline shows the transmembrane portion from the CD8 transmembrane region.
SEQ ID NO: 12:

YPYDVPDYAGGGGSGGGGSYPYDVPDYAGGGGSGGGGSYPYDVPDYAGG
GGSGGGGSASFVPVFLPAKPTTTPAPRPPTPAPTIASQPLSLRPEACRPAAGGA
VHTRGLDFACDIYIWAPLAGTCGVLLLSLVITLYCNHRN

The boldface shows the antigenic epitope polypeptide, the gray portion shows the spacer portion from the CD8 hinge region, and the underline shows the transmembrane portion from the CD8 transmembrane region.
SEQ ID NO: 13:

NWSHPQFEKGGGGSGGGGSNWSHPQFEKGGGGSGGGGSNWSHPQFEKG
GGGSGGGGSASFVPVFLPAKPTTTPAPRPPTPAPTIASQPLSLRPEACRPAAGG
AVHTRGLDFACDIYIWAPLAGTCGVLLLSLVITLYCNHRN

The boldface shows the antigenic epitope polypeptide, the gray portion shows the spacer portion from the CD8 hinge region, and the underline shows the transmembrane portion from the CD8 transmembrane region.
SEQ ID NO: 14:

EQKLISEEDLGEQKLISEEDLGEQKLISEEDLGGSYPYDVPDYAGYPYDVP
DYAGYPYDVPDYAASFVPVFLPAKPTTTPAPRPPTPAPTIASQPLSLRPEACRP
AAGGAVHTRGLDFACDIYIWAPLAGTCGVLLLSLVITLYCNHRN

The boldface shows the antigenic epitope polypeptide, the gray portion shows the spacer portion from the CD8 hinge region, and the underline shows the transmembrane portion from the CD8 transmembrane region.
SEQ ID NO: 15:

EQKLISEEDLGEQKLISEEDLGGSYPYDVPDYAGYPYDVPDYAGTGTIETT
GNISAEKGGSIILQCHLSSTTAQVTQVNWEQQDQLLAICNADLGWHISPSFKD
RVAPGPGLGLTLQSLTVNDTGEYFCIYHTYPDGTYTGRIFLEVLESSVAEHGA
RFQIASFVPVFLPAKPTTTPAPRPPTPAPTIASQPLSLRPEACRPAAGGAVHTRG
LDFACDIYIWAPLAGTCGVLLLSLVITLYCNHRN

The boldface shows the antigenic epitope polypeptide, the dark gray portion shows the portion from the TIGIT spacer region in the spacer portion, the light gray portion shows the portion from the CD8 hinge region in the spacer portion, and the underline shows the transmembrane portion from the CD8 transmembrane region.

### Nucleic Acid Having Labeling Polypeptide Encoding Sequence

In obtaining the labeling polypeptide encoding sequence above, encoding sequences encoding these labeling polypeptides can be obtained using rules common in the art. The nucleic acid having a labeling polypeptide encoding sequence includes a DNA or an RNA; and the RNA comprises an mRNA transcribed from the DNA. Preferably, the nucleic acid comprises in sequence a promoter and the labeling polypeptide encoding sequence that are operably linked. Examples of the promoter include any promoters known in the art for promoting the transcription of a downstream DNA sequence, for example, a CMV promoter, a T7 promoter, and the like. The labeling polypeptide encoding sequence above is also described in detail in the PCT patent application with the International Application No. PCT/CN2019/102480 and the International Publication No. WO2020038490A, and will not be reiterated in detail herein.

### Recombinant Virus

In one embodiment of the present disclosure, the first active ingredient is a recombinant virus, and a genome of the recombinant virus has a promoter, a signal peptide encoding sequence, and the labeling polypeptide encoding sequence that are operably linked in sequence, wherein the recombinant virus includes a replication-selective recombinant oncolytic virus or a replication-deficient recombinant virus. The labeling polypeptide encoding sequence encodes the labeling polypeptide.

Preferably, the recombinant oncolytic virus is derived from a genetically mutated virus having an oncolytic effect and a wild-type virus having an oncolytic effect; preferably, the recombinant oncolytic virus is derived from an adenovirus, a poxvirus, a herpes simplex virus, a measles virus, a Semliki forest virus, a vesicular stomatitis virus, a poliovirus, a retrovirus, a reovirus, a Seneca valley virus, an Echo-type enterovirus, a Coxsackie virus, a Newcastle disease virus, and a Malaba virus having an oncolytic effect.

Oncolytic virus therapy has a long history, and a wide variety of viruses have been modified for oncolytic virus development, including an adenovirus, a herpesvirus, a poxviruses, a picornavirus, a paramyxovirus, a reovirus, a parvovirus, and a rhabdovirus. Up to now, several oncolytic viruses (H101, T-VEC, and Delytact) have been approved for clinical tumor treatment. Oncolytic viruses are artificially modified viruses whose pathogenic genes have been knocked out, and thus they are basically unable to replicate in normal tissue cells, but can selectively replicate in tumor cells to exert oncolytic effects. Taking poxvirus as an example, oncolytic poxvirus is generally constructed by knocking out the endogenous thymidine kinase (TK) gene of poxvirus or double knocking out TK and vaccinia growth factor (VGF) genes using homologous recombination technology by means of genetic bioengineering. TK is one of the key enzymes in DNA synthesis, and poxvirus needs to use TK to form a high-concentration nucleic acid pool during the replication process to complete the smooth replication of progeny. The poxvirus often promotes the proliferation of the host and surrounding cells through VGF gene products to achieve its own replication and proliferation, while the damage to normal cells of the poxvirus with deletion of VGF is greatly reduced. Due to the deletion of TK and VGF genes, the replication ability of the virus in normal cells is restricted and expression of an exogenous gene inserted into an oncolytic virus vector will also be greatly restricted. However, the TK gene is in a high-expression state in most tumor cell environments, which provides a favorable environment for the replication and packaging of the poxvirus, enabling the poxvirus to selectively replicate within tumor cells and exert oncolytic functions and the high expression of the exogenous gene inserted into the oncolytic virus vector. In addition to the direct oncolytic effect, the oncolytic virus can also make tumor cells release holographic tumor antigens to activate the inherent anti-tumor immunity of the body and have a certain regulatory effect on the tumor microenvironment simultaneously.

In the present disclosure, the labeling polypeptide encoding sequence is inserted into the genome of the oncolytic virus, and the ability of the oncolytic virus to specifically replicate within tumor cells allows the introduction of the labeling polypeptide encoding nucleic acid into tumor cells and/or cancer cells, such that while the oncolytic virus exerts its killing effect on tumor cells and/or cancer cells, the exogenous antigenic epitope peptide is expressed on the surface of tumor cells, and a synergistic therapeutic effect is achieve through the combination with the multispecific antibody. As the oncolytic virus destroys the microenvironment of the tumor, the therapeutic effect of the multispecific antibody can be further improved, thereby improving the treatment effectiveness of a solid tumor. In addition, the multispecific antibody can also effectively eliminate tumor cells that are infected by the oncolytic virus but fail to complete a replication cycle and generate a sufficient quantity of progeny viruses to be lysed by recruiting immune cells; thereby achieving a further synergistic effect. In addition, the antigens released by the tumor cells lysed by the oncolytic virus can further activate the anti-tumor immunity of the organism, such that a better tumor killing effect can be achieved compared with that of the independent use of the oncolytic virus or the multispecific antibody, and a synergistic therapeutic effect is achieved.

In one embodiment of the present disclosure, a nucleic acid of the labeling polypeptide encoding sequence above may be inserted into the genome of the resulting replication-selective recombinant oncolytic poxvirus.

Preferably, the recombinant oncolytic virus is a recombinant oncolytic poxvirus that is functionally defective in the TK gene and the VGF gene. The TK gene can be functionally defective by inserting an exogenous nucleotide sequence. The VGF gene can be functionally defective by gene knockout or inserting an exogenous nucleotide sequence, but it is preferable to knock out the VGF gene.

The term "functional defect" or "functionally defective" used herein when referring to the gene of an oncolytic virus means that the oncolytic virus is unable to exert the function that the gene should have, that is, the function is lost. This purpose can be achieved by, for example, inserting an extraneous fragment into the gene or knocking out the gene. Further preferably, the recombinant oncolytic poxvirus is a Wyeth strain or a WR strain.

The labeling polypeptide encoding sequence can be inserted into the TK gene to obtain the recombinant oncolytic virus of the present disclosure. The labeling polypeptide encoding sequence can also be inserted into the VGF gene to obtain the recombinant oncolytic virus of the present disclosure.

In a preferred embodiment, the recombinant oncolytic poxvirus is obtained by genetically modifying a VSC20 poxvirus. The VSC20 poxvirus is a poxvirus with deletion of a VGF gene and can be prepared by the methods described in the scientific literature: "McCart, JA, et al. Systemic cancer therapy with a tumor-selective vaccinia virus mutant lacking thymidine kinase and vaccinia growth factor genes. Cancer Res (2001) 61: 8751-8757.". The genetic modification includes inserting an exogenous labeling polypeptide encoding sequence into the TK gene of the VSC20 poxvirus to make the TK gene functionally defective.

The genome of the recombinant virus may also integrate other exogenous genes, for example, an exogenous screening gene which includes a puromycin gene, a gpt gene, and/or a LacZ gene. The exogenous screening gene can be knocked out by a gene knockout system (e.g., LoxP) at the time of purifying the virus comprising the exogenous labeling polypeptide encoding gene.

In some embodiments, the present disclosure adopts the poxvirus early/late promoter p7.5 to control the exogenous screening gene and the artificially synthetized poxvirus early promoter pSEL to control the signal peptide sequence and the exogenous labeling polypeptide encoding sequence, and inserts the exogenous screening gene encoding sequence, the signal peptide encoding sequence, and the labeling polypeptide encoding sequence into the TK gene region of the poxvirus VSC20 strain through *in vitro* intracellular recombination technology to construct an oncolytic virus. The two promoters each initiate expression of their respective regulatory genes in a back-to-back manner, separately.

In another embodiment of the present disclosure, Crisper-Cas9 gene editing technology is used to construct a recombinant oncolytic poxvirus. Preferably, the Crisper-Cas9 gene editing method can perform cutting and homologous recombination at two sites simultaneously so as to achieve the purpose of knocking out a sequence and inserting a transgene at one time.

Preferably, the Crisper-Cas9 gene editing method can cut a target sequence and a donor sequence simultaneously to improve the efficiency of homologous recombination. The design of cutting and homologous recombination by the Crisper-Cas9 gene editing method is also described in detail in the PCT patent application with the International Application No. PCT/CN2019/102480 (International Publication No. WO2020038490A), and will not be reiterated in detail herein.

### Multispecific Antibody

The term "antibody" is used herein in its broadest sense to refer to a protein or polypeptide that comprises an antigen-binding site or antigen-binding moiety or antigen-binding fragment, and to encompass natural and artificial antibodies of various structures, including but not limited to intact antibody forms or antigen-binding fragments of antibodies.

"Intact antibody" or "complete antibody" or the like is used herein to present the same meaning-a protein that comprises at least two heavy chains (H) and two light chains (L) that are linked to each other by disulfide bonds. Each heavy chain consists of a heavy chain variable region (abbreviated as VH) and a heavy chain constant region (also referred to as a heavy chain constant domain, abbreviated as CH). The heavy chain constant region comprises a heavy chain constant domain (CH1), a heavy chain constant domain (CH2), and a heavy chain constant domain (CH3). Each light chain consists of a light chain variable region (abbreviated as VL) and a light chain constant region (also referred to as a light chain constant domain, abbreviated as CL). The VH and VL can be further divided into complementarity-determining regions (also referred to as hypervariable regions, abbreviated as CDRs or HVRs) with conserved framework regions (FRs) interposed therebetween. Each VH and VL comprises three CDRs and four FRs, which are arranged from the amino-terminus (N-terminus) to the carboxy-terminus (C-terminus) as follows: FR1, CDR1, FR2, CDR2, FR3, CDR3, and FR4. Starting from the amino terminus, the CDRs of the heavy chain variable region are referred to as HCDR1, HCDR2, and HCDR3. Starting from the amino terminus, the CDRs of the light chain variable region are referred to as LCDR1, LCDR2, and LCDR3.

"Antigen-binding fragment", "antigen-binding moiety", or "antigen-binding site" refers to comprising a portion of the full-length antibody, and the portion exhibits specificity in binding to an antigen. As mentioned above, the hypervariable regions of the heavy chain and the light chain of an intact antibody exhibit specific binding to an antigen. Examples of antigen-binding fragments or antigen-binding moieties include, but are not limited to, Fab, Fab', F(ab')2, bispecific Fab' and Fv fragments, linear antibodies, single-chain antibodies, single-domain antibodies, and the like. Digestion of an intact antibody with papain produces two identical antigen-binding fragments, which are referred to as Fab fragments and each comprises a heavy chain variable region and a light chain variable region, as well as a light chain constant domain and a heavy chain constant domain CH1. Fab' fragments differ from Fab fragments by the addition of a few residues at the carboxy-terminus of the heavy chain constant domain CH1, including one or more cysteines from the antibody hinge region. Digestion of an intact antibody with pepsin produces a F(ab')2 fragment. The F(ab')2 fragment has two antigen-binding F(ab) moieties that are linked together by a disulfide bond. The F(ab')2 fragment is a bivalent antibody. A single-chain antibody is a fusion protein formed by linking an antibody heavy chain variable region and an antibody light chain variable region through a flexible short peptide consisting of about 10-25 amino acids. A single-domain antibody is an antibody fragment consisting of a variable region of a single monomer. Single-domain antibodies are often referred to as nanobodies as they are typically derived from the heavy chain variable regions of antibodies from animals of the family Camelidae or the family Carcharhinidae.

The term "multispecific antibody" (also referred to as a multi-antibody) refers to an antibody comprising antigen-binding moieties that specifically bind to epitopes of at least two different biomolecules or at least two different epitopes of the same biomolecule. For example, the "multispecific antibody" may be a bispecific antibody (also referred to as a bsAb), or a trispecific antibody. "Multi" means two or more. Also for convenience of description, quantifiers are separately added in front of the antigen-binding moieties for distinction. For example, "first antigen-binding moiety", "second antigen-binding moiety", and "third antigen-binding moiety", and the like may be used as needed. "First", "second", and "third" are not used to indicate an order of binding, nor do they signify importance, but are used for distinction only. "First antigen-binding moiety", "second antigen-binding moiety", and "third antigen-binding moiety" are also explained and illustrated below. The order in which the multispecific antibodies listed bind to the antigens is arbitrary unless otherwise indicated. Certainly, when specifically binding to a different biomolecule, a multispecific antibody may bind to more than one antigenic epitope of that particular biomolecule. According to a preferred embodiment of the present disclosure, the multispecific antibody mentioned comprises antigen-binding moieties binding to different epitopes of at least two different biomolecules.

The term "and/or", when used to connect two or more selectable items, should be understood to mean any one of the selectable items or any two or more of the selectable items.

The term "comprise" or "include" means including the stated elements or steps but not excluding other elements or steps. Certainly, "comprise" or "include" also encompasses instances consisting of the stated elements or steps, unless otherwise stated. For example, when referring to an antibody variable region comprising a specific sequence, it is also intended to encompass an antibody variable region that consists of that specific sequence.

The "affinity" or "binding affinity" mentioned herein is to be understood in the general sense of the art and is used to reflect the strength and/or stability between an antigen and a binding site on an antibody or antigen-binding fragment.

"Specifically binding" or "specifically binding to", "binding to", or "specifically targeting" a particular antigen or epitope, or being "specific for" or "capable of binding to" a particular antigen or epitope means being distinguished from non-specific interactions; such specific binding can be measured through some commonly used methods in the art. The ability of an antibody to bind to an antigen can be measured through the enzyme-linked immunosorbent assay (ELISA) or other techniques familiar to a person skilled in the art. For example, antigen-carrying cells can be detected by flow cytometry, and the competitive binding between the test antibody and the labeled antibody can be detected by measuring the positive rate of cells. Since the spatial structures of the antigens on the cell surface are more similar to the forms existing *in vivo,* the real situation can be better reflected through that method. According to a specific embodiment of the present disclosure, the bispecific antibody provided has an EC50 value of 0.5-200 nM, 1-200 nM, 5-200 nM, 10-200 nM, 15-200 nM, 0.5-150 nM, 1-150 nM, 5-150 nM, 10-150 nM, 15-150 nM, 0.5-120 nM, 1-120 nM, 5-120 nM, 10-120 nM, 15-120 nM, 10-100 nM, 15-100 nM, 10-80 nM, 15-80 nM, 10-60 nM, 15-60 nM, 10-50 nM, or 15-50 nM with an immune cell antigen (e.g., a T cell antigen (e.g., a CD3 antigen)), and an EC50 value of 0.01-20 nM, 0.05-20 nM, 0.01-16 nM, 0.05-16 nM, 0.01-15 nM, 0.05-15 nM, 0.01-10 nM, 0.05-10 nM, 0.1-10 nM, or 0.3-5 nM with a labeling polypeptide (e.g., a TT3 antigen). In addition, the binding activity of an antibody to an antigen can also be measured through surface plasmon resonance (SPR) technology or bio-layer interferometry (BLI) technology.

The multispecific antibodies or polynucleotides mentioned herein are generally isolatable or recombinant. "Isolatable" means being capable of being identified and isolated and/or collected from a cell or cell culture where a polypeptide or protein is expressed. In general, an isolated polypeptide is prepared through at least one purification step. "Isolated antibody" refers to an antibody that is substantially free of other antibodies with different antigen specificities or antigen-binding fragments thereof. "Recombinant" means that the antibody can be produced in an exogenous host cell using genetic recombination technology.

"Humanized" antibodies generally refer to antibodies consisting of antigen-binding moieties derived from non-human species and some structures and sequences based on human immunoglobulin molecules. For example, in a humanized antibody, the entire antibody, except for the CDRs, is encoded by a human-derived polynucleotide; it retains the binding activity to the antigen while having reduced immunogenicity.

The CDR sequences shown herein are obtained by analysis in conjunction with an existing definition scheme. For example, the CDR sequences of the antibodies shown, as well as the CDR sequences of the first antigen-binding moiety and the CDR sequences of the second antigen-binding moiety of the multispecific antibodies, are obtained in conjunction with the IMGT definition scheme (Ehrenmann F., Kaas Q. and Lefranc M.-P. Nucleic Acids Res., 38:D301-D307 (2010); Ehrenmann, F., Lefranc, M.-P. Cold Spring Harbor Protoc., 6:737-749 (2011)). These sequences can also be obtained through Kabat (e.g., see U.S. Dept. of Health and Human Servies, "Sequences of Proteins of Immunological Interest" (1983)), Chothia (e.g., see J. Mol. Biol. 196:901-917(1987)), or other definition schemes. It will be appreciated by a person skilled in the art that the differences in CDRs exhibited due to the definition method are also included within the protection scope of the present disclosure.

Bispecific antibodies (BsAbs) are antibodies that can specifically bind to two antigens or different epitopes of the same antigen simultaneously. BsAbs can target multiple antigens or antigenic epitopes and therefore have more advantages than monoclonal antibodies; additionally, they have also gradually shown higher therapeutic effects than monoclonal antibody combination therapy. For example, specific immune effector cells can be redirected to adjacent tumor cells to enhance tumor killing; moreover, the specificity of binding can be increased through the interaction between two different cell surface antigens; meanwhile, compared with combinations with single antibodies, BsAbs enable reductions in development costs and clinical trials. Bispecific antibodies have broad application prospects in fields such as tumor therapy and autoimmune diseases. In addition, with the gradual maturation of genetic engineering antibody technology, bispecific antibodies have developed rapidly.

The second composition comprises a second active ingredient comprising a multispecific antibody. The second composition may contain, in addition to the mentioned multispecific antibody, a pharmaceutically acceptable carrier. Some pharmaceutically acceptable salts are, such as acid addition salts or base addition salts (e.g., as described in Berge, S.M.etal (1977) J. Pharma. Sci. 66:1-19), if desired. The multispecific antibody may be a bispecific antibody comprising a first antigen-binding moiety and a second antigen-binding moiety. The first antigen-binding moiety is capable of specifically recognizing and binding to the extracellular antigen determining region of the labeling polypeptide, and the second antigen-binding moiety is capable of specifically recognizing and binding to an immune cell antigen or a first cytokine or a receptor thereof. By recruiting or bridging immune cells or cytokines or receptors thereof, an immunotherapeutic effect is exerted. The first antigen-binding moiety and the second antigen-binding moiety mentioned are fused, wherein the first antigen-binding moiety and the second antigen-binding moiety target different epitopes of different antigens, respectively. The first antigen-binding moiety and the second antigen-binding moiety mentioned are fused, wherein they may be directly linked (e.g., directly linked by the terminal amino acids of the two), may be indirectly linked by a linker, or may have additional functional region sequences, such as an Fc region, a CH1, and a CL sequence, linked between them. It should be noted that the terms "first", "second", and "third" as used herein, are used for descriptive purposes only and should not be construed as indicating or implying relative importance or implicitly indicating the number of indicated technical features, nor should they be used to indicate an order of appearance. The terms "first antigen-binding moiety", "second antigen-binding moiety", and "third antigen-binding moiety" mean moieties capable of binding to different epitopes of different target antigens or different epitopes of the same target antigen, which may be intact antibodies or part of an intact antibody, such as heavy chain variable regions, Fab, Fab', F(ab')₂, single-chain antibodies (ScFvs), or single-domain antibodies (sdAbs). According to a preferred embodiment of the present disclosure, the "first antigen-binding moiety", the "second antigen-binding moiety", and the "third antigen-binding moiety" specifically bind to different epitopes of different antigens, respectively.

Herein, "linker" and "linking fragment" have the same meaning. Linkers may be some oligopeptides or polypeptides, and may be any amino acid sequences capable of providing flexibility. Useful linkers are those commonly used in the art, including, but not limited to, the group consisting of: GS, SG, GGS, GSG, SGG, GGG, GGGS, SGGG, GGGGS, GGGGGSGS, GGGGSGS, GGGGSGGS, GGGGSGGGGSGGGGS, GGGGSGGGGS, GGGGSGGGGSGGGGSGGGGS, GGGGSGGGGSGGGGSGGGGSGGGGS, GGGGSGGGGSGGGGSGGGGSGGGSGGGS, etc.

The first antigen-binding moiety provided is capable of specifically recognizing and binding to the extracellular antigen determining region of the labeling polypeptide. According to a specific embodiment, the first antigen-binding moiety provided is capable of specifically recognizing and binding to the amino acid sequence set forth in SEQ ID NO: 1, 2, 3, 4, or 5 mentioned above. According to a specific embodiment, the first antigen-binding moiety provided comprises one or more domains capable of specifically recognizing and binding to the antigenic epitope polypeptide, wherein the amino acid sequence of the antigenic epitope polypeptide comprises the amino acid sequence of Strep tag II.

Preferably, the amino acid sequence of the light chain of the antigen-binding domain targeting TT1 is consistent with a sequence of amino acids at positions 1-111 of the light chain of an anti-Myc antibody (e.g., clone 9e10) (in some other embodiments of the present disclosure, the amino acid sequence of the light chain of the antigen-binding domain targeting TT1 may extend downstream of the above sequence by no more than 10 amino acids), and the amino acid sequence of the heavy chain is consistent with a sequence of amino acids at positions 23-143 of the heavy chain of an anti-Myc antibody (e.g., clone 9e10) (in some other embodiments of the present disclosure, the amino acid sequence of the heavy chain of the antigen-binding domain targeting TT1 may extend upstream and downstream of the above sequence by no more than 10 amino acids).

Preferably, the amino acid sequence of the light chain of the antigen-binding domain targeting TT2 is consistent with a sequence of amino acids at positions 2-121 of the light chain of an anti-HA antibody (e.g., clone 12ca5) (in some other embodiments of the present disclosure, the amino acid sequence of the light chain of the antigen-binding domain targeting TT2 may extend downstream of the above sequence by no more than 10 amino acids), and the amino acid sequence of the heavy chain is consistent with a sequence of amino acids at positions 2-114 of the heavy chain of an anti-HA antibody (e.g., clone 12ca5) (in some other embodiments of the present disclosure, the amino acid sequence of the heavy chain of the antigen-binding domain targeting TT2 may extend downstream of the above sequence by no more than 10 amino acids).

Preferably, the amino acid sequence of the light chain of the antigen-binding domain targeting TT3 is consistent with the light chain of an anti-Strep tag II antibody (see, e.g., patent document EP2871189A1), and the amino acid sequence of the heavy chain is consistent with the heavy chain of an anti-Strep tag II antibody (see, e.g., patent document EP2871189A1).

The amino acid sequence of the light chain of the anti-Myc antibody (clone 9e10) may be derived from the amino acid sequence numbered PDB: 2ORB_L (https://www.ncbi.nlm.nih.gov/protein/2ORB_L), and the amino acid sequence of the heavy chain of the anti-Myc antibody (clone 9e10) may be derived from the amino acid sequence numbered GenBank: CAA73271.1 (https://www.ncbi.nlm.nih.gov/protein/CAA73271.1?report=genbank&log$=protalig n&blast_rank=1&RID=P597FX1S014). The amino acid sequence of the linker linking the light chain and the heavy chain is set forth in SEQ ID NO: 16. According to a specific embodiment, the first antigen-binding moiety has the amino acid sequence set forth in SEQ ID NO: 17. The amino acid sequence of the light chain of the anti-HA antibody (clone 12ca5) may be derived from the amino acid sequence numbered PDB: 5XCS_B (https://www.ncbi.nlm.nih.gov/protein/1258501213), and the amino acid sequence of the heavy chain of the anti-HA antibody (clone 12ca5) may be derived from the amino acid sequence numbered PDB: 5XCS_A (https://www.ncbi.nlm.nih.gov/protein/5XCS A). The amino acid sequence of the linker linking the light chain and the heavy chain may be set forth in SEQ ID NO: 18. According to a specific embodiment, the first antigen-binding moiety has the amino acid sequence set forth in SEQ ID NO: 19.

The amino acid sequences of the light and heavy chains of the anti-Strep tag II antibody may be derived from the amino acid sequences disclosed in patent document EP2871189A1, and the amino acid sequence of the linker linking the light and heavy chains may be set forth in SEQ ID NO: 16. According to a specific embodiment, the first antigen-binding moiety has HCDR1, HCDR2, and HCDR3 sequences set forth in SEQ ID NOs: 20, 21, and 22, and LCDR1, LCDR2, and LCDR3 sequences set forth in SEQ ID NOs: 23, 24, and 25. The HCDR1, HCDR2, and HCDR3 sequences and the LCDR1, LCDR2, and LCDR3 sequences shown are obtained according to the IMGT definition scheme. According to a specific embodiment, the first antigen-binding moiety has a light chain variable region sequence set forth in SEQ ID NO: 26 and a heavy chain variable region sequence set forth in SEQ ID NO: 27. The light chain variable region sequence and the heavy chain variable region sequence are linked by a linker; according to a specific embodiment, the light chain variable region sequence and the heavy chain variable region sequence may be linked by a linker set forth in SEQ ID NO: 16. According to a specific embodiment, the first antigen-binding moiety has the sequence set forth in SEQ ID NO: 28. According to a specific embodiment, the first antigen-binding moiety has reduced immunogenicity by humanization of a light chain variable region set forth in SEQ ID NO: 26 and a heavy chain variable region set forth in SEQ ID NO: 27, while retaining the binding activity to TT3. According to a specific embodiment, the first antigen-binding moiety has a heavy chain variable region set forth in SEQ ID NO: 29 and a light chain variable region set forth in SEQ ID NO: 32. According to a specific embodiment, the first antigen-binding moiety has a heavy chain variable region set forth in SEQ ID NO: 29 and a light chain variable region set forth in SEQ ID NO: 33. According to a specific embodiment, the first antigen-binding moiety has a heavy chain variable region set forth in SEQ ID NO: 29 and a light chain variable region set forth in SEQ ID NO: 34. According to a specific embodiment, the first antigen-binding moiety has a heavy chain variable region set forth in SEQ ID NO: 30 and a light chain variable region set forth in SEQ ID NO: 32. According to a specific embodiment, the first antigen-binding moiety has a heavy chain variable region set forth in SEQ ID NO: 30 and a light chain variable region set forth in SEQ ID NO: 33. According to a specific embodiment, the first antigen-binding moiety has a heavy chain variable region set forth in SEQ ID NO: 30 and a light chain variable region set forth in SEQ ID NO: 34. According to a specific embodiment, the first antigen-binding moiety has a heavy chain variable region set forth in SEQ ID NO: 31 and a light chain variable region set forth in SEQ ID NO: 32. According to a specific embodiment, the first antigen-binding moiety has a heavy chain variable region set forth in SEQ ID NO: 31 and a light chain variable region set forth in SEQ ID NO: 33. According to a specific embodiment, the first antigen-binding moiety has a heavy chain variable region set forth in SEQ ID NO: 31 and a light chain variable region set forth in SEQ ID NO: 34.

| SEQ ID NO: | Sequence |
|---|---|
| 16 | GSTSGSGKPGSGEGSTKG |
| 17 | DIVLTQSPAFLAVSLGQRATISCRASESVDNYGFSFMNWFQQKPGQPPKL LIYAISNRGSGVPARFSGSGSGTDFSLNIHPVEEDDPAMYFCQQTKEVPW TFGGGTKLEIKGSTSGSGKPGSGEGSTKGQVQLQESGGDLVKPGGSLKLS CAASGFTFSHYGMSWVRQTPDKRLEWVATIGSRGTYTHYPDSVKGRFTI SRDNDKNALYLQMNSLKSEDTAMYYCARRSEFYYYGNTYYYSAMDY WGQGASVTVSS |
| 18 | GGGGSGGGGSGGGGS |
| 19 | EVKLVESGGDLVKPGGSLKLSCAASGFTFSSYGMSWVRQTPDKRLEWVA TISRGGSYTYYPDSVKGRFTISRDNAKNTLYLQMSSLKSEDTAMYYCAR RETYDEKGFAYWGQGTTVTVSSGGGGSGGGGSGGGGSDIELTQSPSSLT VTAGEKVTMSCKSSQSLLNSGNQKNYLTWYQQKPGQPPKLLIYWASTR ESGVPDRFTGSGSGRDFTLTISSVQAEDLAVYYCQNDNSHPLTFGAGTKL ELK |
| 20 (HCDR1) | GYTFTNYY |
| 21 (HCDR2) | LNPNNGDT |
| 22 (HCDR3) | ARTGRYEENAMDY |
| 23 (LCDR1) | ESVDSYGKSF |
| 24 (LCDR2) | RAS |
| 25 (LCDR3) | QQNNEDPWT |
| 26 (VL) | ELVMTQSPASLAVSLGQRATISCRASESVDSYGKSFMHWYQLKPGQPPK LLIYRASNLESGVPARFSGSGSRTDFTLTIDPVEADDAATYYCQQNNEDP WTFGGGTKLEIK |
| 27 (VH) | EVQLEQSGPELVKPGASVKMSCKASGYTFTNYYMKWVKQSHGKSLEWI |
| | GDLNPNNGDTFYNQKFKGKATLTVDKSSNTAYMQLNSLTSEDSAVYYCA RTGRYEENAMDYWGQGTSVTVSS |
| 28 | ELVMTQSPASLAVSLGQRATISCRASESVDSYGKSFMHWYQLKPGQPPK LLIYRASNLESGVPARFSGSGSRTDFTLTIDPVEADDAATYYCQQNNEDP WTFGGGTKLEIKGSTSGSGKPGSGEGSTKGEVQLEQSGPELVKPGASVK MSCKASGYTFTNYYMKWVKQSHGKSLEWIGDLNPNNGDTFYNQKFKG KATLTVDKSSNTAYMQLNSLTSEDSAVYYCARTGRYEENAMDYWGQGT SVTVSS |
| 29 (VH) | QVQLVQSGAEVKKPGASVKVSCKASGYTFTNYYMKWVKQAPGQRLE WMGDLNPNNGDTFYNQKFKGRVTITVDKSASTAYMELSSLRSEDTAVY YCARTGRYEENAMDYWGQGTLVTVSS |
| 30 (VH) | QVQLVQSGAEVKKPGASVKVSCKASGYTFTNYYMKWVKQAPGQSLEW IGDLNPNNGDTFYNQKFKGRVTITVDKSASTAYMELNSLRSEDTAVYYC ARTGRYEENAMDYWGQGTLVTVSS |
| 31 (VH) | QVQLVQSGAEVKKPGASVKVSCKASGYTFTNYYMKWVKQAPGQSLEW IGDLNPNNGDTFYNQKFKGKATLTVDKSASTAYMELNSLRSEDTAVYYC ARTGRYEENAMDYWGQGTLVTVSS |
| 32 (VL) | DIVLTQSPASLAVSPGQRATITCRASESVDSYGKSFMHWYQQKPGQPPKL LIYRASNLESGVPARFSGSGSRTDFTLTINPVEANDTANYYCQQNNEDPW TFGQGTKLEIK |
| 33 (VL) | DLVMTQSPASLAVSPGQRATITCRASESVDSYGKSFMHWYQQKPGQPPK LLIYRASNLESGVPARFSGSGSRTDFTLTINPVEANDTANYYCQQNNEDP WTFGQGTKLEIK |
| 34 (VL) | DLVMTQSPASLAVSPGQRATITCRASESVDSYGKSFMHWYQLKPGQPPK LLIYRASNLESGVPARFSGSGSRTDFTLTINPVEANDTANYYCQQNNEDP WTFGQGTKLEIK |

The first antigen-binding moiety may be linked to the second antigen-binding moiety by a linker to form a multispecific antibody. Flexible oligopeptides commonly used in the art for linking amino acid fragments may be used as linkers for the first antigen-binding moiety and the second antigen-binding moiety. According to a specific embodiment, the amino-terminal (also referred to as N-terminal) amino acid of the first antigen-binding moiety may be linked to the carboxy-terminal (also referred to as C-terminal) amino acid of the second antigen-binding moiety. According to a specific embodiment, the C-terminal amino acid of the first antigen-binding moiety may be linked to the N-terminal amino acid of the second antigen-binding moiety. When the first antigen-binding moiety and the second antigen-binding moiety are linked, they may be directly linked, may be linked by a linker, or may have an additional functional amino acid sequence linked between them. The additional functional amino acid sequence mentioned may be, for example, an Fc sequence, and thus may function to mediate ADCC (antibody-dependent cell-mediated cytotoxicity), ADCP (antibody-dependent cell-mediated phagocytosis), CDC (complement-mediated cytotoxicity), or the like. Sequences such as a CH₁, a CH₂, and a CL may be linked between the first antigen-binding moiety and the second antigen-binding moiety, if desired. According to a specific embodiment, the C-terminal amino acid of the first antigen-binding moiety is linked to an Fc sequence and, by a linker, to the second antigen-binding moiety. According to a specific embodiment, the C-terminal amino acid of the second antigen-binding moiety, a CH1, a CL sequence, and an Fc region form an IgG-like antibody structure, and the C-terminal amino acid of the Fc region is linked to the N-terminal amino acid of the first antigen-binding moiety by a linker. According to a specific embodiment, the light chain variable region and the heavy chain variable region sequences of the first antigen-binding moiety may be directly linked by a linker to form a single-chain antibody (scFv) format, or may be directly linked.

In some specific embodiments, the second antigen-binding moiety is capable of specifically recognizing and binding to a first cytokine or a receptor thereof. The first cytokine or the receptor thereof mentioned may be a full-length cytokine, a fragment of the cytokine, or a variant thereof. In some embodiments, the first cytokine mentioned includes, but is not limited to: at least one of interleukin-1 (IL-1α), interleukin-1β (IL-1β), interleukin-2 (IL-2), interleukin-4 (IL-4), IL-5, IL-6, IL-7, IL-9, IL-10, IL-11, IL-12, IL-13, IL-15, IL-16, IL-17, IL-17a, IL-17b, IL-17c, IL-17d, IL-17f, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-37, EPO, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL25, CCL26, CCL27, CCL28, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, XCL1, XCL2, CX3CL1, IFNα, IFNβ, IFNγ, TNFα, TGFβ, VEGF, GM-CSF, GCSF, or a related receptor thereof. By binding to a cytokine or a receptor thereof, an immunomodulatory effect may be exerted.

In some specific embodiments, the second antigen-binding moiety is capable of specifically recognizing and binding to an immune cell antigen, particularly an immune cell surface antigen, recruiting immune cells for tumor killing purposes. Herein, "immune cell" refers to any one of various cells that function in the immune system, e.g., protect against infection and foreign bodies. In a specific embodiment, the term may include leukocytes, such as neutrophils, eosinophils, basophils, lymphocytes, and monocytes. Immune cells also include immune effector cells. Immune effector cells refer to cells involved in an immune response, e.g., in the promotion of an immune effector response. Examples of immune effector cells include, but are not limited to, T cells, e.g., CD4+ T cells, CD8+ T cells, αT cells, βT cells, γT cells, and δT cells; B cells; natural killer (NK) cells; natural killer T (NKT) cells; dendritic cells; and mast cells and the like. In some embodiments, the immune cell is a T cell or an NK cell. In some embodiments, the immune cell is a cytotoxic T cell (e.g., a CD8+ T cell). In some embodiments, the immune cell is a helper T cell, e.g., a CD4+ T cell. The immune cell antigen mentioned includes, but is not limited to, CD1, CD1a, CD1b, CD1c, CD1d, CD1e, CD2, CD3, CD3d, CD3e, CD3g, CD4, CD5, CD6, CD7, CD8, CD8a, CD8b, CD9, CD10, CD11, CD11a, CD11b, CD11c, CD11d, CD12w, CD13, CD14, CD15, CD16, CD16a,CD16b, CD17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD42a, CD42b, CD42c, CD42d, CD43, CD44, CD45, CD45RA, CD45RB, CD45RC, CD45RO, CD46, CD47, CD48, CD49a, CD49b, CD49c, CD49d, CD49e, CD49f, CD50, CD52, CD53, CD54, CD55, CD56, CD57, CD58, CD59, CD60a, CD60b, CD60c, CD61, CD62E, CD62L, CD62P, CD63, CD64, CD65, CD66a, CD66b, CD66c, CD66d, CD66e, CD66f, CD67, CD68, CD69, CD70, CD71, CD72, CD73, CD74, CD75, CD75s, CD77, CD79, CD79a, CD79b, CD80, CD81, CD82, CD83, CD84, CD85, CD85a, CD85b, CD85c, CD85d, CD85e, CD85f, CD85g, CD85h, CD85i, CD85j, CD85k, CD86, CD87, CD88, CD89, CD90, CD91, CD92, CD93, CD94, CD95, CD96, CD97, CD98, CD99, CD100, CD101, CD102, CD103, CD104, CD105, CD106, CD107, CD107a, CD107b, CD108, CD109, CD110, CD11, CD112, CD115, CD116, CD117, CD119, CD120, CD121, CD122, CD123, CD124, CD125, CD126, CD127, CD129, CD130, CD133, CD131, CD132, CD134, CD135, CD137, CD138, CD139, CD141, CD142, CD143, CD144, CD147, CD146, CD148, CD150, CD151, CD152, CD153, CD154, CD155, CD156, CD157, CD158, CD158a, CD158b1, CD158b2, CD158c, CD158d, CD158e, CD158f1, CD158f2, CD158g, CD158h, CD158i, CD158j, CD158k, CD158z, CD159a, CD159c, CD160, CD161, CD162, CD163, CD166, CD165, CD166, CD168, CD169, CD170, CD171, CD172a, CD172b, CD172g, CD173, CD177, CD178, CD179, CD180, CD181, CD183, CD184, CD185, CD186, CD191, CD192, CD193, CD194, CD195, CD196, CD197,CD198, CD199, CD200, CD203, CD205, CD208, CD209, CD210, CD210b, CD212, CD231a1, CD213a2, CD217, CD218a, CD218b, CD222, CD224, CD225, CD226, CD227, CD229, CD232, CD243, CD244, CD245, CD247, CD252, CD253, CD256, CD257, CD258, CD261, CD262, CD262, CD263, CD264, CD265, CD268, CD269, CD271, CD272, CD273, CD274, CD275, CD276, CD277, CD278, CD279, CD280, CD281, CD282, CD283, CD284, CD286, CD288, CD289, CD290, CD292, CDw293, CD294, CD296, CD297, CD300a, CD300b, CD300c, CD300d, CD300e, CD303, CD304, CD305, CD306, CD307a, CD307b, CD307c, CD307d, CD309, CD312, CD314, CD315, CD316, CD317, CD318, CD319, CD320, CD328, CD329, CD335, CD336, CD337, CD352, CD353, CD357, CD361, CD362, and the like.

According to a specific embodiment, the second antigen-binding moiety is capable of specifically recognizing and binding to a CD3 antigen. CD3 (T cell surface glycoprotein CD3, a signal-transducing co-receptor of the T cell receptor) is a differentiated antigen expressed on the surface of all T lymphocytes, mainly mediates the transduction of T cell activation signals, and plays an important role in the body's immune responses against infections. The CD3 mentioned herein includes any natural CD3 of any vertebrate (including mammals, such as primates (e.g., humans) and rodents (e.g., mice and rats)), unless otherwise indicated. The term "CD3" encompasses "full-length", unprocessed CD3, as well as any form of CD3 resulting from intracellular processing or any fragment thereof. The term also includes variants of naturally occurring CD3, such as splice variants or allelic variants. In some specific embodiments, CD3 refers to full-length CD3 from humans and cynomolgus monkeys or a fragment thereof. In some specific embodiments, CD3 refers to full-length CD3 from mice or rats or a fragment thereof.

In some embodiments, the second antigen-binding moiety is capable of specifically recognizing and binding to a CD3 antigen. The anti-CD3 antibody may be from any known CD3-targeting antibody, or may be a CD3-targeting antibody that is under development or will be developed in the future, for example, antibody SP34 (see, e.g., Pessano, S. et al., EMBO J4 (1985) 337-344), antibody OKT3 (see, e.g., Kung, P. et al., Science 206 (1979) 347-349; Salmeron, A. et al., J Immunol 147 (1991) 3047-3052), or antibody UCHT1 (see, e.g., Callard, R.E. et al., Clin Exp Immunol 43 (1981) 497-505). Murine SP34 is sequence-optimized and humanized to obtain a second antigen-binding moiety specifically recognizing and targeting a CD3 antigen. Moreover, humanization enables high affinities for the human and cynomolgus monkey CD3 antigens.

According to a specific embodiment, the second antigen-binding moiety has HCDR1, HCDR2, and HCDR3 sequences set forth in SEQ ID NOs: 35, 36, and 37, and LCDR1, LCDR2, and LCDR3 sequences set forth in SEQ ID NOs: 38, 39, and 40. The HCDR1, HCDR2, and HCDR3 sequences and the LCDR1, LCDR2, and LCDR3 sequences shown are obtained according to the IMGT definition scheme.

| SEQ ID NO: | Sequence | SEQ ID NO: | Sequence |
|---|---|---|---|
| 35 | GFTFNTYA | 38 | TGAVTTSNY |
| 36 | IRSKYNNYAT | 39 | GTN |
| 37 | VRHGNFGNSYVSWFAY | 40 | ALWYSNLWV |

According to a specific embodiment, the second antigen-binding moiety has a heavy chain variable region sequence set forth in SEQ ID NO: 41 and a light chain variable region sequence set forth in SEQ ID NO: 42.

According to a specific embodiment, the sequence of the second antigen-binding moiety is humanized; the humanized second antigen-binding moiety exhibits a high affinity for the human or cynomolgus monkey CD3 antigen; when the second antigen-binding moiety and the first antigen-binding moiety form a bispecific antibody, the affinity for a CD3 antigen is somewhat reduced compared to a humanized CD3 monoclonal antibody. Even so, the multispecific antibody maintains a high affinity for labeling polypeptides through modification of the first antigen-binding moiety, and thus can effectively achieve tumor targeting and tumor killing effects. The design and screening of the present disclosure allow the multispecific antibody to exhibit particularly suitable affinity overall. According to a specific embodiment, the second antigen-binding moiety has a heavy chain variable region sequence set forth in SEQ ID NO: 43, 44, or 45. According to a specific embodiment, the second antigen-binding moiety has a light chain variable region sequence set forth in SEQ ID NO: 46, 47, 48, 49, 50, 51, 52, or 53. The humanized heavy chain variable region or light chain variable region and the light chain variable region or heavy chain variable region before humanization can form a sequence targeting a CD3 antigen. For example, the second antigen-binding moiety has a heavy chain variable region sequence set forth in SEQ ID NO: 41 and a light chain variable region sequence set forth in SEQ ID NO: 46. As another example, the second antigen-binding moiety has a heavy chain variable region sequence set forth in SEQ ID NO: 41 and a light chain variable region sequence set forth in SEQ ID NO: 47. As another example, the second antigen-binding moiety has a heavy chain variable region sequence set forth in SEQ ID NO: 41 and a light chain variable region sequence set forth in SEQ ID NO: 48. As another example, the second antigen-binding moiety has a heavy chain variable region sequence set forth in SEQ ID NO: 41 and a light chain variable region sequence set forth in SEQ ID NO: 49. As another example, the second antigen-binding moiety has a heavy chain variable region sequence set forth in SEQ ID NO: 41 and a light chain variable region sequence set forth in SEQ ID NO: 50. As another example, the second antigen-binding moiety has a heavy chain variable region sequence set forth in SEQ ID NO: 41 and a light chain variable region sequence set forth in SEQ ID NO: 51. As another example, the second antigen-binding moiety has a heavy chain variable region sequence set forth in SEQ ID NO: 41 and a light chain variable region sequence set forth in SEQ ID NO: 52. As another example, the second antigen-binding moiety has a heavy chain variable region sequence set forth in SEQ ID NO: 41 and a light chain variable region sequence set forth in SEQ ID NO: 53. As another example, the second antigen-binding moiety has a heavy chain variable region sequence set forth in SEQ ID NO: 43 and a light chain variable region sequence set forth in SEQ ID NO: 42. As another example, the second antigen-binding moiety has a heavy chain variable region sequence set forth in SEQ ID NO: 44 and a light chain variable region sequence set forth in SEQ ID NO: 42. As another example, the second antigen-binding moiety has a heavy chain variable region sequence set forth in SEQ ID NO: 45 and a light chain variable region sequence set forth in SEQ ID NO: 42.

According to a specific embodiment, the second antigen-binding moiety has a heavy chain variable region sequence set forth in SEQ ID NO: 43 and a light chain variable region sequence set forth in SEQ ID NO: 46.

According to a specific embodiment, the second antigen-binding moiety has a heavy chain variable region sequence set forth in SEQ ID NO: 43 and a light chain variable region sequence set forth in SEQ ID NO: 47.

According to a specific embodiment, the second antigen-binding moiety has a heavy chain variable region sequence set forth in SEQ ID NO: 43 and a light chain variable region sequence set forth in SEQ ID NO: 48.

According to a specific embodiment, the second antigen-binding moiety has a heavy chain variable region sequence set forth in SEQ ID NO: 43 and a light chain variable region sequence set forth in SEQ ID NO: 49.

According to a specific embodiment, the second antigen-binding moiety has a heavy chain variable region sequence set forth in SEQ ID NO: 43 and a light chain variable region sequence set forth in SEQ ID NO: 50.

According to a specific embodiment, the second antigen-binding moiety has a heavy chain variable region sequence set forth in SEQ ID NO: 43 and a light chain variable region sequence set forth in SEQ ID NO: 51.

According to a specific embodiment, the second antigen-binding moiety has a heavy chain variable region sequence set forth in SEQ ID NO: 43 and a light chain variable region sequence set forth in SEQ ID NO: 52.

According to a specific embodiment, the second antigen-binding moiety has a heavy chain variable region sequence set forth in SEQ ID NO: 43 and a light chain variable region sequence set forth in SEQ ID NO: 53.

According to a specific embodiment, the second antigen-binding moiety has a heavy chain variable region sequence set forth in SEQ ID NO: 44 and a light chain variable region sequence set forth in SEQ ID NO: 46.

According to a specific embodiment, the second antigen-binding moiety has a heavy chain variable region sequence set forth in SEQ ID NO: 44 and a light chain variable region sequence set forth in SEQ ID NO: 47.

According to a specific embodiment, the second antigen-binding moiety has a heavy chain variable region sequence set forth in SEQ ID NO: 44 and a light chain variable region sequence set forth in SEQ ID NO: 48.

According to a specific embodiment, the second antigen-binding moiety has a heavy chain variable region sequence set forth in SEQ ID NO: 44 and a light chain variable region sequence set forth in SEQ ID NO: 49.

According to a specific embodiment, the second antigen-binding moiety has a heavy chain variable region sequence set forth in SEQ ID NO: 44 and a light chain variable region sequence set forth in SEQ ID NO: 50.

According to a specific embodiment, the second antigen-binding moiety has a heavy chain variable region sequence set forth in SEQ ID NO: 44 and a light chain variable region sequence set forth in SEQ ID NO: 51.

According to a specific embodiment, the second antigen-binding moiety has a heavy chain variable region sequence set forth in SEQ ID NO: 44 and a light chain variable region sequence set forth in SEQ ID NO: 52.

According to a specific embodiment, the second antigen-binding moiety has a heavy chain variable region sequence set forth in SEQ ID NO: 44 and a light chain variable region sequence set forth in SEQ ID NO: 53.

According to a specific embodiment, the second antigen-binding moiety has a heavy chain variable region sequence set forth in SEQ ID NO: 45 and a light chain variable region sequence set forth in SEQ ID NO: 46.

According to a specific embodiment, the second antigen-binding moiety has a heavy chain variable region sequence set forth in SEQ ID NO: 45 and a light chain variable region sequence set forth in SEQ ID NO: 47.

According to a specific embodiment, the second antigen-binding moiety has a heavy chain variable region sequence set forth in SEQ ID NO: 45 and a light chain variable region sequence set forth in SEQ ID NO: 48.

According to a specific embodiment, the second antigen-binding moiety has a heavy chain variable region sequence set forth in SEQ ID NO: 45 and a light chain variable region sequence set forth in SEQ ID NO: 49.

According to a specific embodiment, the second antigen-binding moiety has a heavy chain variable region sequence set forth in SEQ ID NO: 45 and a light chain variable region sequence set forth in SEQ ID NO: 50.

According to a specific embodiment, the second antigen-binding moiety has a heavy chain variable region sequence set forth in SEQ ID NO: 45 and a light chain variable region sequence set forth in SEQ ID NO: 51.

According to a specific embodiment, the second antigen-binding moiety has a heavy chain variable region sequence set forth in SEQ ID NO: 45 and a light chain variable region sequence set forth in SEQ ID NO: 52.

According to a specific embodiment, the second antigen-binding moiety has a heavy chain variable region sequence set forth in SEQ ID NO: 45 and a light chain variable region sequence set forth in SEQ ID NO: 53.

| SEQ ID NO: | Sequence |
|---|---|
| 41(VH) | EVKLLESGGGLVQPKGSLKLSCAASGFTFNTYAMNWVRQAPGKGLEW VARIRSKYNNYATYYADSVKDRFTISRDDSQSILYLQMNNLKTEDTAMY YCVRHGNFGNSYVSWFAYWGQGTLVTVSA |
| 42(VL) | QAVVTQESALTTSPGETVTLTCRSSTGAVTTSNYANWVQEKPDHLFTGLI GGTNKRAPGVPARFSGSLIGDKAALTITGAQTEDEAIYFCALWYSNLWV FGGGTKLTVL |
| 43(VH) | EVQLVESGGGLVQPGGSLRLSCAASGFTFNTYAMNWVRQAPGKGLEW VARIRSKYNNYATYYADSVKDRFTISRDDSKNSLYLQMNSLKTEDTAVY YCVRHGNFGNSYVSWFAYWGQGTLVTVSA |
| 44(VH) | EVQLVESGGGLVQPGGSLRLSCAASGFTFNTYAMNWVRQAPGKGLEW VARIRSKYNNYATYYADSVKDRFTISRDDSKNILYLQMNSLKTEDTAVY YCVRHGNFGNSYVSWFAYWGQGTLVTVSA |
| 45(VH) | EVQLVESGGGLVQPGGSLRLSCAASGFTFNTYAMNWVRQAPGKGLEW VARIRSKYNNYATYYADSVKDRFTISRDDSKNILYLQMNSLKTEDTAMY YCVRHGNFGNSYVSWFAYWGQGTLVTVSA |
| 46(VL) | QAVVTQEPSLTVSPGGTVTLTCRSSTGAVTTSNYANWVQQKPGQAPRGL IGGTNKRAPWTPARFSGSLLGGKAALTLSGAQPEDEAEYYCALWYSNL WVFGGGTKLTVL |
| 47(VL) | QAVVTQEPSLTVSPGGTVTLTCRSSTGAVTTSNYANWVQQKPGQAPRGL IGGTNKRAPWTPARFSGSLLGGKAALTLSGAQTEDEAEYYCALWYSNL WVFGGGTKLTVL |
| 48(VL) | QAVVTQEPSLTVSPGGTVTLTCRSSTGAVTTSNYANWVQQKPGQAPRGL IGGTNKRAPWTPARFSGSLLGGKAALTITGAQTEDEAEYYCALWYSNL WVFGGGTKLTVL |
| 49(VL) | QAVVTQEPSLTVSPGGTVTLTCRSSTGAVTTSNYANWVQQKPGQAPRGL IGGTNKRAPWVPARFSGSLLGDKAALTLSGAQTEDEAEYYCALWYSNL WVFGGGTKLTVL |
| 50(VL) | QAVVTQEPSLTVSPGGTVTLTCRSSTGAVTTSNYANWVQEKPGQAPRGL IGGTNKRAPWVPARFSGSLLGDKAALTLSGAQTEDEAEYYCALWYSNL WVFGGGTKLTVL |
| 51(VL) | QAVVTQEPSLTVSPGGTVTLTCRSSTGAVTTSNYANWVQQKPGQAPRTL IGGTNKRAPWTPARFSGSLLGGKAALTLSGAQPEDEAEYYCALWYSNL WVFGGGTKLTVL |
| 52(VL) | QAVVTQEPSLTVSPGGTVTLTCRSSTGAVTTSNYANWVQQKPGQAPRTL IGGTNKRAPWVPARFSGSLLGDKAALTLSGAQPEDEAEYYCALWYSNL WVFGGGTKLTVL |
| 53(VL) | QAVVTQEPSLTVSPGGTVTLTCRSSTGAVTTSNYANWVQEKPGQAPRTLI GGTNKRAPWVPARFSGSLLGDKAALTLSGAQPEDEAEYYCALWYSNL WVFGGGTKLTVL |
| 54 (heavy chain constant domain) | ASTKGPSVFPLAPSSKSTSGGTAALGCLVKDYFPEPVTVSWNSGALTSG VHTFPAVLQSSGLYSLSSVVTVPSSSLGTQTYICNVNHKPSNTKVDKRVE PKSCDKTHTCPPCPAPEFEGGPSVFLFPPKPKDTLMISRTPEVTCVVVDV SHEDPEVKFNWYVDGVEVHNAKTKPREEQYNSTYRVVSVLTVLHQDW LNGKEYKCKVSNKALPASIEKTISKAKGQPREPQVYTLPPSREEMTKNQ VSLTCLVKGFYPSDIAVEWESNGQPENNYKTTPPVLDSDGSFFLYSKLTV DKSRWQQGNVFSCSVMHEALHNHYTQKSLSLSPGK |
| 55 (light chain constant domain) | |
| 56 (Fc region) | PCPAPEFEGGPSVFLFPPKPKDTLMISRTPEVTCVVVDVSHEDPEVKFNW YVDGVEVHNAKTKPREEQYNSTYRVVSVLTVLHQDWLNGKEYKCKVS NKALPASIEKTISKAKGQPREPQVYTLPPSREEMTKNQVSLTCLVKGFYP SDIAVEWESNGQPENNYKTTPPVLDSDGSFFLYSKLTVDKSRWQQGNVF SCSVMHEALHNHYTQKSLSLSPGK |
| 57 (heavy chain constant domain) | |

According to a specific embodiment, the second antigen-binding moiety and the first antigen-binding moiety are linked by a linker. According to a specific embodiment, the linker is (G₄S)₃. The heavy chain variable region of the second antigen-binding moiety is linked to a CH1, the CH1 is linked to an Fc region, and the light chain variable region is linked to a CL1. The N-terminal amino acid of the first antigen-binding moiety is linked to the C-terminal amino acid of the Fc region by a linker. The light chain variable region sequence and the heavy chain variable region sequence of the first antigen-binding moiety are linked directly or by a linker. The Fc region mentioned may be a human Fc region sequence. The Fc region sequence may be derived from, including, but not limited to, IgA, IgD, IgE, IgG (e.g., IgG1, IgG2, IgG3, and IgG4), IgM, and variants thereof. The variants mentioned result from mutations in the Fc region, including, but not limited to K214R, L234F, L235E, P331S, D356E, L358M, or any combination thereof, e.g., L234F/235E/P331S or K214R/L234F/L235E/P331S, and the like.

According to a specific embodiment, the bispecific antibody provided comprises a first antigen-binding moiety and a second antigen-binding moiety; the heavy chain variable region of the second antigen-binding moiety is directly linked to a heavy chain constant domain (a CH1 and an Fc region), and the light chain variable region of the second antigen-binding moiety is directly linked to a CL to form an IgG-like structure; the C-terminal amino acid of the Fc region is linked to the N-terminal amino acid sequence of the light chain variable region of the first antigen-binding moiety by a linker (the linker used is set forth in SEQ ID NO: 18), wherein the C-terminal amino acid of the light chain variable region of the first antigen-binding moiety is linked to the N-terminal amino acid of the heavy chain variable region of the first antigen-binding moiety by a linker (the linker used is set forth in SEQ ID NO: 16), as shown in a of FIG. 2. The bispecific antibody formed has a number starting with "BK-A". According to one specific embodiment, the bispecific antibody provided is BK-A-101, wherein the light chain variable region and the heavy chain variable region of the first antigen-binding moiety form a sequence set forth in SEQ ID NO: 28; the second antigen-binding moiety has a heavy chain variable region sequence set forth in SEQ ID NO: 41 and a light chain variable region sequence set forth in SEQ ID NO: 42; the heavy chain variable region sequence of the second antigen-binding moiety is linked to a heavy chain constant domain CH (the sequence set forth in SEQ ID NO: 57), and the light chain variable region sequence of the second antigen-binding moiety is linked to a light chain constant domain CL (the sequence set forth in SEQ ID NO: 55) to form an IgG-type structure; the N-terminal amino acid of the first antigen-binding moiety is linked to the C-terminal amino acid of an Fc region.

According to a specific embodiment, the bispecific antibody provided is BK-A-101-hu1, which is a bispecific antibody formed by humanizing the sequence of the second antigen-binding moiety of antibody BK-A-101; the second antigen-binding moiety of BK-A-101-hu1 has a heavy chain variable region set forth in SEQ ID NO: 44 and a light chain variable region sequence set forth in SEQ ID NO: 51. The other sequences were the same as those of BK-A-101.

According to a specific embodiment, the bispecific antibody provided is BK-A-101-hu2, which is a bispecific antibody formed by humanizing the sequence of the second antigen-binding moiety of antibody BK-A-101; the second antigen-binding moiety of BK-A-101-hu2 has a heavy chain variable region set forth in SEQ ID NO: 44 and a light chain variable region sequence set forth in SEQ ID NO: 52. The other sequences were the same as those of BK-A-101.

According to a specific embodiment, the bispecific antibody provided is BK-A-101-hu3, which is a bispecific antibody formed by humanizing the sequence of the second antigen-binding moiety of antibody BK-A-101; the second antigen-binding moiety of BK-A-101-hu3 has a heavy chain variable region set forth in SEQ ID NO: 44 and a light chain variable region sequence set forth in SEQ ID NO: 53. The other sequences were the same as those of BK-A-101.

According to a specific embodiment, the bispecific antibody provided is BK-A-101-hu4, which is a bispecific antibody formed by humanizing the sequence of the second antigen-binding moiety of antibody BK-A-101; the second antigen-binding moiety of BK-A-101-hu4 has a heavy chain variable region set forth in SEQ ID NO: 45 and a light chain variable region sequence set forth in SEQ ID NO: 51. The other sequences were the same as those of BK-A-101.

According to a specific embodiment, the bispecific antibody provided is BK-A-101-hu5, which is a bispecific antibody formed by humanizing the sequence of the second antigen-binding moiety of antibody BK-A-101; the second antigen-binding moiety of BK-A-101-hu5 has a heavy chain variable region set forth in SEQ ID NO: 45 and a light chain variable region sequence set forth in SEQ ID NO: 52. The other sequences were the same as those of BK-A-101.

According to a specific embodiment, the bispecific antibody provided is BK-A-101-hu6, which is a bispecific antibody formed by humanizing the sequence of the second antigen-binding moiety of antibody BK-A-101; the second antigen-binding moiety of BK-A-101-hu6 has a heavy chain variable region set forth in SEQ ID NO: 45 and a light chain variable region sequence set forth in SEQ ID NO: 53. The other sequences were the same as those of BK-A-101.

According to a specific embodiment, the bispecific antibody provided comprises a first antigen-binding moiety and a second antigen-binding moiety; the heavy chain variable region of the first antigen-binding moiety is directly linked to a heavy chain constant domain, and the light chain variable region of the first antigen-binding moiety is directly linked to a light chain constant domain to form an IgG-like structure; the carboxy-terminal amino acid of an Fc region of the IgG-like structure is linked to the N-terminal amino acid of the light chain variable region of the second antigen-binding moiety by a linker, and the N-terminal amino acid of the heavy chain variable region of the second antigen-binding moiety is linked to the C-terminal amino acid of the light chain variable region of the second antigen-binding moiety, as shown in b of FIG. 2; the bispecific antibody formed has a number starting with "BK-D", wherein the linkers used are all (G₄S)₃. According to a specific embodiment, the bispecific antibody provided is BK-D-01, which comprises a first antigen-binding moiety set forth in SEQ ID NO: 31 and SEQ ID NO: 32 and a second antigen-binding moiety set forth in SEQ ID NO: 43 and SEQ ID NO: 46. According to a specific embodiment, the bispecific antibody provided is BK-D-02, which comprises a first antigen-binding moiety set forth in SEQ ID NO: 31 and SEQ ID NO: 34 and a second antigen-binding moiety set forth in SEQ ID NO: 45 and SEQ ID NO: 46. According to a specific embodiment, the bispecific antibody provided is BK-D-03, which comprises a first antigen-binding moiety set forth in SEQ ID NO: 29 and SEQ ID NO: 34 and a second antigen-binding moiety set forth in SEQ ID NO: 43 and SEQ ID NO: 46. According to a specific embodiment, the bispecific antibody provided is BK-D-04, which comprises a first antigen-binding moiety set forth in SEQ ID NO: 29 and SEQ ID NO: 32 and a second antigen-binding moiety set forth in SEQ ID NO: 43 and SEQ ID NO: 49. According to a specific embodiment, the bispecific antibody provided is BK-D-05, which comprises a first antigen-binding moiety set forth in SEQ ID NO: 29 and SEQ ID NO: 32 and a second antigen-binding moiety set forth in SEQ ID NO: 44 and SEQ ID NO: 48. According to a specific embodiment, the bispecific antibody provided is BK-D-06, which comprises a first antigen-binding moiety set forth in SEQ ID NO: 29 and SEQ ID NO: 32 and a second antigen-binding moiety set forth in SEQ ID NO: 44 and SEQ ID NO: 50. According to a specific embodiment, the bispecific antibody provided is BK-D-07, which comprises a first antigen-binding moiety set forth in SEQ ID NO: 31 and SEQ ID NO: 32 and a second antigen-binding moiety set forth in SEQ ID NO: 45 and SEQ ID NO: 46. According to a specific embodiment, the bispecific antibody provided is BK-D-08, which comprises a first antigen-binding moiety set forth in SEQ ID NO: 29 and SEQ ID NO: 32 and a second antigen-binding moiety set forth in SEQ ID NO: 45 and SEQ ID NO: 46. According to a specific embodiment, the bispecific antibody provided is BK-D-09, which comprises a first antigen-binding moiety set forth in SEQ ID NO: 29 and SEQ ID NO: 32 and a second antigen-binding moiety set forth in SEQ ID NO: 44 and SEQ ID NO: 47. According to a specific embodiment, the bispecific antibody provided is BK-D-10, which comprises a first antigen-binding moiety set forth in SEQ ID NO: 31 and SEQ ID NO: 34 and a second antigen-binding moiety set forth in SEQ ID NO: 43 and SEQ ID NO: 46. According to a specific embodiment, the bispecific antibody provided is BK-D-11, which comprises a first antigen-binding moiety set forth in SEQ ID NO: 29 and SEQ ID NO: 32 and a second antigen-binding moiety set forth in SEQ ID NO: 43 and SEQ ID NO: 46. According to a specific embodiment, the bispecific antibody provided is BK-D-12, which comprises a first antigen-binding moiety set forth in SEQ ID NO: 29 and SEQ ID NO: 34 and a second antigen-binding moiety set forth in SEQ ID NO: 45 and SEQ ID NO: 46. According to a specific embodiment, the bispecific antibody provided is BK-D-13, which comprises a first antigen-binding moiety set forth in SEQ ID NO: 29 and SEQ ID NO: 32 and a second antigen-binding moiety set forth in SEQ ID NO: 45 and SEQ ID NO: 50. According to a specific embodiment, the heavy chain constant domain sequence of the bispecific antibody of the BK-D structure is set forth in SEQ ID NO: 54, and the light chain constant domain sequence is set forth in SEQ ID NO: 55. The Fc region sequence is set forth in SEQ ID NO: 56. The sequence of the heavy chain constant domain in the bispecific antibody of the BK-D structure (SEQ ID NO: 54) contains mutations in the Fc region compared to the sequence set forth in SEQ ID NO: 57. According to a specific embodiment, the affinity activity of the bispecific antibody provided for a CD3 antigen is somewhat reduced compared to that of a humanized monoclonal antibody the Fc of which is not mutated; the bispecific antibody has an EC50 value of 0.5-200 nM, 1-200 nM, 5-200 nM, 10-200 nM, 15-200 nM, 0.5-150 nM, 1-150 nM, 5-150 nM, 10-150 nM, 15-150 nM, 0.5-120 nM, 1-120 nM, 5-120 nM, 10-120 nM, 15-120 nM, 10-100 nM, 15-100 nM, 10-80 nM, 15-80 nM, 10-60 nM, 15-60 nM, 10-50 nM, or 15-50 nM for a CD3 antigen.

According to a specific embodiment, the bispecific antibody provided comprises a first antigen-binding moiety and a second antigen-binding moiety; the C-terminal amino acid of the first antigen-binding moiety is directly linked to an Fc region sequence (CH2 and CH3); the C-terminal amino acid of the Fc region is linked to the N-terminal amino acid of the light chain variable region of the second antigen-binding moiety by a linker; the C-terminal amino acid of the light chain variable region of the second antigen-binding moiety is linked to the N-terminal amino acid of the heavy chain variable region of the second antigen-binding moiety by a linker (according to a specific embodiment, the linker is set forth in SEQ ID NO: 18), as shown in FIG. 3. According to one specific embodiment, the bispecific antibody provided is BK-B-110, wherein the light chain variable region and the heavy chain variable region of the first antigen-binding moiety form a sequence set forth in SEQ ID NO: 28; the second antigen-binding moiety has a heavy chain variable region sequence set forth in SEQ ID NO: 41 and a light chain variable region sequence set forth in SEQ ID NO: 42; the Fc region sequence is set forth in SEQ ID NO: 56. According to a specific embodiment, the bispecific antibody provided is BK-B-110-hu1, which is a bispecific antibody formed by humanizing the second antigen-binding moiety of antibody BK-B-110; the second antigen-binding moiety of BK-B-110-hu1 has a heavy chain variable region set forth in SEQ ID NO: 44 and a light chain variable region sequence set forth in SEQ ID NO: 51. According to a specific embodiment, the bispecific antibody provided is BK-B-110-hu2, which is a bispecific antibody formed by humanizing the second antigen-binding moiety of antibody BK-B-110; the second antigen-binding moiety of BK-B-110-hu2 has a heavy chain variable region set forth in SEQ ID NO: 44 and a light chain variable region sequence set forth in SEQ ID NO: 52. According to a specific embodiment, the bispecific antibody provided is BK-B-110-hu3, which is a bispecific antibody formed by humanizing the second antigen-binding moiety of antibody BK-B-110; the second antigen-binding moiety of BK-B-110-hu2 has a heavy chain variable region set forth in SEQ ID NO: 44 and a light chain variable region sequence set forth in SEQ ID NO: 53. According to a specific embodiment, the bispecific antibody provided is BK-B-110-hu4, which is a bispecific antibody formed by humanizing the second antigen-binding moiety of antibody BK-B-110; the second antigen-binding moiety of BK-B-110-hu4 has a heavy chain variable region set forth in SEQ ID NO: 45 and a light chain variable region sequence set forth in SEQ ID NO: 51. According to a specific embodiment, the bispecific antibody provided is BK-B-110-hu5, which is a bispecific antibody formed by humanizing the second antigen-binding moiety of antibody BK-B-110; the second antigen-binding moiety of BK-B-110-hu4 has a heavy chain variable region set forth in SEQ ID NO: 45 and a light chain variable region sequence set forth in SEQ ID NO: 52. According to a specific embodiment, the bispecific antibody provided is BK-B-110-hu6, which is a bispecific antibody formed by humanizing the second antigen-binding moiety of antibody BK-B-110; the second antigen-binding moiety has a heavy chain variable region set forth in SEQ ID NO: 45 and a light chain variable region sequence set forth in SEQ ID NO: 53.

According to a specific embodiment, the bispecific antibody provided comprises a first antigen-binding moiety and a second antigen-binding moiety; the C-terminal amino acid of the first antigen-binding moiety is directly linked to an Fc region sequence; the C-terminal amino acid of the Fc region is linked to the N-terminal amino acid of the heavy chain variable region of the second antigen-binding moiety by a linker (according to a specific embodiment, the linker used is set forth in SEQ ID NO: 18); the C-terminal amino acid of the heavy chain variable region of the second antigen-binding moiety is linked to the N-terminal amino acid of the light chain variable region of the second antigen-binding moiety by a linker (according to a specific embodiment, the linker used is set forth in SEQ ID NO: 18), as shown in FIG. 4. According to one specific embodiment, the bispecific antibody provided is BK-C-120, wherein the first antigen-binding moiety has the sequence set forth in SEQ ID NO: 28; the second antigen-binding moiety has a heavy chain variable region sequence set forth in SEQ ID NO: 41 and a light chain variable region sequence set forth in SEQ ID NO: 42; the Fc region sequence is set forth in SEQ ID NO: 56. According to a specific embodiment, the bispecific antibody provided is BK-C-120-hu1, which is a bispecific antibody formed by humanizing the second antigen-binding moiety of antibody BK-C-120; the second antigen-binding moiety of BK-C-120-hu1 has a heavy chain variable region set forth in SEQ ID NO: 44 and a light chain variable region sequence set forth in SEQ ID NO: 51. According to a specific embodiment, the bispecific antibody provided is BK-C-120-hu2, which is a bispecific antibody formed by humanizing the second antigen-binding moiety of antibody BK-C-120; the second antigen-binding moiety has a heavy chain variable region sequence set forth in SEQ ID NO: 44 and a light chain variable region sequence set forth in SEQ ID NO: 52. According to a specific embodiment, the bispecific antibody provided is BK-C-120-hu3, which is a bispecific antibody formed by humanizing the second antigen-binding moiety of antibody BK-C-120; the second antigen-binding moiety has a heavy chain variable region set forth in SEQ ID NO: 44 and a light chain variable region sequence set forth in SEQ ID NO: 53. According to a specific embodiment, the bispecific antibody provided is BK-C-120-hu4, which is a bispecific antibody formed by humanizing the second antigen-binding moiety of antibody BK-C-120; the second antigen-binding moiety has a heavy chain variable region set forth in SEQ ID NO: 45 and a light chain variable region sequence set forth in SEQ ID NO: 51. According to a specific embodiment, the bispecific antibody provided is BK-C-120-hu5, which is a bispecific antibody formed by humanizing the second antigen-binding moiety of antibody BK-C-120; the second antigen-binding moiety has a heavy chain variable region set forth in SEQ ID NO: 45 and a light chain variable region sequence set forth in SEQ ID NO: 52. According to a specific embodiment, the bispecific antibody provided is BK-C-120-hu6, which is a bispecific antibody formed by humanizing the second antigen-binding moiety of antibody BK-C-120; the second antigen-binding moiety has a heavy chain variable region set forth in SEQ ID NO: 45 and a light chain variable region sequence set forth in SEQ ID NO: 53.

The multispecific antibody may also optionally comprise a third antigen-binding moiety specifically recognizing and binding to a tumor antigen or an immune checkpoint or a second cytokine or a receptor thereof, if desired. The tumor antigen mentioned includes tumor-associated antigens (TAAs). Tumor-associated antigens are antigenic substances produced by tumor cells and are capable of eliciting an immune response in a host. Tumor-associated antigens may be from a cancer, including, but not limited to, primary or metastatic melanoma, thymoma, lymphoma, sarcoma, lung cancer, liver cancer, non-Hodgkin lymphoma, leukemia, uterine cancer, cervical cancer, bladder cancer, ovarian cancer, breast cancer, and the like. TAAs may be patient-specific. Examples include, but are not limited to, p53, Ras, β-catenin, CDK4, α-actinin-4, CD20, PSMA, Her2, EGFR, and the like. According to a specific embodiment, the tumor antigen includes, but is not limited to, at least one of AFP, BCMA, β-catetin, CD5, CD19, CD20, CD22, CD30, CD38, CD70, CD123, CD133, CD171, CD138, CDK-4, CEA, CA-125, Caspase 8, c-Met, EGFR, Ep-CAM, EphA2, ETA, FAP, GPC3, GP100, GD2, Her2, HPV16, an Igk chain, Ley, Mesothlin, MUC-1, MUC16, MAGE, MART1, an NKG2D ligand, CLL1, NY-ESO-1, P53, RAS, OSA, PAP, PSCA, ROR1, ROR2, PSMA, SSTR2, Trop-2, TYR, VEGFR, and WT1. The immune checkpoint mentioned includes, but is not limited to, PD-1, PD-L1, CTLA-4, LAG-3, BTLA, TIM-3, TIGIT, VISTA, TIPE2, Galectin-9, NKG2A, KIRs, B7-H3, B7-H7, 2B4, DNAM-1, PTA1, TLISA1, Vstm3, VSIG9, the Siglecs family, GITR, OX40, 4-1BB, LAYN, LIRB1-5, or a related receptor thereof. The third antigen-binding moiety may also specifically recognize and bind to a second cytokine or a receptor thereof, including, but not limited to, IL-1α, IL-1β, IL-2, IL-4, IL-5, IL-6, IL-7, IL-9, IL-10, IL-11, IL-12, IL-13, IL-15, IL-16, IL-17, IL-17a, IL-17b, IL-17c, IL-17d, IL-17f, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-37, EPO, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL25, CCL26, CCL27, CCL28, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, XCL1, XCL2, CX3CL1, IFNα, IFNβ, IFNγ, TNFα, TGFβ, VEGF, GM-CSF, GCSF, or a related receptor thereof. It should be noted that the first cytokine or the receptor thereof and the second cytokine or the receptor thereof may be identical or different.

The multispecific antibody provided may be encoded by a polynucleotide. The term "nucleic acid" or "polynucleotide" refers to DNA or RNA and polymers thereof in single- or double-stranded form. According to a specific embodiment, the polynucleotide encodes the multispecific antibody and can be used to express the multispecific antibody *in vivo* or *in vitro.* The polynucleotide mentioned is isolatable and includes, but is not limited to, DNA, RNA, cDNA, or the like. Isolated polynucleotide sequences can be obtained using conventional methods in the art and be used to encode multispecific antibodies.

To prepare the antibody mentioned, a polynucleotide sequence encoding the antibody may be inserted into a replicable expression vector and expressed in a host cell or cell-free expression system. To this end, the present disclosure further provides a construct comprising the polynucleotide described above. "Construct" refers to any recombinant polynucleotide molecule (such as a plasmid, virus, autonomously replicating polynucleotide molecule, phage, or linear or circular single-stranded or double-stranded DNA or RNA polynucleotide molecule), derived from any source and capable of integrating into the genome of the host or autonomously replicating and effecting expression of an antibody in a host cell. The construct can be obtained by a variety of methods commonly used in the art, including *in vitro* recombinant DNA technology, DNA synthesis technology, *in vivo* recombinant technology, etc.; for example, the construct can be formed by inserting the polynucleotide into the multiple cloning site of an expression vector. The construct may comprise, if desired, various operators such as a promoter, a terminator, a marker gene, etc., and these operators are operably linked to the polynucleotide. Promoters are generally used to provide a signal for starting transcription. The lactose promoter (Lac), the Trp promoter, the Tac promoter, or the PL and PR promoters of phages may be selected, if desired. Terminators provide a signal for terminating transcription during transcription. Marker genes on constructs are often used for screening. Certainly, an enhancer may be included, if desired, to enhance protein expression. The expression vector is not particularly limited and may be some of the commercially available expression vectors, or may be an expression vector that is artificially modified if desired, e.g., a plasmid, a phage, a virus, or the like. The virus may be a plant cell virus, a mammalian cell virus, or the like. The construct can express the antibody or protein *in vitro,* and can also be transferred into a cell to express the antibody or protein.

To produce or express the multispecific antibodyto be used as a component in a therapeutic agent, the present disclosure further provides a host cell comprising the polynucleotide described above or the construct described above. Any cell suitable for antibody or protein expression of the polynucleotide or the construct may be used as a host cell. The host cell may be a prokaryotic cell, such as a bacterial cell; or a eukaryotic cell, such as a yeast cell, a mammalian cell, or the like. Commonly used host cells may be yeast cells, Chinese hamster ovary (CHO) cells (e.g., CHO-K1, CHO DG44, CHO-S, CHO-DXB11, and CHO-M), HEK-293 cells, COS cells, insect cells of Drosophila S2 or Sf9, NS0 cells, Hela cells, 3T3 cells, BHK cells, etc. The host cell comprising the polynucleotide or the construct may be obtained by methods commonly used in the art, such as microinjection, electroporation, chemical transfection, virus-mediated transformation, etc.

The present disclosure further provides a kit of a combinational drug with a synergistic effect for treating a tumor and/or a cancer, the kit comprising:
a first container containing the first composition in the therapeutic agent according to the present disclosure;
a second container containing the second composition in the therapeutic agent according to the present disclosure or containing the multispecific antibody mentioned in the present disclosure, wherein the first container and the second container are independent; and
instructions for the timing and mode of administration.

The first composition, the second composition, and the multispecific antibody mentioned herein may be formulated in different dosage forms, if desired, to meet the requirements of administration, e.g., injections, powders, or liquid preparations. In preparing different dosage forms, the first active ingredient or the second active ingredient may be compounded with different pharmaceutically acceptable carriers.

The present disclosure further provides use of the isolated nucleic acid having the labeling polypeptide encoding sequence according to the present disclosure in the preparation of a medicament for treating or preventing a tumor and/or a cancer.

The present disclosure further provides use of the kit in the preparation of a medicament for treating or preventing a tumor and/or a cancer.

The tumor and/or the cancer includes breast cancer, head and neck tumor, synovial cancer, kidney cancer, connective tissue cancer, melanoma, lung cancer, esophageal cancer, colon cancer, rectal cancer, brain cancer, liver cancer, bone cancer, choriocarcinoma, gastrinoma, pheochromocytoma, prolactinoma, von Hippel-Lindau disease, Zollinger-Ellison syndrome, anal cancer, bile duct cancer, bladder cancer, ureteral cancer, glioma, neuroblastoma, meningioma, spinal cord tumor, osteochondroma, chondrosarcoma, Ewing's sarcoma, cancer of unknown primary site, carcinoid, fibrosarcoma, Paget's disease, cervical cancer, gallbladder cancer, eye cancer, Kaposi's sarcoma, prostate cancer, testicular cancer, cutaneous squamous cell carcinoma, mesothelioma, multifocal myeloma, ovarian cancer, pancreatic endocrine tumor, glucagonoma, pancreatic cancer, penile cancer, pituitary cancer, soft tissue sarcoma, retinoblastoma, small intestine cancer, gastric cancer, thymic cancer, trophoblastic cancer, hydatidiform mole, endometrial cancer, vaginal cancer, vulvar cancer, mycosis fungoides, insulinoma, heart cancer, meningeal cancer, peritoneal cancer, pleural cancer, and hematological cancer.

The present disclosure further provides a method for treating a tumor and/or a cancer, comprising: administering to a subject the first composition in the therapeutic agent according to the present disclosure; and administering to the subject the second composition in the therapeutic agent according to the present disclosure or the multispecific antibody mentioned in the present disclosure. "Subject" includes any human or non-human animal. The term "non-human animal" includes all vertebrates, e.g., mammals and non-mammals, such as non-human primates, sheep, dogs, cats, horses, cattle, chickens, amphibians, and reptiles. According to a specific embodiment, the subject is a patient with a tumor and/or a cancer. The "treating" mentioned means being able to cause a reduction in the severity of the symptoms of the disease, an increase in the frequency and duration of the asymptomatic phase of the disease, or reductions in the suffering caused by the disease. After being treated, the subject achieves an inhibition rate, against tumor cells or cancer cells in the subject, of 10% or more, 15% or more, 20% or more, 25% or more, 30% or more, 40% or more, 45% or more, 50% or more, 55% or more, 60% or more, 65% or more, 70% or more, 75% or more, 80% or more, 85% or more, even 90% or more, or 95% or more, compared to an untreated subject.

The first composition and the second composition in the therapeutic agent or the multispecific antibody may be administered simultaneously (e.g., as a mixture), separately but simultaneously (e.g., by intratumoral administration and intravenous injection, respectively), or sequentially (e.g., the first composition is administered first, and the second composition or the multispecific antibody is then administered).

Preferably, the method comprises the following steps performed in sequence:
1) first, administering to the subject the first composition; and
2) administering to the subject the second composition in the therapeutic agent or the multispecific antibody after administration of the first composition.

Preferably, on day 1-60 after the first administration of the first composition, the second composition in the therapeutic agent or the multispecific antibody is administered to the subject.

Taking administration of the first composition and the second composition as an example, "on day 1-60 after the first administration of the first composition, the second composition in the therapeutic agent is administered to the subject or patient" means that the time interval between the first administration of the second composition and the first administration of the first composition is 1-60 days (e.g., 1, 2, 3, 4, 5, 6, 7, 8, 9, 10, 11, 12, 13,... 60 days), or the time interval between the first administration of the second composition and the last administration of the first composition prior to the first administration of the second composition is 1-60 days (e.g., 1, 2, 3, 4, 5, 6, 7, 8, 9, 10, 11, 12, 13,... 60 days). Preferably, the time interval between the first administration of the second composition and the last administration of the first composition prior to the first administration of the second composition is 1-30 days (e.g., 1, 2, 3, 4, 5, 6, 7, 8, 9, 10, 11, 12, 13,... 30 days). More preferably, the time interval between the first administration of the second composition and the last administration of the first composition prior to the first administration of the second composition is 5-20 days (e.g., 5, 6, 7, 8, 9, 10,... 20 days).

It should be noted that after administration of the first composition to the subject, the second composition may be administered to the subject more than once, e.g., multiple times such as twice, three times, or four times. The number of administrations of the second composition may be adjusted depending on the condition of the subject. Likewise, before administration of the second composition to the subject, the first composition may be administered more than once, e.g., multiple times such as twice, three times, or four times. The number of administrations of the first composition may be adjusted depending on the condition of the subject. Similarly, the subject may be administered multiple times the first composition and multiple times the second composition. Both the number of administrations of the first composition and the number of administrations of the second composition may be adjusted depending on the condition of the subject.

In one preferred embodiment of the present disclosure, the nucleic acid is administered at a dose of 0.01-10 mg/day, 1-3 times a day, for 1-7 consecutive days. The nucleic acid is capable of encoding a labeling polypeptide in a subject.

In one preferred embodiment of the present disclosure, the recombinant oncolytic poxvirus is administered at such a dose that the total dose per treatment course ranges from about 5 × 10⁷ to 5 × 10¹² viral particles (which may be administered in a single administration or multiple administrations) or is any value between the above ranges, or the recombinant oncolytic poxvirus is administered at such a dose that the total dose per treatment course ranges from about 5 × 10⁷ to 5 × 10¹² PFU (which may be administered in a single administration or multiple administrations) or is any value between the above ranges. The precise dosage will also depend on the practitioner's judgment and the individual's particular condition. In the case of multiple administrations, administration was performed 1-3 times a day for 1-7 consecutive days.

In one preferred embodiment of the present disclosure, the multispecific antibody is administered at a dose of 0.1-50 mg/kg body weight, e.g., 0.5-50 mg/kg body weight, 1-50 mg/kg body weight, 2-49 mg/kg body weight, 3-48 mg/kg body weight, 4-47 mg/kg body weight, 5-46 mg/kg body weight, 6-45 mg/kg body weight, 7-44 mg/kg body weight, 8-43 mg/kg body weight, 9-42 mg/kg body weight, 10-41 mg/kg body weight, 11-40 mg/kg body weight, 12-39 mg/kg body weight, 13-38 mg/kg body weight, 14-37 mg/kg body weight, 15-36 mg/kg body weight, 16-35 mg/kg body weight, 17-34 mg/kg body weight, 18-33 mg/kg body weight, 19-32 mg/kg body weight, 20-31 mg/kg, 21-30 mg/kg body weight, 22-29 mg/kg body weight, 23-28 mg/kg body weight, 24-27 mg/kg body weight, 25-26 mg/kg body weight, 10-20 mg/kg body weight, or 15-25 mg/kg body weight.

In certain embodiments, the method for treating a tumor and/or a cancer further comprises administering to the patient an additional medicament for treating the tumor and/or the cancer, and/or a medicament for modulating the patient's immune system, to enhance the effectiveness of the tumor treatment. The additional medicament for treating the tumor and/or the cancer includes, but is not limited to, chemotherapeutic medicaments, such as cyclophosphamide and fludarabine; radiotherapeutic medicaments; immunosuppressants, such as cyclosporin, azathioprine, methotrexate, mycophenolate, and FK50; antibodies, such as antibodies against CD3, IL-2, IL-6, IL-17, and TNFa.

The DNA may be formulated for intratumoral injection or intravenous administration; the mRNA may be formulated for intratumoral injection or intravenous administration. For example, it is administered by direct intratumoral injection in the form of a plasmid, by intratumoral injection after being packaged with a liposome, by intratumoral injection after being connected to a nanoparticle (e.g., a polymer such as poly-L-lysine, a polyamino acid, polyethyleneimine, or chitosan), or by intratumoral injection followed by an electroporation process for enhancing the transfection rate.

The recombinant virus may be formulated for different administration modes, including, but not limited to, intratumoral injection or intravenous administration or intracranial injection or intraperitoneal administration, and the like. Any administration mode through which the recombinant virus can be injected into the subject to achieve a therapeutic effect is acceptable. The administration mode may be adjusted as required by the subject, or multiple administration modes may be used in combination. The multispecific antibody may be administered using a variety of modes, such as intratumoral injection, intravenous administration, and intraperitoneal injection. The administration mode of the multispecific antibody may be adjusted as required by the subject, or multiple administration modes may be used in combination.

The present disclosure further provides a novel immunotherapy comprising: administering to a subject a therapeutically effective amount of an oncolytic virus, wherein the genome of the oncolytic virus comprises a labeling polypeptide encoding sequence, and the labeling polypeptide has an extracellular antigen determining region, a spacer portion, and a transmembrane portion that are operably linked; the amino acid sequence of the extracellular antigen determining region comprises the amino acid sequences of one or more antigenic epitope polypeptides; and in a natural state, the amino acid sequence of a cell membrane protein or secreted protein of the subject does not comprise the amino acid sequences of the antigenic epitope polypeptides; and administering to the subject a therapeutically effective amount of a multispecific antibody, wherein the multispecific antibody comprises at least a first antigen-binding moiety and a second antigen-binding moiety; the first antigen-binding moiety is capable of specifically recognizing and binding to the extracellular antigen determining region of the labeling polypeptide, and the second antigen-binding moiety is capable of specifically recognizing and binding to an immune cell antigen or a first cytokine or a receptor thereof; the multispecific antibody may also optionally comprise a third antigen-binding moiety specifically recognizing and binding to a tumor antigen or an immune checkpoint or a second cytokine or a receptor thereof. According to a specific embodiment, the second antigen-binding moiety is capable of specifically recognizing and binding to an immune cell surface antigen.

According to a specific embodiment, the novel immunotherapy comprises the following steps performed in sequence:
1) administering to the subject a therapeutically effective amount of the oncolytic virus; and
2) administering to the subject a therapeutically effective amount of the multispecific antibody after administration of the oncolytic virus.

The present disclosure further provides an anti-CD3 antibody capable of specifically targeting a CD3 antigen and exhibiting higher affinities for human and cynomolgus monkey CD3 antigens by sequence optimization and humanization of murine SP34. According to a specific embodiment, the anti-CD3 antibody has a heavy chain variable region sequence set forth in SEQ ID NO: 43, 44, or 45. According to a specific embodiment, the anti-CD3 antibody has a light chain variable region sequence set forth in SEQ ID NO: 46, 47, 48, 49, 50, 51, 52, or 53. According to a specific embodiment, the anti-CD3 antibody has a heavy chain variable region sequence set forth in SEQ ID NO: 44 and a light chain variable region sequence set forth in SEQ ID NO: 51. According to a specific embodiment, the anti-CD3 antibody has a heavy chain variable region sequence set forth in SEQ ID NO: 44 and a light chain variable region sequence set forth in SEQ ID NO: 52. According to a specific embodiment, the anti-CD3 antibody has a heavy chain variable region sequence set forth in SEQ ID NO: 44 and a light chain variable region sequence set forth in SEQ ID NO: 53. According to a specific embodiment, the anti-CD3 antibody has a heavy chain variable region sequence set forth in SEQ ID NO: 45 and a light chain variable region sequence set forth in SEQ ID NO: 51. According to a specific embodiment, the anti-CD3 antibody has a heavy chain variable region sequence set forth in SEQ ID NO: 45 and a light chain variable region sequence set forth in SEQ ID NO: 52. According to a specific embodiment, the anti-CD3 antibody has a heavy chain variable region sequence set forth in SEQ ID NO: 45 and a light chain variable region sequence set forth in SEQ ID NO: 53. In the antibody, the heavy chain constant domain sequence is set forth in SEQ ID NO: 57, and the light chain constant domain sequence is set forth in SEQ ID NO: 55. According to a specific embodiment, the anti-CD3 antibody provided binds to human CD3 or monkey (e.g., cynomolgus monkey) CD3 with an EC50 of 10 nM or less; for example, the anti-CD3 antibody provided has an affinity EC50 value of less than 5 nM, less than 4 nM, less than 3 nM, less than 2 nM, less than 1 nM, less than 0.8 nM, less than 0.5 nM, less than 0.3 nM, or less than 0.1 nM, or less than 0.05 nM for CD3.

The technical solutions of the present disclosure are described in detail below with reference to examples. It should be noted that these examples are merely used to facilitate understanding for a person skilled in the art and should not be construed as limiting the protection scope of the present disclosure. The examples of the present disclosure are described in detail below. The described examples are illustrative, are intended to explain the present disclosure, and should not be construed as limiting the present disclosure.

### Examples

The cell strains used in the examples were as follows:
The human ovarian cancer cell strain SKOV3, the human colorectal cancer cell strain HCT116, and the human pancreatic cancer cell strain PANC-1 were all purchased from ATCC.

The human liver cancer cell strain SK-HEP-1, the human glioma cell strain U251, and the human melanoma cell strain A375 were purchased from Cell Bank, Chinese Academy of Sciences, Shanghai.

The cell strain MC38 was purchased from Nanjing Cobioer Biosciences Co., Ltd.

The human embryonic kidney 293T cells were from Shanghai Genechem Co., Ltd.

The human peripheral blood mononuclear cells (PBMCs) were purchased from Shanghai Maishun.

The Jurkat cells were purchased from Shanghai ChemPartner.

SKOV3 and HCT116 were cultured with McCoy 5A (purchased from Gibco) + 10% FBS (purchased from PAN). SK-HEP-1 and U251 were cultured with MEM (purchased from Gibco) + 10% FBS. PANC-1, A375, and 293T were cultured with DMEM (purchased from Gibco) + 10% FBS.

The cytometry buffer was 1× DPBS + 2% FBS. The 1× DPBS was purchased from Biosharp Biotechnology Co., Ltd., and the FBS was purchased from PAN.

### Packaging of LV-TT3-GFP lentivirus

293T cells were digested with 0.25% trypsin (purchased from Beyotime) into single cells and resuspended in a packing medium at 2 × 10⁶/mL. The formula for the packaging medium was Opti-MEM (purchased from Gibco) + 5% FBS (purchased from Gibco) + sodium pyruvate (purchased from Gibco; final concentration: 200 mM). The cells were plated at 1 mL/well in a 6-well plate. A lentiviral plasmid mixture was prepared according to the instructions for use of Lipo3000: (1) 250 µL of Opti-MEM + 7 µL of Lipo3000, and (2) 250 µL of Opti-MEM + 6 µL of p3000 + lentiviral 4 plasmids (molar mass ratio = 1:1:1:1). Then (1) and (2) were well mixed, and the mixture was incubated for 15 min at room temperature. The plasmid mixture was added to the 6-well plate at 500 µL/well, and the contents in the wells were well mixed. Then the plate was further incubated in an incubator. After 48 h of transfection, the culture solution was harvested and centrifuged at 3000× rpm for 10 min, and the supernatant virus solution was collected, aliquoted, and cryopreserved at -80 °C for later use.

### Construction of cell strain HCT116 stably expressing TT3 (HCT116-TT3) and SK-HEP-1 stably expressing TT3 (SK-HEP-1-TT3)

Wild-type HCT116 was digested with 0.25% trypsin into single cells and plated at 1 × 10⁵/500 µL/ well in a 48-well plate, and 300 µL of lentivirus LV-TT3-GFP and polybrene (purchased from Shanghai Yeasen Biotech Co., Ltd.; final concentration: 8 µg/mL) were added. The cells were cultured in an incubator and passaged for expansion. After one week, the infected cells were digested, plated at 2 cells/well in a 96-well plate at 100 µL/well, and further cultured in an incubator. After about 10 days of continuous culture, monoclonal cells were obtained. Wells in which all cells were positive for GFP and only a cluster of cell clones was present were selected under a microscope, and the cells were passaged for expansion. The expression of GFP and TT3 was assessed by flow cytometry. Screening was performed based on the expression ratio and intensity of GFP and TT3, and optimal single clones were finally obtained. A stably transfected cell strain expressing the TT3 antigen (HCT116-TT3) was obtained.

Similarly, a stably transfected cell strain expressing the TT3 antigen, SK-HEP-1-TT3, was constructed.

### Preparation of T cells

PBMCs were thawed, incubated in a complete culture medium in an incubator for 2 h, plated at 5 × 10⁵/500 µL/well in a 24-well plate, and activated with 10 µL of Dynabeads^{®} Human T-Activator CD3/CD28 (purchased from Gibco). A complete culture medium was added every other day based on the growth of the cells. The culture was continued until the 10th day. The magnetic beads were removed, and the cells were cryopreserved for later use. The complete culture medium was AIM-V + 5% FBS + hIL-2 (final concentration: 100 IU/mL). The AIM-V was purchased from Gibco, and the human recombinant interleukin 2 (hIL-2) was purchased from Jiangsu Kingsley Pharmaceutical Co., Ltd.

Construction of poxvirus (DDVV-TT3): The poxvirus DDVV-TT3 can be obtained with reference to a method in a PCT patent with International Publication No. WO2020038490A (International Application No. PCT/CN2019/102480). It should be noted that in the patent WO2020038490A, the poxvirus was represented by vvDD-TT3. Both represent the same recombinant poxvirus.

The mentioned humanized anti-CD3 monoclonal antibody and the activity characterization results were specifically as follows:
Humanized sequence templates were retrieved from the database based on the SP34 antibody sequence, and the importance of different amino acids was analyzed based on the 3D structure. If necessary, key amino acids that affect the structure were back-mutated. Corresponding humanized antibodies were determined. The humanized antibodies were fused with human-derived constant region sequences and constructed into the pcDNA3.1 mammal expression vector to construct chimeric antibody forms. The different antibodies obtained were then expressed using a mammalian cell system and purified, and different antibodies with at least 90% or higher purity were obtained. The expressed antibody sequences were CD3 Ab01 to CD3 Ab15. The expressed antibodies were IgG-type antibodies. The heavy chain constant domain of the antibodies is set forth in SEQ ID NO: 57, and the light chain constant domain of the antibodies is set forth in SEQ ID NO: 55. The sequences of the heavy chain variable region and the light chain variable region of these antibodies are numbered herein as follows: The heavy chain variable region of antibody CD3 Ab01 is set forth in SEQ ID NO: 43, and its light chain variable region is set forth in SEQ ID NO: 46. The heavy chain variable region of antibody CD3 Ab02 is set forth in SEQ ID NO: 43, and its light chain variable region is set forth in SEQ ID NO: 47. The heavy chain variable region of antibody CD3 Ab03 is set forth in SEQ ID NO: 43, and its light chain variable region is set forth in SEQ ID NO: 48. The heavy chain variable region of antibody CD3 Ab04 is set forth in SEQ ID NO: 43, and its light chain variable region is set forth in SEQ ID NO: 49. The heavy chain variable region of antibody CD3 Ab05 is set forth in SEQ ID NO: 43, and its light chain variable region is set forth in SEQ ID NO: 50. The heavy chain variable region of antibody CD3 Ab06 is set forth in SEQ ID NO: 44, and its light chain variable region is set forth in SEQ ID NO: 46. The heavy chain variable region of antibody CD3 Ab07 is set forth in SEQ ID NO: 44, and its light chain variable region is set forth in SEQ ID NO: 47. The heavy chain variable region of antibody CD3 Ab08 is set forth in SEQ ID NO: 44, and its light chain variable region is set forth in SEQ ID NO: 48. The heavy chain variable region of antibody CD3 Ab09 is set forth in SEQ ID NO: 44, and its light chain variable region is set forth in SEQ ID NO: 49. The heavy chain variable region of antibody CD3 Ab10 is set forth in SEQ ID NO: 44, and its light chain variable region is set forth in SEQ ID NO: 50. The heavy chain variable region of antibody CD3 Ab11 is set forth in SEQ ID NO: 45, and its light chain variable region is set forth in SEQ ID NO: 46. The heavy chain variable region of antibody CD3 Ab12 is set forth in SEQ ID NO: 45, and its light chain variable region is set forth in SEQ ID NO: 47. The heavy chain variable region of antibody CD3 Ab13 is set forth in SEQ ID NO: 45, and its light chain variable region is set forth in SEQ ID NO: 48. The heavy chain variable region of antibody CD3 Ab14 is set forth in SEQ ID NO: 45, and its light chain variable region is set forth in SEQ ID NO: 49. The heavy chain variable region of antibody CD3 Ab15 is set forth in SEQ ID NO: 45, and its light chain variable region is set forth in SEQ ID NO: 50.

The activity of the prepared humanized antibodies was assessed by ELISA as follows: hCD3 (human CD3 antigen) was diluted to 0.5 µg/mL using a coating solution and then added to a 96-well plate at 100 µL/well, and the plate was incubated overnight at 4 °C. The plate was washed with PBST. Then 200 µL of a blocking solution was added to each well, and blocking was performed at a temperature of 37 °C for 2 h. The plate was washed with PBST. Then diluted antibodies (the initial concentration was 40 nM, and serial dilution was performed in a 1:6 ratio) were added at 100 µL/well, and the plate was incubated at a temperature of 37 °C for 2 h. The plate was washed with PBST. Then an HRP-labeled goat anti-human IgG was added at 100 µL/well, and the plate was incubated at 37 °C for 1 h. The plate was washed with PBST, and TMB was added at 100 µL/well. Color development was performed at 37 °C for 10-20 min, and 50 µL of 2 M H₂SO₄ was added to each well to stop the reaction. Readings were taken at 450 nm. The EC50 results for antibodies CD3 Ab01, CD3 Ab02, CD3 Ab03, CD3 Ab04, CD3 Ab05, CD3 Ab06, CD3 Ab07, CD3 Ab08, CD3 Ab09, CD3 Ab10, CD3 Ab11, CD3 Ab12, CD3 Ab13, CD3 Ab14, and CD3 Ab15 were 0.0096 nM, 0.0117 nM, 0.0042 nM, 0.0068 nM, 0.0099 nM, 0.0126 nM, 0.0129 nM, 0.0116 nM, 0.0080 nM, 0.0020 nM, 0.0118 nM, 0.0095 nM, 0.0099 nM, 0.0087 nM, and 0.0099 nM, respectively.

The binding activity of the prepared humanized antibodies was assessed by FACs as follows: Jurkat cells or 293T-CD3 cells were plated, washed with a FACS buffer, and centrifuged at 1200 rpm for 3-5 min. Then diluted antibodies with different concentrations were added. The cells were incubated at 4 °C for 0.5 h, centrifuged at 1200 rpm for 3-5 min, and washed with a FACS buffer. Then a goat anti-human IgG secondary antibody (manufacturer: Abcam, ab98596) was added at 100 µL/well. The cells were incubated at 4 °C for 0.5 h, centrifuged at 1200 rpm for 3-5 min, washed with a FACs buffer, and resuspended in 50 µL of a FACs buffer, and assays were performed using a flow cytometer. The FACs assay results, EC50 results, for antibodies CD3 Ab01, CD3 Ab02, CD3 Ab03, CD3 Ab04, CD3 Ab05, CD3 Ab06, CD3 Ab07, CD3 Ab08, CD3 Ab09, CD3 Ab10, CD3 Ab11, CD3 Ab12, CD3 Ab13, CD3 Ab14, and CD3 Ab15 with Jurkat cells were 3.299 nM, 2.642 nM, 2.284 nM, 1.182 nM, 1.024 nM, 3.309 nM, 2.415 nM, 1.823 nM, 0.862 nM, 0.784 nM, 4.451 nM, 3.518 nM, 3.194 nM, 1.142 nM, and 1.108 nM, respectively, and their FACs assay results, EC50 results, with 293T-CD3 cells were 22.600 nM, 12.300 nM, 10.060 nM, 5.234 nM, 4.267 nM, 12.990 nM, 9.020 nM, 4.238 nM, 4.401 nM, 2.438 nM, 21.550 nM, 15.770 nM, 12.360 nM, 5.728 nM, and 3.479 nM, respectively.

The mentioned humanized anti-TT3 monoclonal antibody and the activity characterization results were specifically as follows:
The antibody (No. Ab-TT3; its heavy chain variable region was SEQ ID NO: 26, and its light chain variable region was SEQ ID NO: 27) was humanized. Specifically, humanization sequence templates were retrieved from the database based on the Ab-TT3 antibody sequence. To maintain the conformation of the CDR regions, the main focuses were three types of residues, including residues on the binding interface of the VL and VH, residues close to the CDR regions and embedded within the protein, and residues having direct interactions with the CDR regions, the interactions including: hydrophobic interactions, hydrogen bonds, and salt bridges. The importance of different amino acids was analyzed based on the 3D structure. If necessary, key amino acids that affect the structure were back-mutated. Corresponding humanized antibodies were determined.

The humanized antibodies were fused with human-derived constant region sequences and constructed into the pcDNA3.1 mammal expression vector to construct chimeric antibody forms. Then the different antibodies obtained were expressed using a mammalian cell system and purified, and different antibodies with at least 90% or higher purity were obtained. The expressed antibodies were TT3 Ab-1, TT3 Ab-2, and TT3 Ab-3, in which the heavy chain constant domain of the antibodies is set forth in SEQ ID NO: 57, and the light chain constant domain of the antibodies is set forth in SEQ ID NO: 55. TT3 Ab-1 has a heavy chain variable region set forth in SEQ ID NO: 29 and a light chain variable region set forth in SEQ ID NO: 32. TT3 Ab-2 has a heavy chain variable region set forth in SEQ ID NO: 30 and a light chain variable region set forth in SEQ ID NO: 33. TT3 Ab-3 has a heavy chain variable region set forth in SEQ ID NO: 31 and a light chain variable region set forth in SEQ ID NO: 34. Meanwhile, the Fc region of antibody TT3 Ab-3 was mutated. The mutated antibody was numbered TT3 Ab-3mu; its heavy chain constant domain is set forth in SEQ ID NO: 54, and its light chain constant domain is set forth in SEQ ID NO: 55.

The activity of the prepared humanized antibodies was assessed by ELISA as follows: The TT3 antigen was diluted to 0.5 µg/mL using a coating solution and added to a 96-well plate at 100 µL/well, and the plate was incubated overnight at 4 °C. The plate was washed with PBST. Then 200 µL of an antibody blocking solution was added to each well, and blocking was performed at 37 °C for 2 h. The plate was washed with PBST. Different antibodies (initial concentration: 100 nM; serially diluted in a 1:6 ratio) were added at 100 µL/well, and the plate was incubated at 37 °C for 2 h. The plate was washed with PBST. An HRP-bound goat anti-human/mouse IgG antibody was added at 100 µL/well, and the plate was incubated at 37 °C for 1 h. The plate was washed with PBST, and TMB was added at 100 µL/well. Color development was performed at 37 °C for 10-20 min, and 50 µL of 2 M sulfuric acid was added to each well to stop the reaction. OD readings were taken at a wavelength of 450 nm. The EC50 values for antibodies TT3 Ab-1, TT3 Ab-2, TT3 Ab-3, and Ab-TT3 were 0.0104 nM, 0.0105 nM, 0.0140 nM, and 0.0878 nM, respectively.

The activity of the prepared humanized antibodies was assessed by FACs as follows: Different cells (HCT116-TT3, SK-HEP-1-TT3, and MC38-TT3) were plated, then washed with a FACS buffer, and centrifuged at 1200 rpm for 3-5 min. Diluted antibodies with different concentrations (initial concentration: 100 nM; serially diluted in a 1:5 ratio) were added. The cells were incubated at 4 °C for 0.5 h and centrifuged at 1200 rpm for 3-5 min. The cells were washed with a FACS buffer. Then a goat anti-human IgG secondary antibody (Abcam, ab98596) was diluted in a 1:250 ratio and added to the plates at 100 µL/well. The cells were incubated at 4 °C for 0.5 h, centrifuged at 1200 rpm for 3-5 min, washed with a FACs buffer, and resuspended in 50 µL of a FACs buffer, and assays were performed using a flow cytometer. The FACs affinity activity EC50 results for antibodies TT3 Ab-1, TT3 Ab-2, and TT3 Ab-3 with HCT116-TT3 cells were 0.568 nM, 0.958 nM, and 0.692 nM, respectively, their FACs affinity activity EC50 results with SK-HEP-1-TT3 cells were 0.546 nM, 1.527 nM, and 0.738 nM, respectively, and their FACs affinity activity EC50 results with MC38-TT3 cells were 1.201 nM, 2.327 nM, and 0.588 nM, respectively.

The antibody with Fc mutation (TT3 Ab-3mu) was tested using the same method. The results show that the antibody can bind to the TT3 antigen both before Fc mutation and after Fc mutation; the antibody exhibited biological activity in both cases, and there was not a big difference in binding activity.

### Example 1

Bispecific antibody BK-A-101 was prepared through a genetic engineering method. Specifically, nucleotide sequences encoding the heavy chain moiety and the light chain moiety of the bispecific antibody were synthesized and then cloned into a pcDNA3.1 expression vector (purchased from GenScript) using a conventional technique in the art. Then the vector was transiently transfected into CHO-S cells through electroporation, and the bispecific antibody protein was expressed. The supernatant was collected and purified using a protein A affinity column. The purity of the purified antibody was measured by HPLC and SDS-PAGE, and the measurements show that the purity of bispecific antibody BK-A-101 was above 90%. The binding activity of bispecific antibody BK-A-101 to the target antigens was then assessed by flow cytometry. Similarly, other bispecific antibodies of the BK-A structure (including BK-A-101-hu1, BK-A-101-hu2, BK-A-101-hu3, BK-A-101-hu4, BK-A-101-hu5, and BK-A-101-hu6) were obtained.

Similarly, bispecific antibodies of the BK-B structure (BK-B-110, BK-B-110-hu1, BK-B-110-hu2, BK-B-110-hu3, BK-B-110-hu4, BK-B-110-hu5, and BK-B-110-hu6), bispecific antibodies of the BK-C structure (BK-C-120, BK-C-120-hu1, BK-C-120-hu2, BK-C-120-hu3, BK-C-120-hu4, BK-C-120-hu5, and BK-C-120-hu6), and bispecific antibodies of the BK-D structure (including BK-D-01, BK-D-02, BK-D-03, BK-D-04, BK-D-05, BK-D-06, BK-D-07, BK-D-08, BK-D-09, BK-D-10, BK-D-11, BK-D-12, and BK-D-13) were prepared by transient transfection of 293F cells through electroporation.

The binding activity of the bispecific antibodies to T cells was assessed by flow cytometry (FACS) as follows: T cells were resuspended in a flow cytometry buffer, and the suspension was aliquoted in 96-well plates. Then different antibodies were prepared for incubation. Group 1 was a blank control group, in which no antibody was added for staining. In group 2, an isotype control antibody (negative control, human IgG1, purchased from Biolegend) was added, with a final concentration of 50 nM. In group 3, an anti-CD3 monoclonal antibody (L2K antibody, purchased from Kyinno, the same as the anti-CD3 monoclonal antibody used below) was added, with a final concentration of 50 nM. In group 4, bispecific antibody BK-A-101 was added, with a final concentration of 50 nM. After each group was incubated at 2-8 °C for 30 min, the cells were diluted, washed, and resuspended with a flow cytometry buffer. A PE-labeled anti-human IgG secondary antibody (purchased from ABCAM, diluted in a 1:200 ratio) was added to each group, and the plates were incubated at 2-8 °C for 30 min. The cells were diluted and washed with a flow cytometry buffer and then resuspended in 200 µL of a flow cytometry buffer, and the suspensions were assayed on a flow cytometer (Novocyte). The results are shown in FIG. 5. The flow cytometry results for the blank control and the negative control are negative. The positive control exhibited a positive rate of 99.45%, as determined by flow cytometry. The bispecific antibody exhibited a positive rate of 99.44%. The results show that bispecific antibody BK-A-101 has relatively high binding activity to T cells.

The binding activity of bispecific antibody BK-A-101 to HCT116 cells or HCT116-TT3 cells was assessed by flow cytometry (FACS) as follows: HCT116 and HCT116-TT3 cells were separately digested with 0.25% trypsin into single cells and resuspended in a flow cytometry buffer. Bispecific antibody BK-A-101 was added, with final concentrations of 0.1 nM, 1 nM, 10 nM, and 100 nM. After thorough mixing, the cells were incubated at 2-8 °C for 30 min. The cells were diluted and washed with a flow cytometry buffer and then resuspended. A PE-labeled anti-human IgG secondary antibody (diluted in a 1:200 ratio) was added to each group, and the cells were incubated at 2-8 °C for 30 min. The cells were diluted and washed with a flow cytometry buffer and then resuspended in 200 µL of a flow cytometry buffer, and the suspensions were assayed on a flow cytometer (Novocyte). The results are shown in FIGs. 6 and 7.

FIG. 6 shows the binding activity of the bispecific antibody to cell strain HCT116. The results show that even with the increase in the concentration of the bispecific antibody, it did not exhibit binding activity to cell strain HCT116. FIG. 7 shows the binding activity of bispecific antibody BK-A-101 to HCT116-TT3 cells. The results show that as the concentration of the bispecific antibody increases, the positive rate determined by flow cytometry shows an upward trend. The results in FIGs. 6 and 7 show the binding specificity of the bispecific antibody for TT3.

Similarly, the binding activity of the bispecific antibody to cell strain SK-HEP-1 and cell strain SK-HEP-1-TT3 was assessed. The assay results show that the bispecific antibody did not exhibit binding activity to cell strain SK-HEP-1, but exhibited binding activity for SK-HEP-1-TT3. Moreover, as the concentration of the bispecific antibody increases, the binding ratio shows an upward trend, further indicating the binding specificity of the bispecific antibody for TT3.

The affinity activity (i.e., affinity) of the bispecific antibodies (BK-D-01, BK-D-02, BK-D-03, BK-D-04, BK-D-05, BK-D-06, BK-D-07, BK-D-08, BK-D-09, BK-D-10, BK-D-11, and BK-D-12) for the CD3 end and the TT3 end was assessed by FACs. The assessment method and the results were as follows:
Jurkat cells were plated, washed with a FACS buffer, and centrifuged at 1200 rpm for 3-5 min. Then diluted antibodies with different concentrations were added at 100 µL/well. The cells were incubated at 4 °C for 0.5 h, centrifuged at 1200 rpm for 3-5 min, and washed with a FACS buffer. Then a diluted goat anti-human IgG (FITC, Abcam/ab97224) antibody was added. The cells were incubated at 4 °C for 0.5 h, centrifuged at 1200 rpm for 3-5 min, washed with a FACS buffer, and resuspended in 50 µL of a FACs buffer, and assays were performed using a flow cytometer.

Similarly, the affinity activity of the bispecific antibodies for the TT3 end was assessed as follows: HCT116-TT3 cells were plated, washed with a FACS buffer, and centrifuged at 1200 rpm for 3-5 min. Then diluted antibodies with different concentrations were added. The cells were incubated at 4 °C for 0.5 h, centrifuged at 1200 rpm for 3-5 min, and washed with a FACS buffer. A diluted goat anti-human IgG Fc (Dylight 650, Abcam/ab98622) antibody was added. The cells were incubated at 4 °C for 0.5 h, centrifuged at 1200 rpm for 3-5 min, washed with a FACS buffer, and resuspended in 50 µL of a FACs buffer, and assays were performed using a flow cytometer.

The corresponding assay results are shown in the table below and FIG. 8:

**Table 1. The affinity activity of the bispecific antibodies for the CD3 end and the TT3 end determined by FACs**

| Bispecific antibody No. | CD3 end EC50 (nM) | HCT116-TT3 cells TT3 end EC50 (nM) |
|---|---|---|
| BK-A-101 | 0.80 | 15.80 |
| BK-D-01 | 62.55 | 0.070 |
| BK-D-02 | 80.30 | 0.099 |
| BK-D-03 | 111.10 | 0.464 |
| BK-D-04 | 18.16 | 0.470 |
| BK-D-05 | 71.40 | 1.038 |
| BK-D-06 | 32.59 | 0.908 |
| BK-D-07 | 49.40 | 0.127 |
| BK-D-08 | 135.60 | 0.368 |
| BK-D-09 | 97.81 | - |
| BK-D-10 | 142.90 | 0.248 |
| BK-D-11 | 169.00 | 0.336 |
| BK-D-12 | 80.59 | 0.523 |
| hIgG1 | NA | NA |

In Table 1, "-" indicates that the experiment was not performed.

The experimental results show that: the prepared bispecific antibodies exhibited high affinity activity for the TT3 end and the CD3 end. Similarly, the affinity of the bispecific antibodies for the TT3 end was assessed using SKHEP1-TT3 cells or MC38-TT3 cells, and the results show that the bispecific antibodies exhibited high affinity activity for the TT3 end.

Other bispecific antibodies of the BK-B and BK-D structures also exhibited similar affinity activity for the CD3 end and the TT3 end.

Further, the non-specific binding of the bispecific antibodies to a wild-type tumor cell strain was assessed by FACS as follows: HCT116 and SK-HEP-1 cells were separately digested with 0.25% trypsin into single cells and resuspended in a flow cytometry buffer. Bispecific antibodies BK-A-101 and BK-D-01 to 09 were added respectively, with final concentrations of 10 nM and 100 nM respectively. After thorough mixing, the cells were incubated at 2-8 °C for 30 min. The cells were diluted and washed with a flow cytometry buffer and then resuspended. A APC-labeled anti-human IgG secondary antibody (diluted in a 1:200 ratio) was added to each group, and the cells were incubated at 2-8 °C for 30 min. The cells were diluted and washed with a flow cytometry buffer and then resuspended in 200 µL of a flow cytometry buffer, and the suspensions were assayed on a flow cytometer (Novocyte). The results are shown in FIG. 9. The groups of bispecific antibodies BK-A-101 and BK-D-01 to 09 exhibited relatively low non-specific binding ratios at a general concentration of 10 nm. The groups of bispecific antibodies BK-D-01, 02, 03, 04, 05, 06, and 08 also exhibited relatively low non-specific binding ratios even at a very high concentration of 100 nm. This indicates that in the absence of specific targets, the bispecific antibodies of the present disclosure exhibited relatively low levels of non-specific binding, suggesting that they have a relatively good safety profile.

### Example 2

Example 2 provides an assessment of the killing effects of bispecific antibodies, combined with pre-activated expanded T cells, on cell strains HCT116 and HCT116-TT3 using an RTCA real-time killing system.

First, 96 E-Plates (purchased from Agilent Technologies, Inc.) were equilibrated with 50 µL of McCoy 5A + 10% FBS culture medium. Wild-type HCT116 and HCT116-TT3 were then taken, separately digested with 0.25% trypsin into single cells, and resuspended in McCoy 5A + 10% FBS culture medium, and the tumor cells were plated at 5 × 10³ cells/50 µL/well in the 96 E-Plates. Five hours after plating, T cells were added to the corresponding wells of the 96 E-Plates in a cell ratio of E:T = 2:1 (E:T refers to the effector-to-target ratio, which represents the ratio of effector cells to target cells) at 50 µL/well. Meanwhile, the bispecific antibody (BK-A-101) or anti-CD3 monoclonal antibody (CD3 Ab) was serially diluted and then added to the corresponding wells of the 96 E-Plates, with the final concentrations of BK-A-101 being 10 nM, 1 nM, and 0.1 nM and the final concentrations of CD3 Ab being 10 nM, 1 nM, and 0.1 nM. The final volume in each well was 200 µL. The system was set to perform a scan and collect data every 15 min.

The results are shown in FIG. 10. T cells acting alone and the various concentrations of CD3 Ab or BK-A-101, combined with T cells, did not exhibit a significant killing effect on wild-type HCT116, and T cells acting alone and the various concentrations of CD3 Ab, combined with T cells, did not exhibit a significant killing effect on HCT116-TT3 either. However, the various concentrations of BK-A-101, combined with T cells, exhibited a very significant killing effect on HCT116-TT3, and the effect was dose-dependent on the concentration of BK-A-101. Specifically, 1 nM and 1 nM BK-A-101, combined with T cells, can almost completely eliminate HCT116-TT3 with relatively good persistence, while 0.1 nM BK-A-101, combined with T cells, can almost completely eliminate HCT116-TT3 before 40 h.

Similarly, results for the bispecific antibodies of the BK-D structure were obtained, with the difference lying in that after the tumor cell strain was plated and incubated overnight, effector cells and bispecific antibodies were added, with a ratio of effector cells to target cells of 5:1. The results are shown in FIG. 11. A and B of FIG. 11 show the killing effects of bispecific antibodies, combined with pre-activated expanded T cells, on cell strain HCT116-TT3 at different concentrations (0.1 nM or 1 nM), respectively, wherein labels 1-9 represent the groups of HCT116-TT3 + T cells + bispecific antibodies BK-D-01 to BK-D-09, respectively, label 10 represents the HCT116-TT3 + T cell + BK-A-101 group, label 11 represents HCT116-TT3 cells only, without the treatment with bispecific antibodies and T cells, and label 12 represents HCT116-TT3 cells treated with T cells only, without the addition of bispecific antibodies. From the results, it can be seen that at each concentration, the bispecific antibodies of the present disclosure, combined with T cells, can almost completely eliminate specific cancer cells with very good persistence, showing excellent tumor cell killing effects.

Similarly, the killing of wild-type tumor cell strains HCT116 (A of FIG. 12) and SK-HEP-1 (B of FIG. 12) by bispecific antibodies at high concentrations was assessed, wherein labels 1-6 represent the groups of the wild-type tumor cell strain + T cells + bispecific antibodies BK-D-01, 04, 05, 06, 07, and 08, respectively, label 7 represents the wild-type tumor cell strain + T cell + TT3 monoclonal antibody-1 group, label 8 represents the wild-type tumor cell strain + T cell + CD3 monoclonal antibody SP34 group, label 9 represents wild-type tumor cell strain cells only, without the treatment with antibodies and T cells, and label 10 represents wild-type tumor cell strain cells treated with T cells only, without the addition of antibodies. In A of FIG. 12, the concentration of the bispecific antibodies was 100 nM; in B of FIG. 12, the concentration of the bispecific antibodies was 10 nM. The results are shown in FIG. 12. Bispecific antibodies BK-D-04, 05, 06, 07, and 08 of the present disclosure did not exhibit a significant killing effect on the wild-type cell strains even at very high concentrations, indicating that these bispecific antibodies have relatively good killing specificity.

### Example 3

Example 3 provides an assessment of the killing effect of a bispecific antibody, combined with PBMCs, on cell strain HCT116 using an RTCA real-time killing system.

PBMCs were thawed, then resuspended in a complete culture medium, and restored overnight in an incubator. The next day, first, a 96 E-Plate (Agilent) was equilibrated with 50 µL of McCoy 5A + 10% FBS culture medium. HCT116-TT3 was then taken, digested with 0.25% trypsin into single cells, and resuspended in McCoy 5A + 10% FBS culture medium, and the tumor cells were plated at 5 × 10³ cells/50 µL/well in the 96 E-Plate. Five hours after plating, PBMCs were added to the corresponding wells of the 96 E-Plate in a cell ratio of E:T = 2:1 at 50 µL/well. Meanwhile, BK-A-101 was serially diluted and then added to the corresponding wells of the 96 E-Plate, with the final concentrations of BK-A-101 being 10 nM and 1 nM. The final volume in each well was 200 µL. The system was set to perform a scan and collect data every 15 min.

The results are shown in FIG. 13. PBMCs acting alone did not exhibit a significant killing effect on HCT116-TT3. However, the various concentrations of BK-A-101, combined with PBMCs, exhibited a very significant killing effect on HCT116-TT3. 10 nM and 1 nM BK-A-101, combined with PBMCs, can almost completely eliminate HCT116-TT3 with relatively good persistence.

### Example 4

In Example 4, the binding of BK-A-101 after human tumor cell strains were infected with DDvv-TT3 was assessed by flow cytometry (FACS) as follows:
SKOV3, PANC1, A375, and U251 were digested with 0.25% trypsin into single cells and then resuspended in their respective complete culture medium, and the cells were plated at 1 × 10⁵/2 mL/well in 6-well plates. The next day, the 6-well plates were taken out, and the culture medium was discarded. The DDvv-TT3 recombinant oncolytic poxvirus was serially diluted with a corresponding culture medium supplemented with 5% FBS and then added at 500 µL/well, with MOI = 2, 0.2, 0.02. The 6-well plates were gently shaken for good uniformity, incubated in a 37 °C incubator for 2 h, and then taken out, and 1.5 mL of a culture medium containing 5% FBS was added to each well to make a volume of 2 mL. The plates were gently shaken for good uniformity and then further incubated in the incubator. After 48 h of viral infection, cells were harvested and resuspended in a flow cytometry buffer. The following antibodies were added, and the cells were incubated at 2-8 °C for 30 min.
Group 1: isotype control antibody (isotype, human IgG1, negative control, final concentration: 100 nM),
Group 2: bispecific antibody BK-A-101 (final concentration: 100 nM), and
Group 3: anti-Strep tag II antibody (positive control, anti-TT3 monoclonal antibody, purchased from GenScript, final concentration: 10 µg/mL).

After the cells were washed and resuspended with a flow cytometry buffer, a PE-labeled anti-human IgG secondary antibody (purchased from ABCAM, diluted in a 1:200 ratio) was added to groups 1 and 2, and a PE-labeled anti-mouse IgG antibody (purchased from ABCAM, diluted in a 1:200 ratio) was added to group 3. The cells were incubated at 2-8 °C for 30 min. The cells were diluted and washed with a buffer and then resuspended in 200 µL of a buffer, and the suspensions were assayed on a flow cytometer (Novocyte).

The results are shown in FIG. 14. As the MOI value increases, the flow cytometry assay results for the isotype control group are all negative, and the flow cytometry assay results for the positive control and the bispecific antibody staining group show upward trends. The experimental results show that: after the human tumor cell strains were infected with DDvv-TT3, adding the bispecific antibody can result in the specific binding to tumor cells infected with the oncolytic virus; moreover, as the MOI value increases, the binding percentage increases.

### Example 5 (Experiment of Stimulating Proliferation and Activation of T Cells)

In Example 5, after bispecific antibodies were co-incubated with *in vitro* cultured T cells (shown in A of FIG. 15) or PBMCs (shown in B of FIG. 15) for 3 days, the activation of T cells was assessed by flow cytometry (FACS) as follows:
Pre-activated expanded T cells or PBMCs were resuspended in a complete culture medium, and the cells were plated at 1 × 10⁵/150 µL/well in a 48-well plate. After cell plating, 150 µL of a bispecific antibody diluted to different concentrations was added to the corresponding wells, with the final concentrations of the bispecific antibody being 0.01 nM, 0.1 nM, 1 nM, 10 nM, 100 nM, and 250 nM. In the Blank group, no bispecific antibodies were added; only the same volume of a blank culture medium was added. After 3 days of culture, the 48-well plate was taken out, each group of cells was harvested, diluted and washed with a flow cytometry buffer, and then resuspended. In each group, FITC anti-human CD3, PE anti-human CD4, APC-Cy7 anti-human CD25, and APC anti-human CD127 were added. After thorough mixing, the cells were incubated at 2-8 °C for 30 min. The cells were diluted and washed with a flow cytometry buffer and then resuspended in 200 µL of a flow cytometry buffer, and the suspensions were assayed on a flow cytometer (Novocyte). The results are shown in FIG. 15. The groups of bispecific antibodies BK-D-01 to 09 all exhibited relatively low expression ratios of activation marker CD25 on T cell surfaces at general concentrations of 0.01 nm, 0.1 nm, 1 nm, 10 nm, etc. The groups of bispecific antibodies BK-D-04 to 09 also exhibited relatively low expression ratios of activation marker CD25 on T cell surfaces at even very high concentrations of 100 nm, 250 nm, etc. These results indicate that in the absence of specific targets, the bispecific antibodies of the present disclosure exhibited relatively low capabilities to activate T cells, suggesting that they have a relatively good safety profile.

In Example 5, after bispecific antibodies were co-incubated with PBMCs and cell strain SK-HEP1 for 3 days, the release of cytokine IFN-γ in the culture supernatant (FIG. 16) was assessed by ELISA, with the final concentrations of the bispecific antibodies being 0.1 nM, 1 nM, 10 nM, 100 nM, and 250 nM. A of FIG. 16 shows results for the 3-day co-incubation of PBMCs with bispecific antibodies. B of FIG. 16 shows results for the 3-day co-incubation of PBMCs with bispecific antibodies and the wild-type SK-HEP-1 cell group. C of FIG. 16 shows results for the 3-day co-incubation of PBMCs with bispecific antibodies and SK-HEP-1-TT3. The results show that: when the bispecific antibodies were co-cultured with PBMCs or the bispecific antibodies were co-cultured with PBMCs and SK-HEP-1, the groups of bispecific antibodies BK-D-01, and 04 to 08 all exhibited relatively low levels of IFN-γ secretion at general concentrations of 0.1 nm, 1 nm, 10 nm, etc. When the bispecific antibodies were co-cultured with PBMCs, the groups of bispecific antibodies BK-D-04, 05, and 06 also exhibited relatively low levels of IFN-γ secretion even at very high concentrations of 100 nm, 250 nm, etc. These results indicate that in the absence of specific targets, the bispecific antibodies of the present disclosure have relatively low capabilities to activate T cells, suggesting that they have a relatively good safety profile. When the bispecific antibodies were co-cultured with PBMCs and SK-HEP-1-TT3, all bispecific antibody groups exhibited secretion of large amounts of IFN-γ, indicating that in the presence of specific targets, the bispecific antibodies of the present disclosure can relatively strongly activate T cells.

### Example 6

Example 6 provides an assessment of the killing effects of bispecific antibodies, combined *in vitro* with the recombinant oncolytic poxvirus DDvv-TT3 and pre-activated expanded T cells, on cell strain HCT116 using an RTCA real-time killing system (as shown in FIG. 17).

First, a 96 E-Plate (purchased from Agilent Technologies, Inc.) was equilibrated with 50 µL of McCoy 5A + 10% FBS culture medium. Wild-type HCT116 was then taken, digested with 0.25% trypsin into single cells, and resuspended in McCoy 5A + 10% FBS culture medium, and the tumor cells were plated at 5 × 10³ cells/50 µL/well in the 96 E-Plate. After the cells were plated and incubated overnight, DDvv-TT3 was plated at 50 µL/well in the 96 E-Plate (MOI = 1). After the oncolytic poxvirus was added and the cells were incubated overnight, T cells were added to the corresponding wells of the 96 E-Plate in a cell ratio of E:T = 10:1 at 50 µL/well. Meanwhile, bispecific antibodies BK-A-101, BK-D-04, BK-D-06, and BK-D-07 were added to the corresponding wells of the 96 E-Plate at 50 µL/well, with the final concentration of the bispecific antibodies being 1 nM. The final volume in each well was 250 µL. The system was set to perform a scan and collect data every 15 min. In A of FIG. 17, label 1 represents the HCT116 group, in which T cells, the poxvirus, and the bispecific antibodies were not added; label 2 represents the HCT116 + T cell group; labels 3-6 represent HCT116 + T + bispecific antibody BK-D-04, BK-D-06, BK-D-07, or BK-A-101. In C of FIG. 17, label 1 represents the HCT116 group, in which T cells, the poxvirus, and the bispecific antibodies were not added; label 2 represents the HCT116 + DDvv-TT3 group; label 3 represents the HCT116 + DDvv-TT3 + T cell group; labels 4-7 represent HCT116 + DDvv-TT3 + T + bispecific antibody BK-D-04, BK-D-06, BK-D-07, or BK-A-101. The cell index at 80 h in A of FIG. 17 was converted into the tumor growth inhibition rate (IR, %) to obtain B of FIG. 17; the cell index at 80 h in C of FIG. 17 was converted into the tumor growth inhibition rate to obtain D of FIG. 17.

The results show that in the case that the recombinant oncolytic poxvirus DDvv-TT3 was not added, tumor cells exhibited sustained growth (shown in A of FIG. 17); after the recombinant oncolytic poxvirus DDvv-TT3 was added, bispecific antibody BK-D-04, BK-D-06, BK-D-07, or BK-A-101, combined with T cells, exhibited a significant killing effect (shown in C of FIG. 17). According to the results shown in B and D of FIG. 17, the groups of bispecific antibodies BK-D-04, 06, and 07 + T cells exhibited a growth inhibition rate of less than <10% against HCT116 cells; the DDvv-TT3 + T cell group exhibited a growth inhibition rate of about 40% against HCT116 cells; the groups of bispecific antibodies BK-D-04, 06, and 07, combined with DDvv-TT3 and T cells, exhibited a growth inhibition rate of less than >80% against HCT116 cells, indicating that bispecific antibodies BK-D-04, 06, and 07, combined with DDvv-TT3 and T cells, demonstrated synergistic killing effects.

In the description of the present disclosure, unless otherwise clearly and specifically defined, the term "a plurality of" means at least two, e.g., two, three, etc.

In the specification, the reference term "an embodiment", "some embodiments", "an example", "a specific example", or "some examples" means that a specific feature, structure, material, or characteristic described in connection with the embodiment or example is included in at least one embodiment or example of the present disclosure. In the specification, exemplary descriptions of the foregoing terms are not necessarily intended for the same embodiment or example. Moreover, the specific features, structures, materials, or characteristics described may be combined in any one or more embodiments or examples in a suitable manner. In addition, one skilled in the art may integrate or combine different embodiments or examples described in the specification and features of the different embodiments or examples so long as they are not contradictory to each other.

Although the examples of the present disclosure have been shown and described above, it can be understood that the foregoing examples are exemplary and should not be understood as limitations on the present disclosure. A person of ordinary skill in the art can make changes, modifications, replacements, and variations to the foregoing examples within the scope of the present disclosure.

## Claims

1. A therapeutic agent, comprising:
(a) a first composition comprising a first active ingredient, wherein the first active ingredient comprises or contains a nucleic acid having a labeling polypeptide encoding sequence for being introduced into a tumor cell and/or a cancer cell; the labeling polypeptide has an extracellular antigen determining region, a spacer portion, and a transmembrane portion that are operably linked; an amino acid sequence of the extracellular antigen determining region comprises amino acid sequences of one or more antigenic epitope polypeptides; and in a natural state, an amino acid sequence of a cell membrane protein or a secreted protein of a mammal does not comprise the amino acid sequence of the antigenic epitope polypeptide; and
(b) a second composition comprising a second active ingredient, wherein the second active ingredient comprises a multispecific antibody, and the multispecific antibody comprises at least a first antigen-binding moiety and a second antigen-binding moiety, wherein the first antigen-binding moiety is capable of specifically recognizing and binding to the extracellular antigen determining region of the labeling polypeptide, and
the second antigen-binding moiety is capable of specifically recognizing and binding to an immune cell antigen or a first cytokine or a receptor thereof; and
the multispecific antibody may also optionally comprise a third antigen-binding moiety capable of specifically recognizing and binding to a tumor antigen or an immune checkpoint or a second cytokine or a receptor thereof.

2. The therapeutic agent according to claim 1, wherein the immune cell comprises at least one of a T cell, an NK cell, a DC cell, a B cell, a macrophage, a natural killer T cell, and a neutrophil.

3. The therapeutic agent according to claim 1, wherein the immune cell antigen comprises at least one of CD1, CD1a, CD1b, CD1c, CD1d, CD1e, CD2, CD3, CD3d, CD3e, CD3g, CD4, CD5, CD6, CD7, CD8, CD8a, CD8b, CD9, CD10, CD11, CD11a, CD11b, CD11c, CD11d, CD12w, CD13, CD14, CD15, CD16, CD16a, CD16b, CD17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD42a, CD42b, CD42c, CD42d, CD43, CD44, CD45, CD45RA, CD45RB, CD45RC, CD45RO, CD46, CD47, CD48, CD49a, CD49b, CD49c, CD49d, CD49e, CD49f, CD50, CD52, CD53, CD54, CD55, CD56, CD57, CD58, CD59, CD60a, CD60b, CD60c, CD61, CD62E, CD62L, CD62P, CD63, CD64, CD65, CD66a, CD66b, CD66c, CD66d, CD66e, CD66f, CD67, CD68, CD69, CD70, CD71, CD72, CD73, CD74, CD75, CD75s, CD77, CD79, CD79a, CD79b, CD80, CD81, CD82, CD83, CD84, CD85, CD85a, CD85b, CD85c, CD85d, CD85e, CD85f, CD85g, CD85h, CD85i, CD85j, CD85k, CD86, CD87, CD88, CD89, CD90, CD91, CD92, CD93, CD94, CD95, CD96, CD97, CD98, CD99, CD100, CD101, CD102, CD103, CD104, CD105, CD106, CD107, CD107a, CD107b, CD108, CD109, CD110, CD11, CD112, CD115, CD116, CD117, CD119, CD120, CD121, CD122, CD123, CD124, CD125, CD126, CD127, CD129, CD130, CD133, CD131, CD132, CD134, CD135, CD137, CD138, CD139, CD141, CD142, CD143, CD144, CD147, CD146, CD148, CD150, CD151, CD152, CD153, CD154, CD155, CD156, CD157, CD158, CD158a, CD158b1, CD158b2, CD158c, CD158d, CD158e, CD158f1, CD158f2, CD158g, CD158h, CD158i, CD158j, CD158k, CD158z, CD159a, CD159c, CD160, CD161, CD162, CD163, CD166, CD165, CD166, CD168, CD169, CD170, CD171, CD172a, CD172b, CD172g, CD173, CD177, CD178, CD179, CD180, CD181, CD183, CD184, CD185, CD186, CD191, CD192, CD193, CD194, CD195, CD196, CD197, CD198, CD199, CD200, CD203, CD205, CD208, CD209, CD210, CD210b, CD212, CD231a1, CD213a2, CD217, CD218a, CD218b, CD222, CD224, CD225, CD226, CD227, CD229, CD232, CD243, CD244, CD245, CD247, CD252, CD253, CD256, CD257, CD258, CD261, CD262, CD262, CD263, CD264, CD265, CD268, CD269, CD271, CD272, CD273, CD274, CD275, CD276, CD277, CD278, CD279, CD280, CD281, CD282, CD283, CD284, CD286, CD288, CD289, CD290, CD292, CD293, CD294, CD296, CD297, CD300a, CD300b, CD300c, CD300d, CD300e, CD303, CD304, CD305, CD306, CD307a, CD307b, CD307c, CD307d, CD309, CD312, CD314, CD315, CD316, CD317, CD318, CD319, CD320, CD328, CD329, CD335, CD336, CD337, CD352, CD353, CD357, CD361, and CD362; and
the first cytokine or the receptor thereof and the second cytokine or the receptor thereof each independently comprise at least one of IL-1α, IL-1β, IL-2, IL-4, IL-5, IL-6, IL-7, IL-9, IL-10, IL-11, IL-12, IL-13, IL-15, IL-16, IL-17, IL-17a, IL-17b, IL-17c, IL-17d, IL-17f, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-37, EPO, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL25, CCL26, CCL27, CCL28, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, XCL1, XCL2, CX3CL1, IFNα, IFNβ, IFNγ, TNFα, TGFβ, VEGF, GM-CSF, GCSF, or a related receptor thereof.

4. The therapeutic agent according to claim 1, wherein the tumor antigen is selected from at least one of AFP, BCMA, β-catetin, CD5, CD19, CD20, CD22, CD30, CD38, CD70, CD123, CD133, CD171, CD138, CDK-4, CEA, CA-125, Caspase 8, c-Met, EGFR, Ep-CAM, EphA2, ETA, FAP, GPC3, GP100, GD2, Her2, HPV16, an Igk chain, Ley, Mesothlin, MUC-1, MUC16, MAGE, MART1, an NKG2D ligand, CLL1, NY-ESO-1, P53, RAS, OSA, PAP, PSCA, ROR1, ROR2, PSMA, SSTR2, Trop-2, TYR, VEGFR, and WT1; and
the immune checkpoint is selected from at least one of PD-1, PD-L1, CTLA-4, LAG-3, BTLA, TIM-3, TIGIT, VISTA, TIPE2, Galectin-9, NKG2A, KIRs, B7-H3, B7-H7, 2B4, DNAM-1, PTA1, TLISA1, Vstm3, VSIG9, a Siglecs family, GITR, OX40, 4-1BB, LAYN, LIRB1-5, or a related receptor thereof.

5. The therapeutic agent according to claim 1, wherein the second antigen-binding moiety is capable of specifically recognizing and binding to CD3, and the second antigen-binding moiety comprises at least one selected from OKT3, SP34, L2K, UCHT1, and a variant thereof.

6. The therapeutic agent according to claim 1, wherein the second antigen-binding moiety comprises a heavy chain variable region comprising HCDR1, HCDR2, and HCDR3 and a light chain variable region comprising LCDR1, LCDR2, and LCDR3, wherein amino acid sequences of the HCDR1, the HCDR2, and the HCDR3 are CDR1, CDR2, and CDR3 of a heavy chain variable region set forth in SEQ ID NO: 41, and amino acid sequences of the LCDR1, the LCDR2, and the LCDR3 are CDR1, CDR2, and CDR3 of a light chain variable region set forth in SEQ ID NO: 42;
optionally, the second antigen-binding moiety has the HCDR1, HCDR2, and HCDR3 sequences set forth in SEQ ID NOs: 35, 36, and 37 and the LCDR1, LCDR2, and LCDR3 sequences set forth in SEQ ID NOs: 38, 39, and 40;
or the second antigen-binding moiety has the heavy chain variable region sequence set forth in SEQ ID NO: 41, 43, 44, or 45 and the light chain variable region sequence set forth in SEQ ID NO: 42, 46, 47, 48, 49, 50, 51, 52, or 53;
optionally, the second antigen-binding moiety is selected from:
(a-1) having the heavy chain variable region sequence set forth in SEQ ID NO: 41 and the light chain variable region sequence set forth in SEQ ID NO: 42; or
(a-2) having the heavy chain variable region sequence set forth in SEQ ID NO: 41 and the light chain variable region sequence set forth in SEQ ID NO: 46; or
(a-3) having the heavy chain variable region sequence set forth in SEQ ID NO: 41 and the light chain variable region sequence set forth in SEQ ID NO: 47; or
(a-4) having the heavy chain variable region sequence set forth in SEQ ID NO: 41 and the light chain variable region sequence set forth in SEQ ID NO: 48; or
(a-5) having the heavy chain variable region sequence set forth in SEQ ID NO: 41 and the light chain variable region sequence set forth in SEQ ID NO: 49; or
(a-6) having the heavy chain variable region sequence set forth in SEQ ID NO: 41 and the light chain variable region sequence set forth in SEQ ID NO: 50; or
(a-7) having the heavy chain variable region sequence set forth in SEQ ID NO: 41 and the light chain variable region sequence set forth in SEQ ID NO: 51; or
(a-8) having the heavy chain variable region sequence set forth in SEQ ID NO: 41 and the light chain variable region sequence set forth in SEQ ID NO: 52; or
(a-9) having the heavy chain variable region sequence set forth in SEQ ID NO: 41 and the light chain variable region sequence set forth in SEQ ID NO: 53; or
(b-1) having the heavy chain variable region sequence set forth in SEQ ID NO: 43 and the light chain variable region sequence set forth in SEQ ID NO: 42; or
(b-2) having the heavy chain variable region sequence set forth in SEQ ID NO: 44 and the light chain variable region sequence set forth in SEQ ID NO: 42; or
(b-3) having the heavy chain variable region sequence set forth in SEQ ID NO: 45 and the light chain variable region sequence set forth in SEQ ID NO: 42; or
(c-1) having the heavy chain variable region sequence set forth in SEQ ID NO: 43 and the light chain variable region sequence set forth in SEQ ID NO: 46; or
(c-2) having the heavy chain variable region sequence set forth in SEQ ID NO: 43 and the light chain variable region sequence set forth in SEQ ID NO: 47; or
(c-3) having the heavy chain variable region sequence set forth in SEQ ID NO: 43 and the light chain variable region sequence set forth in SEQ ID NO: 48; or
(c-4) having the heavy chain variable region sequence set forth in SEQ ID NO: 43 and the light chain variable region sequence set forth in SEQ ID NO: 49; or
(c-5) having the heavy chain variable region sequence set forth in SEQ ID NO: 43 and the light chain variable region sequence set forth in SEQ ID NO: 50; or
(c-6) having the heavy chain variable region sequence set forth in SEQ ID NO: 43 and the light chain variable region sequence set forth in SEQ ID NO: 51; or
(c-7) having the heavy chain variable region sequence set forth in SEQ ID NO: 43 and the light chain variable region sequence set forth in SEQ ID NO: 52; or
(c-8) having the heavy chain variable region sequence set forth in SEQ ID NO: 43 and the light chain variable region sequence set forth in SEQ ID NO: 53; or
(d-1) having the heavy chain variable region sequence set forth in SEQ ID NO: 44 and the light chain variable region sequence set forth in SEQ ID NO: 46; or
(d-2) having the heavy chain variable region sequence set forth in SEQ ID NO: 44 and the light chain variable region sequence set forth in SEQ ID NO: 47; or
(d-3) having the heavy chain variable region sequence set forth in SEQ ID NO: 44 and the light chain variable region sequence set forth in SEQ ID NO: 48; or
(d-4) having the heavy chain variable region sequence set forth in SEQ ID NO: 44 and the light chain variable region sequence set forth in SEQ ID NO: 49; or
(d-5) having the heavy chain variable region sequence set forth in SEQ ID NO: 44 and the light chain variable region sequence set forth in SEQ ID NO: 50; or
(d-6) having the heavy chain variable region sequence set forth in SEQ ID NO: 44 and the light chain variable region sequence set forth in SEQ ID NO: 51; or
(d-7) having the heavy chain variable region sequence set forth in SEQ ID NO: 44 and the light chain variable region sequence set forth in SEQ ID NO: 52; or
(d-8) having the heavy chain variable region sequence set forth in SEQ ID NO: 44 and the light chain variable region sequence set forth in SEQ ID NO: 53; or
(e-1) having the heavy chain variable region sequence set forth in SEQ ID NO: 45 and the light chain variable region sequence set forth in SEQ ID NO: 46; or
(e-2) having the heavy chain variable region sequence set forth in SEQ ID NO: 45 and the light chain variable region sequence set forth in SEQ ID NO: 47; or
(e-3) having the heavy chain variable region sequence set forth in SEQ ID NO: 45 and the light chain variable region sequence set forth in SEQ ID NO: 48; or
(e-4) having the heavy chain variable region sequence set forth in SEQ ID NO: 45 and the light chain variable region sequence set forth in SEQ ID NO: 49; or
(e-5) having the heavy chain variable region sequence set forth in SEQ ID NO: 45 and the light chain variable region sequence set forth in SEQ ID NO: 50; or
(e-6) having the heavy chain variable region sequence set forth in SEQ ID NO: 45 and the light chain variable region sequence set forth in SEQ ID NO: 51; or
(e-7) having the heavy chain variable region sequence set forth in SEQ ID NO: 45 and the light chain variable region sequence set forth in SEQ ID NO: 52; or
(e-8) having the heavy chain variable region sequence set forth in SEQ ID NO: 45 and the light chain variable region sequence set forth in SEQ ID NO: 53.

7. The therapeutic agent according to claim 1, wherein the multispecific antibody is produced by a host cell comprising a construct comprising a nucleotide encoding the multispecific antibody.

8. The therapeutic agent according to claim 7, wherein the host cell is selected from at least one of a CHO series cell strain, a 293 series cell strain, an NS0 cell, a Hela cell, a 3T3 cell, and a BHK cell.

9. The therapeutic agent according to claim 1, wherein the amino acid sequence of the antigenic epitope polypeptide is derived from an amino acid sequence of a naturally occurring protein or is an artificially synthesized amino acid sequence that is not naturally occurring.

10. The therapeutic agent according to claim 1, wherein the amino acid sequence of the antigenic epitope polypeptide comprises amino acid sequences of the following tags: a Myc tag, an HA tag, Strep tag I, Strep tag II, a Flag tag, a HAT tag, an S tag, an Sl tag, a protein C tag, a tag-100 tag, an E2 tag, a TAP tag, an HSV tag, a KT3 tag, a V5 tag, a VSV-G tag, a His tag, or an RFP tag.

11. The therapeutic agent according to claim 1, wherein the extracellular antigen determining region has an amino acid sequence set forth in SEQ ID NO: 1, 2, 3, 4, or 5.

12. The therapeutic agent according to claim 1, wherein the spacer portion is derived from a hinge region of CD8α, a hinge region of IgG, or a hinge region of IgD; preferably, the spacer portion has an amino acid sequence set forth in SEQ ID NO: 6; and
the transmembrane portion is derived from a transmembrane region of CD8, CD3ζ, CD4, or CD28; preferably, the transmembrane portion has an amino acid sequence set forth in SEQ ID NO: 7.

13. The therapeutic agent according to claim 1, wherein the labeling polypeptide has an amino acid sequence set forth in SEQ ID NO: 11, 12, 13, 14, or 15.

14. The therapeutic agent according to claim 1, wherein the first antigen-binding moiety comprises a heavy chain variable region comprising HCDR1, HCDR2, and HCDR3 and a light chain variable region comprising LCDR1, LCDR2, and LCDR3, wherein amino acid sequences of the HCDR, the HCDR2, and the HCDR3 are CDR1, CDR2, and CDR3 of a heavy chain variable region set forth in SEQ ID NO: 27, and amino acid sequences of the LCDR1, the LCDR2, and the LCDR3 are CDR1, CDR2, and CDR3 of a light chain variable region set forth in SEQ ID NO: 26;
optionally, the first antigen-binding moiety has the HCDR1, HCDR, and HCDR3 sequences set forth in SEQ ID NOs: 20, 21, and 22 and the LCDR1, LCDR2, and LCDR3 sequences set forth in SEQ ID NOs: 23, 24, and 25;
or the first antigen-binding moiety has the heavy chain variable region sequence set forth in SEQ ID NO: 27, 29, 30, or 31 and the light chain variable region sequence set forth in SEQ ID NO: 26, 32, 33, or 34;
optionally, the first antigen-binding moiety is selected from:
(1-1) having the heavy chain variable region sequence set forth in SEQ ID NO: 27 and the heavy chain variable region sequence set forth in SEQ ID NO: 26; or
(1-2) having the heavy chain variable region sequence set forth in SEQ ID NO: 27 and the heavy chain variable region sequence set forth in SEQ ID NO: 32; or
(1-3) having the heavy chain variable region sequence set forth in SEQ ID NO: 27 and the heavy chain variable region sequence set forth in SEQ ID NO: 33; or
(1-4) having the heavy chain variable region sequence set forth in SEQ ID NO: 27 and the heavy chain variable region sequence set forth in SEQ ID NO: 34; or
(1-5) having the heavy chain variable region sequence set forth in SEQ ID NO: 29 and the heavy chain variable region sequence set forth in SEQ ID NO: 26; or
(1-6) having the heavy chain variable region sequence set forth in SEQ ID NO: 30 and the heavy chain variable region sequence set forth in SEQ ID NO: 26; or
(1-7) having the heavy chain variable region sequence set forth in SEQ ID NO: 31 and the heavy chain variable region sequence set forth in SEQ ID NO: 26; or
(1-8) having the heavy chain variable region sequence set forth in SEQ ID NO: 29 and the light chain variable region sequence set forth in SEQ ID NO: 32; or
(1-9) having the heavy chain variable region sequence set forth in SEQ ID NO: 29 and the light chain variable region sequence set forth in SEQ ID NO: 33; or
(1-10) having the heavy chain variable region sequence set forth in SEQ ID NO: 29 and the light chain variable region sequence set forth in SEQ ID NO: 34; or
(1-11) having the heavy chain variable region sequence set forth in SEQ ID NO: 30 and the light chain variable region sequence set forth in SEQ ID NO: 32; or
(1-12) having the heavy chain variable region sequence set forth in SEQ ID NO: 30 and the light chain variable region sequence set forth in SEQ ID NO: 33; or
(1-13) having the heavy chain variable region sequence set forth in SEQ ID NO: 30 and the light chain variable region sequence set forth in SEQ ID NO: 34; or
(1-14) having the heavy chain variable region sequence set forth in SEQ ID NO: 31 and the light chain variable region sequence set forth in SEQ ID NO: 32;
(1-15) having the heavy chain variable region sequence set forth in SEQ ID NO: 31 and the light chain variable region sequence set forth in SEQ ID NO: 33; or
(1-16) having the heavy chain variable region sequence set forth in SEQ ID NO: 31 and the light chain variable region sequence set forth in SEQ ID NO: 34.

15. The therapeutic agent according to claim 1, wherein the nucleic acid comprises a DNA or an RNA; and the RNA comprises an mRNA transcribed from a DNA.

16. The therapeutic agent according to claim 15, wherein the first composition comprises a therapeutically effective amount of the DNA or a therapeutically effective amount of the mRNA.

17. The therapeutic agent according to claim 15, wherein the DNA is formulated for administration by intratumoral injection, intravenous injection, intraperitoneal injection, or intracranial injection; and the mRNA is formulated for administration by intratumoral injection, intravenous injection, intraperitoneal injection, or intracranial injection.

18. The therapeutic agent according to claim 1, wherein the first active ingredient is a recombinant virus, wherein a genome of the recombinant virus has the labeling polypeptide encoding sequence; and the recombinant virus comprises a replication-selective recombinant oncolytic virus or a replication-deficient recombinant virus.

19. The therapeutic agent according to claim 18, wherein the first composition comprises a therapeutically effective amount of the recombinant virus.

20. The therapeutic agent according to claim 18, wherein the recombinant oncolytic virus is derived from a genetically mutated virus having an oncolytic effect and a wild-type virus having an oncolytic effect; preferably, the recombinant oncolytic virus is derived from an adenovirus, a poxvirus, a herpes simplex virus, a measles virus, a Semliki forest virus, a vesicular stomatitis virus, a poliovirus, a retrovirus, a reovirus, a Seneca valley virus, an Echo-type enterovirus, a Coxsackie virus, a Newcastle disease virus, and a Malaba virus having an oncolytic effect.

21. The therapeutic agent according to claim 18, wherein the recombinant virus is formulated for administration by intratumoral injection, intravenous administration, administration by intraperitoneal injection, or administration by intracranial injection.

22. The therapeutic agent according to claim 1, wherein the first composition and the second composition are each independently present in the therapeutic agent without intermixing.

23. The therapeutic agent according to claim 1, wherein the therapeutic agent consists of the first composition and the second composition.

24. The therapeutic agent according to claim 1, wherein the first antigen-binding moiety and the second antigen-binding moiety are linked in the following manner:
1) a heavy chain variable region and a light chain variable region of the second antigen-binding moiety are respectively linked to a heavy chain constant region and a light chain constant region to form an IgG-like structure, a carboxyl-terminal amino acid of an Fc region of the IgG-like structure is linked to an amino-terminal amino acid of a light chain variable region of the first antigen-binding moiety, and a carboxyl-terminal amino acid of a light chain variable region of the first antigen-binding moiety is linked to an amino-terminal amino acid of a heavy chain variable region of the first antigen-binding moiety; or
2) a heavy chain variable region and a light chain variable region of the first antigen-binding moiety are respectively linked to a heavy chain constant region and a light chain constant region to form an IgG-like structure, a carboxyl-terminal amino acid of an Fc region of the IgG-like structure is linked to an amino-terminal amino acid of a light chain variable region of the second antigen-binding moiety, and a carboxyl-terminal amino acid of a light chain variable region of the second antigen-binding moiety is linked to an amino-terminal amino acid of a heavy chain variable region of the second antigen-binding moiety; or
3) a carboxyl-terminal amino acid of a light chain variable region of the first antigen-binding moiety is linked to an amino-terminal amino acid of a heavy chain variable region of the first antigen-binding moiety, a carboxy-terminal amino acid of the first antigen-binding moiety is directly linked to an Fc region, a carboxy-terminal amino acid of the Fc region is linked to an amino-terminal amino acid of the second antigen-binding moiety, and a carboxy-terminal amino acid of a light chain variable region of the second antigen-binding moiety is linked to an amino-terminal amino acid of a heavy chain variable region of the second antigen-binding moiety; or
4) a carboxyl-terminal amino acid of a light chain variable region of the first antigen-binding moiety is linked to an amino-terminal amino acid of a heavy chain variable region of the first antigen-binding moiety, a carboxy-terminal amino acid of the first antigen-binding moiety is directly linked to an Fc region, a carboxy-terminal amino acid of the Fc region is linked to an amino-terminal amino acid of the second antigen-binding moiety, and a carboxy-terminal amino acid of a heavy chain variable region of the second antigen-binding moiety is linked to an amino-terminal amino acid of a light chain variable region of the second antigen-binding moiety.

25. The therapeutic agent according to claim 24, wherein the Fc region is a humanized sequence or a mutated humanized sequence.

26. The therapeutic agent according to claim 1, wherein the second antigen-binding moiety comprises one or more domains capable of specifically recognizing and binding to CD3, and the first antigen-binding moiety comprises one or more domains capable of specifically recognizing and binding to the antigenic epitope polypeptide, wherein the amino acid sequence of the antigenic epitope polypeptide comprises an amino acid sequence of Strep tag I or Strep tag II.

27. A multispecific antibody, comprising at least a first antigen-binding moiety and a second antigen-binding moiety, wherein the first antigen-binding moiety comprises one or more domains capable of specifically recognizing and binding to an amino acid sequence of Strep tag I or Strep tag II, and the second antigen-binding moiety comprises one or more domains capable of specifically recognizing and binding to an immune cell antigen or a first cytokine or a receptor thereof; and
the multispecific antibody may also optionally comprise a third antigen-binding moiety comprising one or more domains capable of specifically recognizing and binding to a tumor antigen or an immune checkpoint or a second cytokine or a receptor thereof.

28. The multispecific antibody according to claim 27, wherein the immune cell comprises at least one of a T cell, an NK cell, a DC cell, a B cell, a macrophage, a natural killer T cell, and a neutrophil.

29. The multispecific antibody according to claim 27, wherein the immune cell antigen comprises at least one of CD1, CD1a, CD1b, CD1c, CD1d, CD1e, CD2, CD3, CD3d, CD3e, CD3g, CD4, CD5, CD6, CD7, CD8, CD8a, CD8b, CD9, CD10, CD11, CD11a, CD11b, CD11c, CD11d, CD12w, CD13, CD14, CD15, CD16, CD16a, CD16b, CD17, CD18, CD19, CD20, CD21, CD22, CD23, CD24, CD25, CD26, CD27, CD28, CD29, CD30, CD31, CD32, CD33, CD34, CD35, CD36, CD37, CD38, CD39, CD40, CD41, CD42, CD42a, CD42b, CD42c, CD42d, CD43, CD44, CD45, CD45RA, CD45RB, CD45RC, CD45RO, CD46, CD47, CD48, CD49a, CD49b, CD49c, CD49d, CD49e, CD49f, CD50, CD52, CD53, CD54, CD55, CD56, CD57, CD58, CD59, CD60a, CD60b, CD60c, CD61, CD62E, CD62L, CD62P, CD63, CD64, CD65, CD66a, CD66b, CD66c, CD66d, CD66e, CD66f, CD67, CD68, CD69, CD70, CD71, CD72, CD73, CD74, CD75, CD75s, CD77, CD79, CD79a, CD79b, CD80, CD81, CD82, CD83, CD84, CD85, CD85a, CD85b, CD85c, CD85d, CD85e, CD85f, CD85g, CD85h, CD85i, CD85j, CD85k, CD86, CD87, CD88, CD89, CD90, CD91, CD92, CD93, CD94, CD95, CD96, CD97, CD98, CD99, CD100, CD101, CD102, CD103, CD104, CD105, CD106, CD107, CD107a, CD107b, CD108, CD109, CD110, CD11, CD112, CD115, CD116, CD117, CD119, CD120, CD121, CD122, CD123, CD124, CD125, CD126, CD127, CD129, CD130, CD133, CD131, CD132, CD134, CD135, CD137, CD138, CD139, CD141, CD142, CD143, CD144, CD147, CD146, CD148, CD150, CD151, CD152, CD153, CD154, CD155, CD156, CD157, CD158, CD158a, CD158b1, CD158b2, CD158c, CD158d, CD158e, CD158f1, CD158f2, CD158g, CD158h, CD158i, CD158j, CD158k, CD158z, CD159a, CD159c, CD160, CD161, CD162, CD163, CD166, CD165, CD166, CD168, CD169, CD170, CD171, CD172a, CD172b, CD172g, CD173, CD177, CD178, CD179, CD180, CD181, CD183, CD184, CD185, CD186, CD191, CD192, CD193, CD194, CD195, CD196, CD197, CD198, CD199, CD200, CD203, CD205, CD208, CD209, CD210, CD210b, CD212, CD231a1, CD213a2, CD217, CD218a, CD218b, CD222, CD224, CD225, CD226, CD227, CD229, CD232, CD243, CD244, CD245, CD247, CD252, CD253, CD256, CD257, CD258, CD261, CD262, CD262, CD263, CD264, CD265, CD268, CD269, CD271, CD272, CD273, CD274, CD275, CD276, CD277, CD278, CD279, CD280, CD281, CD282, CD283, CD284, CD286, CD288, CD289, CD290, CD292, CD293, CD294, CD296, CD297, CD300a, CD300b, CD300c, CD300d, CD300e, CD303, CD304, CD305, CD306, CD307a, CD307b, CD307c, CD307d, CD309, CD312, CD314, CD315, CD316, CD317, CD318, CD319, CD320, CD328, CD329, CD335, CD336, CD337, CD352, CD353, CD357, CD361, and CD362; and
the first cytokine or the receptor thereof and the second cytokine or the receptor thereof each independently comprise at least one of IL-1α, IL-1β, IL-2, IL-4, IL-5, IL-6, IL-7, IL-9, IL-10, IL-11, IL-12, IL-13, IL-15, IL-16, IL-17, IL-17a, IL-17b, IL-17c, IL-17d, IL-17f, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-37, EPO, CCL1, CCL2, CCL3, CCL4, CCL5, CCL6, CCL7, CCL8, CCL9, CCL10, CCL11, CCL12, CCL13, CCL14, CCL15, CCL16, CCL17, CCL18, CCL19, CCL20, CCL21, CCL22, CCL23, CCL25, CCL26, CCL27, CCL28, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, XCL1, XCL2, CX3CL1, IFNα, IFNβ, IFNγ, TNFα, TGFβ, VEGF, GM-CSF, GCSF, or a related receptor thereof.

30. The multispecific antibody according to claim 27, wherein the tumor antigen is selected from at least one of AFP, BCMA, β-catetin, CD5, CD19, CD20, CD22, CD30, CD38, CD70, CD123, CD133, CD171, CD138, CDK-4, CEA, CA-125, Caspase 8, c-Met, EGFR, Ep-CAM, EphA2, ETA, FAP, GPC3, GP100, GD2, Her2, HPV16, an Igk chain, Ley, Mesothlin, MUC-1, MUC16, MAGE, MART1, an NKG2D ligand, CLL1, NY-ESO-1, P53, RAS, OSA, PAP, PSCA, ROR1, ROR2, PSMA, SSTR2, Trop-2, TYR, VEGFR, and WT1; and
the immune checkpoint is selected from at least one of PD-1, PD-L1, CTLA-4, LAG-3, BTLA, TIM-3, TIGIT, VISTA, TIPE2, Galectin-9, NKG2A, KIRs, B7-H3, B7-H7, 2B4, DNAM-1, PTA1, TLISA1, Vstm3, VSIG9, a Siglecs family, GITR, OX40, 4-1BB, LAYN, LIRB1-5, or a related receptor thereof.

31. The multispecific antibody according to claim 27, wherein the second antigen-binding moiety comprises one or more domains capable of specifically recognizing and binding to CD3, and the first antigen-binding moiety comprises one or more domains capable of specifically recognizing and binding to an amino acid sequence of Strep tag I or Strep tag II.

32. The multispecific antibody according to claim 27, wherein the second antigen-binding moiety comprises a heavy chain variable region comprising HCDR1, HCDR2, and HCDR3 and a light chain variable region comprising LCDR1, LCDR2, and LCDR3, wherein amino acid sequences of the HCDR1, the HCDR2, and the HCDR3 are CDR1, CDR2, and CDR3 of a heavy chain variable region set forth in SEQ ID NO: 41, and amino acid sequences of the LCDR1, the LCDR2, and the LCDR3 are CDR1, CDR2, and CDR3 of a light chain variable region set forth in SEQ ID NO: 42; and/or
the first antigen-binding moiety comprises a heavy chain variable region comprising HCDR1, HCDR2, and HCDR3 and a light chain variable region comprising LCDR1, LCDR2, and LCDR3, wherein amino acid sequences of the HCDR, the HCDR2, and the HCDR3 are CDR1, CDR2, and CDR3 of a heavy chain variable region set forth in SEQ ID NO: 27, and amino acid sequences of the LCDR1, the LCDR2, and the LCDR3 are CDR1, CDR2, and CDR3 of a light chain variable region set forth in SEQ ID NO: 26;
optionally, the second antigen-binding moiety has the HCDR1, HCDR2, and HCDR3 sequences set forth in SEQ ID NOs: 35, 36, and 37 and the LCDR1, LCDR2, and LCDR3 sequences set forth in SEQ ID NOs: 38, 39, and 40; and the first antigen-binding moiety has the HCDR1, HCDR2, and HCDR3 sequences set forth in SEQ ID NOs: 20, 21, and 22; and the LCDR1, LCDR2, and LCDR3 sequences set forth in SEQ ID NOs: 23, 24, and 25;
optionally, the second antigen-binding moiety has the heavy chain variable region sequence set forth in SEQ ID NO: 41, 43, 44, or 45 and the light chain variable region sequence set forth in SEQ ID NO: 42, 46, 47, 48, 49, 50, 51, 52, or 53; and the first antigen-binding moiety has the heavy chain variable region sequence set forth in SEQ ID NO: 27, 29, 30, or 31 and the light chain variable region sequence set forth in SEQ ID NO: 26, 32, 33, or 34;
optionally, the second antigen-binding moiety is selected from:
(a-1) having the heavy chain variable region sequence set forth in SEQ ID NO: 41 and the light chain variable region sequence set forth in SEQ ID NO: 42; or
(a-2) having the heavy chain variable region sequence set forth in SEQ ID NO: 41 and the light chain variable region sequence set forth in SEQ ID NO: 46; or
(a-3) having the heavy chain variable region sequence set forth in SEQ ID NO: 41 and the light chain variable region sequence set forth in SEQ ID NO: 47; or
(a-4) having the heavy chain variable region sequence set forth in SEQ ID NO: 41 and the light chain variable region sequence set forth in SEQ ID NO: 48; or
(a-5) having the heavy chain variable region sequence set forth in SEQ ID NO: 41 and the light chain variable region sequence set forth in SEQ ID NO: 49; or
(a-6) having the heavy chain variable region sequence set forth in SEQ ID NO: 41 and the light chain variable region sequence set forth in SEQ ID NO: 50; or
(a-7) having the heavy chain variable region sequence set forth in SEQ ID NO: 41 and the light chain variable region sequence set forth in SEQ ID NO: 51; or
(a-8) having the heavy chain variable region sequence set forth in SEQ ID NO: 41 and the light chain variable region sequence set forth in SEQ ID NO: 52; or
(a-9) having the heavy chain variable region sequence set forth in SEQ ID NO: 41 and the light chain variable region sequence set forth in SEQ ID NO: 53; or
(b-1) having the heavy chain variable region sequence set forth in SEQ ID NO: 43 and the light chain variable region sequence set forth in SEQ ID NO: 42; or
(b-2) having the heavy chain variable region sequence set forth in SEQ ID NO: 44 and the light chain variable region sequence set forth in SEQ ID NO: 42; or
(b-3) having the heavy chain variable region sequence set forth in SEQ ID NO: 45 and the light chain variable region sequence set forth in SEQ ID NO: 42; or
(c-1) having the heavy chain variable region sequence set forth in SEQ ID NO: 43 and the light chain variable region sequence set forth in SEQ ID NO: 46; or
(c-2) having the heavy chain variable region sequence set forth in SEQ ID NO: 43 and the light chain variable region sequence set forth in SEQ ID NO: 47; or
(c-3) having the heavy chain variable region sequence set forth in SEQ ID NO: 43 and the light chain variable region sequence set forth in SEQ ID NO: 48; or
(c-4) having the heavy chain variable region sequence set forth in SEQ ID NO: 43 and the light chain variable region sequence set forth in SEQ ID NO: 49; or
(c-5) having the heavy chain variable region sequence set forth in SEQ ID NO: 43 and the light chain variable region sequence set forth in SEQ ID NO: 50; or
(c-6) having the heavy chain variable region sequence set forth in SEQ ID NO: 43 and the light chain variable region sequence set forth in SEQ ID NO: 51; or
(c-7) having the heavy chain variable region sequence set forth in SEQ ID NO: 43 and the light chain variable region sequence set forth in SEQ ID NO: 52; or
(c-8) having the heavy chain variable region sequence set forth in SEQ ID NO: 43 and the light chain variable region sequence set forth in SEQ ID NO: 53; or
(d-1) having the heavy chain variable region sequence set forth in SEQ ID NO: 44 and the light chain variable region sequence set forth in SEQ ID NO: 46; or
(d-2) having the heavy chain variable region sequence set forth in SEQ ID NO: 44 and the light chain variable region sequence set forth in SEQ ID NO: 47; or
(d-3) having the heavy chain variable region sequence set forth in SEQ ID NO: 44 and the light chain variable region sequence set forth in SEQ ID NO: 48; or
(d-4) having the heavy chain variable region sequence set forth in SEQ ID NO: 44 and the light chain variable region sequence set forth in SEQ ID NO: 49; or
(d-5) having the heavy chain variable region sequence set forth in SEQ ID NO: 44 and the light chain variable region sequence set forth in SEQ ID NO: 50; or
(d-6) having the heavy chain variable region sequence set forth in SEQ ID NO: 44 and the light chain variable region sequence set forth in SEQ ID NO: 51; or
(d-7) having the heavy chain variable region sequence set forth in SEQ ID NO: 44 and the light chain variable region sequence set forth in SEQ ID NO: 52; or
(d-8) having the heavy chain variable region sequence set forth in SEQ ID NO: 44 and the light chain variable region sequence set forth in SEQ ID NO: 53; or
(e-1) having the heavy chain variable region sequence set forth in SEQ ID NO: 45 and the light chain variable region sequence set forth in SEQ ID NO: 46; or
(e-2) having the heavy chain variable region sequence set forth in SEQ ID NO: 45 and the light chain variable region sequence set forth in SEQ ID NO: 47; or
(e-3) having the heavy chain variable region sequence set forth in SEQ ID NO: 45 and the light chain variable region sequence set forth in SEQ ID NO: 48; or
(e-4) having the heavy chain variable region sequence set forth in SEQ ID NO: 45 and the light chain variable region sequence set forth in SEQ ID NO: 49; or
(e-5) having the heavy chain variable region sequence set forth in SEQ ID NO: 45 and the light chain variable region sequence set forth in SEQ ID NO: 50; or
(e-6) having the heavy chain variable region sequence set forth in SEQ ID NO: 45 and the light chain variable region sequence set forth in SEQ ID NO: 51; or
(e-7) having the heavy chain variable region sequence set forth in SEQ ID NO: 45 and the light chain variable region sequence set forth in SEQ ID NO: 52; or
(e-8) having the heavy chain variable region sequence set forth in SEQ ID NO: 45 and the light chain variable region sequence set forth in SEQ ID NO: 53;
optionally, the first antigen-binding moiety is selected from:
(1-1) having the heavy chain variable region sequence set forth in SEQ ID NO: 27 and the heavy chain variable region sequence set forth in SEQ ID NO: 26; or
(1-2) having the heavy chain variable region sequence set forth in SEQ ID NO: 27 and the heavy chain variable region sequence set forth in SEQ ID NO: 32; or
(1-3) having the heavy chain variable region sequence set forth in SEQ ID NO: 27 and the heavy chain variable region sequence set forth in SEQ ID NO: 33; or
(1-4) having the heavy chain variable region sequence set forth in SEQ ID NO: 27 and the heavy chain variable region sequence set forth in SEQ ID NO: 34; or
(1-5) having the heavy chain variable region sequence set forth in SEQ ID NO: 29 and the heavy chain variable region sequence set forth in SEQ ID NO: 26; or
(1-6) having the heavy chain variable region sequence set forth in SEQ ID NO: 30 and the heavy chain variable region sequence set forth in SEQ ID NO: 26; or
(1-7) having the heavy chain variable region sequence set forth in SEQ ID NO: 31 and the heavy chain variable region sequence set forth in SEQ ID NO: 26; or
(1-8) having the heavy chain variable region sequence set forth in SEQ ID NO: 29 and the light chain variable region sequence set forth in SEQ ID NO: 32; or
(1-9) having the heavy chain variable region sequence set forth in SEQ ID NO: 29 and the light chain variable region sequence set forth in SEQ ID NO: 33; or
(1-10) having the heavy chain variable region sequence set forth in SEQ ID NO: 29 and the light chain variable region sequence set forth in SEQ ID NO: 34; or
(1-11) having the heavy chain variable region sequence set forth in SEQ ID NO: 30 and the light chain variable region sequence set forth in SEQ ID NO: 32; or
(1-12) having the heavy chain variable region sequence set forth in SEQ ID NO: 30 and the light chain variable region sequence set forth in SEQ ID NO: 33; or
(1-13) having the heavy chain variable region sequence set forth in SEQ ID NO: 30 and the light chain variable region sequence set forth in SEQ ID NO: 34; or
(1-14) having the heavy chain variable region sequence set forth in SEQ ID NO: 31 and the light chain variable region sequence set forth in SEQ ID NO: 32;
(1-15) having the heavy chain variable region sequence set forth in SEQ ID NO: 31 and the light chain variable region sequence set forth in SEQ ID NO: 33; or
(1-16) having the heavy chain variable region sequence set forth in SEQ ID NO: 31 and the light chain variable region sequence set forth in SEQ ID NO: 34.

33. The multispecific antibody according to claim 32, wherein the first antigen-binding moiety and the second antigen-binding moiety are linked in the following manner:
1) a heavy chain variable region and a light chain variable region of the second antigen-binding moiety are respectively linked to a heavy chain constant region and a light chain constant region to form an IgG-like structure, a carboxyl-terminal amino acid of an Fc region of the IgG-like structure is linked to an amino-terminal amino acid of a light chain variable region of the first antigen-binding moiety, and a carboxyl-terminal amino acid of a light chain variable region of the first antigen-binding moiety is linked to an amino-terminal amino acid of a heavy chain variable region of the first antigen-binding moiety; or
2) a heavy chain variable region and a light chain variable region of the first antigen-binding moiety are respectively linked to a heavy chain constant region and a light chain constant region to form an IgG-like structure, a carboxyl-terminal amino acid of an Fc region of the IgG-like structure is linked to an amino-terminal amino acid of a light chain variable region of the second antigen-binding moiety, and a carboxyl-terminal amino acid of a light chain variable region of the second antigen-binding moiety is linked to an amino-terminal amino acid of a heavy chain variable region of the second antigen-binding moiety; or
3) a carboxyl-terminal amino acid of a light chain variable region of the first antigen-binding moiety is linked to an amino-terminal amino acid of a heavy chain variable region of the first antigen-binding moiety, a carboxy-terminal amino acid of the first antigen-binding moiety is directly linked to an Fc region, a carboxy-terminal amino acid of the Fc region is linked to an amino-terminal amino acid of the second antigen-binding moiety, and a carboxy-terminal amino acid of a light chain variable region of the second antigen-binding moiety is linked to an amino-terminal amino acid of a heavy chain variable region of the second antigen-binding moiety; or
4) a carboxyl-terminal amino acid of a light chain variable region of the first antigen-binding moiety is linked to an amino-terminal amino acid of a heavy chain variable region of the first antigen-binding moiety, a carboxy-terminal amino acid of the first antigen-binding moiety is directly linked to an Fc region, a carboxy-terminal amino acid of the Fc region is linked to an amino-terminal amino acid of the second antigen-binding moiety, and a carboxy-terminal amino acid of a heavy chain variable region of the second antigen-binding moiety is linked to an amino-terminal amino acid of a light chain variable region of the second antigen-binding moiety.

34. The multispecific antibody according to claim 33, wherein the Fc region is a humanized sequence or a mutated humanized sequence.

35. A polynucleotide, wherein the polynucleotide encodes the multispecific antibody according to any one of claims 27-34.

36. A construct, comprising the polynucleotide according to claim 35.

37. A host cell, comprising the construct according to claim 36,
wherein optionally, the host cell is a prokaryotic cell or a eukaryotic cell.

38. A method for producing the multispecific antibody according to any one of claims 27-34, comprising:
culturing the host cell according to claim 37, and
collecting the multispecific antibody from the culture.

39. Use of the therapeutic agent according to any one of claims 1-26 or the multispecific antibody according to any one of claims 27-34 in the manufacture of a medicament for treating a tumor and/or a cancer.

40. The use according to claim 39, wherein the tumor and/or the cancer comprises at least one of breast cancer, head and neck tumor, synovial cancer, kidney cancer, connective tissue cancer, melanoma, lung cancer, esophageal cancer, colon cancer, rectal cancer, brain cancer, liver cancer, bone cancer, choriocarcinoma, gastrinoma, pheochromocytoma, prolactinoma, von Hippel-Lindau disease, Zollinger-Ellison syndrome, anal cancer, bile duct cancer, bladder cancer, ureteral cancer, glioma, neuroblastoma, meningioma, spinal cord tumor, osteochondroma, chondrosarcoma, Ewing's sarcoma, cancer of unknown primary site, carcinoid, fibrosarcoma, Paget's disease, cervical cancer, gallbladder cancer, eye cancer, Kaposi's sarcoma, prostate cancer, testicular cancer, cutaneous squamous cell carcinoma, mesothelioma, multifocal myeloma, ovarian cancer, pancreatic endocrine tumor, glucagonoma, pancreatic cancer, penile cancer, pituitary cancer, soft tissue sarcoma, retinoblastoma, small intestine cancer, gastric cancer, thymic cancer, trophoblastic cancer, hydatidiform mole, endometrial cancer, vaginal cancer, vulvar cancer, mycosis fungoides, insulinoma, heart cancer, meningeal cancer, peritoneal cancer, pleural cancer, and hematological cancer.

41. A kit of combinational drugs with a synergistic effect for treating a tumor and/or a cancer, comprising:
a first container containing the first composition in the therapeutic agent according to any one of claims 1-26;
a second container containing the second composition in the therapeutic agent according to any one of claims 1-26 or containing the multispecific antibody according to any one of claims 27-34, wherein the first container and the second container are independent; and
instructions for the timing and mode of administration.

42. A method for treating a tumor and/or a cancer, comprising:
administering to a subject the first composition in the therapeutic agent according to any one of claims 1-26; and
administering to the subject the second composition in the therapeutic agent according to any one of claims 1-26, or the multispecific antibody according to any one of claims 27-34.

43. The method according to claim 42, comprising the following steps performed in sequence:
1) administering to the subject the first composition; and
2) administering to the subject the second composition or the multispecific antibody after administration of the first composition.

44. The method according to claim 43, wherein the tumor and/or the cancer comprises at least one of breast cancer, head and neck tumor, synovial cancer, kidney cancer, connective tissue cancer, melanoma, lung cancer, esophageal cancer, colon cancer, rectal cancer, brain cancer, liver cancer, bone cancer, choriocarcinoma, gastrinoma, pheochromocytoma, prolactinoma, von Hippel-Lindau disease, Zollinger-Ellison syndrome, anal cancer, bile duct cancer, bladder cancer, ureteral cancer, glioma, neuroblastoma, meningioma, spinal cord tumor, osteochondroma, chondrosarcoma, Ewing's sarcoma, cancer of unknown primary site, carcinoid, fibrosarcoma, Paget's disease, cervical cancer, gallbladder cancer, eye cancer, Kaposi's sarcoma, prostate cancer, testicular cancer, cutaneous squamous cell carcinoma, mesothelioma, multifocal myeloma, ovarian cancer, pancreatic endocrine tumor, glucagonoma, pancreatic cancer, penile cancer, pituitary cancer, soft tissue sarcoma, retinoblastoma, small intestine cancer, gastric cancer, thymic cancer, trophoblastic cancer, hydatidiform mole, endometrial cancer, vaginal cancer, vulvar cancer, mycosis fungoides, insulinoma, heart cancer, meningeal cancer, peritoneal cancer, pleural cancer, and hematological cancer.

45. An immunotherapy, comprising:
administering to a subject a therapeutically effective amount of an oncolytic virus, wherein a genome of the oncolytic virus comprises a labeling polypeptide encoding sequence, and the labeling polypeptide has an extracellular antigen determining region, a spacer portion, and a transmembrane portion that are operably linked; an amino acid sequence of the extracellular antigen determining region comprises one or more of amino acid sequences of an antigenic epitope polypeptide; and in a natural state, an amino acid sequence of a cell membrane protein or a secreted protein of the subject does not comprise the amino acid sequence of the antigenic epitope polypeptide; and
administering to the subject a therapeutically effective amount of a multispecific antibody, wherein the multispecific antibody comprises at least a first antigen-binding moiety and a second antigen-binding moiety, wherein the first antigen-binding moiety is capable of specifically recognizing and binding to the extracellular antigen determining region of the labeling polypeptide, and the second antigen-binding moiety is capable of specifically recognizing and binding to an immune cell antigen or a first cytokine or a receptor thereof; and
the multispecific antibody may also optionally comprise a third antigen-binding moiety capable of specifically recognizing and binding to a tumor antigen or an immune checkpoint or a second cytokine or a receptor thereof.

46. The method according to claim 45, comprising the following steps performed in sequence:
1) administering to the subject a therapeutically effective amount of the oncolytic virus; and
2) administering to the subject a therapeutically effective amount of the multispecific antibody after administration of the oncolytic virus.
